# EUROPEAN PATENT APPLICATION

(11) **EP 4 067 359 A1**
(43) Date of publication of application: **05.10.2022**
(21) Application number: 20892868.9
(22) Date of filing: 25.11.2020
(51) Int. Cl.: C07D 473/18, C07D 519/00, A61K 31/522, A61P 35/00

(54) **PYRIMIDOIMIDAZOLE COMPOUNDS USED AS DNA-PK INHIBITORS**

(30) Priority: 25.11.2019 CN 201911164961; 23.03.2020 CN 202010209352; 02.09.2020 CN 202010911879; 13.11.2020 CN 202011269768
(71) Applicant: Medshine Discovery Inc., Nanjing, Jiangsu 210032 (CN)
(72) Inventor: CHEN, Kevin X, Shanghai 200131 (CN); XIA, Shanghua, Shanghai 200131 (CN); CHEN, Zhaoguo, Shanghai 200131 (CN); GUO, Zuhao, Shanghai 200131 (CN); YU, Yanxin, Shanghai 200131 (CN); ZHOU, Kai, Shanghai 200131 (CN); HU, Boyu, Shanghai 200131 (CN); ZHANG, Li, Shanghai 200131 (CN); JIANG, Fen, Shanghai 200131 (CN); WANG, Jingjing, Shanghai 200131 (CN); HU, Guoping, Shanghai 200131 (CN); LI, Jian, Shanghai 200131 (CN); CHEN, Shuhui, Shanghai 200131 (CN)
(74) Representative: Dehns
(86) International application number: PCT/CN2020/131275
(87) International publication number: WO 2021/104277

(57) **Abstract**

A class of DNA-PK inhibitors, in particular a compound represented by formula (IV) or a pharmaceutically acceptable salt thereof, and an application thereof in the preparation of a drug relating to a DNA-PK inhibitor.

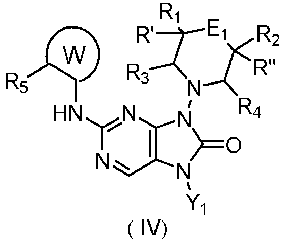

## Description

### The present invention claims the following priorities:

CN201911164961.5, filing date: November 25, 2019;

CN202010209352.3, filing date: March 23, 2020;

CN202010911879.0, filing date: September 02, 2020;

CN202011269768.0, filing date: November 13, 2020.

### Technical Field

The present disclosure relates to a DNA-PK inhibitor, in particlular to a compound represented by formula (IV) or a pharmaceutically acceptable salt thereof, and a use thereof in the manufacture of a madicament related to a DNA-PK inhibitor.

### Background

DNA breaks, especially double-strand breaks (DSBs), are extremely serious damages that can cause loss of genetic material, genetic recombination, and lead to cancer or cell death. Eukaryotic cells have evolved a variety of mechanisms to deal with the serious threat of DNA double-strand breaks, which are the DNA damage response mechanism (DDR), which mainly include DNA damage detection, signal transduction, and damage repair. DNA double-strand break repair mainly includes homologous recombination (HR) repair and non-homologous end joining (NHEJ) repair. In higher eukaryotes, NHEJ repair, preferentially used during early G1/S phase, is the main mechanism. DDR initial damage factors such as MRN detect and identify the damage site, recruit members of the phosphatidylinositol kinase family (ATM, ATR, DNA-PK), phosphorylate H2AX to promote the formation of γH2AX, guide downstream signal transduction and recruit related proteins to complete the repair of damaged DNA.

DNA-PK catalytic subunit (DNA-PKcs), belonging to the phosphoinositide-3-kinaserelated protein (PI3K-related kinase, PIKK) family, is mainly for the repair of non-homologous end joining (NHEJ) of DNA double strand breaks, and is an important member of DNA damage repair. During the repair of DNA double-strand damage, the Ku70/Ku80 heterodimer specifically connects to the double-strand damage site through a pre-formed channel to identify double-strand breaks and bind to the ends of the breaks respectively. Then, the ATPdependent manner is used to slide a distance along the DNA chain to both ends to form KU-DNA complexes and recruit DNA-PKcs to bind to the double-strand break sites. Subsequently, Ku dimer moves inward to activate DNA-PKcs and make them selfphosphorylated. Finally, phosphorylated DNA-PKcs guides damage signal transduction and recruits DNA end processing-related proteins such as PNKP, XRCC4, XLF, Pol X, and DNA ligase IV to participate in double-strand break repair.

At present, the main mechanisms of DNA damaging chemotherapeutic drugs (such as bleomycin, topoisomerase II inhibitors such as etoposide and doxorubicin) and radiotherapy commonly used in tumor therapy are to cause fatal double-strand breaks of DNA molecules, and then induce the death of tumor cells. Studies have shown that high expression of DNA-PK is found in tumor tissues treated with chemoradiotherapy, and the increase of DNA-PKcs activity to a certain extent enhances the repair of damaged DNA, prevents tumor cell death, and leads to the tolerance of chemoradiotherapy. In addition, the surviving cells in the tumor tissue after chemoradiotherapy are often cells with high DNA-PKcs activity that are not sensitive to the treatment, which are also the reason for the poor curative effect and poor prognosis. Combined with chemoradiotherapy drugs, DNA-PK inhibitors can inhibit the activity of DNA-PKcs, thereby greatly reducing tumor DNA repair, inducing cells to enter the apoptosis process, and achieving better therapeutic effects.

ATM plays an important role in homologous recombination (HR) repair, and when tumor cells are deficient in ATM, DNA break repair becomes more dependent on DNA-PKcsdominated NHEJ repair for their survival. Therefore, DNA-PK inhibitors can also act as single drugs in tumors with defects in other DNA repair pathways.

The present disclosure aims to discover a DNA-PK small molecule inhibitor, which can not only be used as a single drug to treat tumors with defects in other DNA repair pathways. It can also be combined with chemoradiotherapy drugs to enhance the sensitivity of tumor tissues to chemoradiotherapy, overcome the drug resistance problem, and enhance the inhibitory effect on various solid tumors and hematological tumors. Such compounds have good activity and show excellent effects and functions, with broad prospects.

### Content of the present invention

The present disclosure provides a compound represented by formula (IV) or a pharmaceutically acceptable salt thereof, 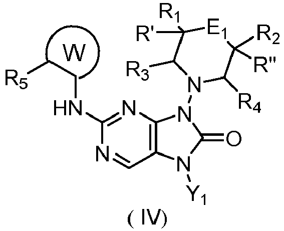 wherein,
the structural moiety 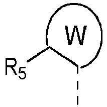 is selected from 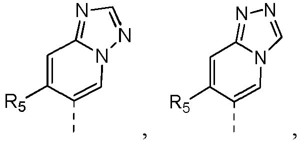 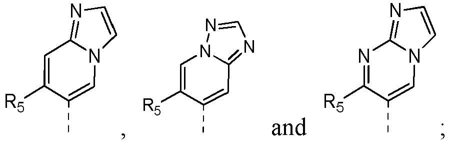
E₁ is selected from a single bond, -O- and -C(R₆R₇)-;
R₁, R₂, R₃, R₄, R' and R" are each independently selected from H, F and Cl;
or R₁ and R₂ are connected together such that the structural moiety 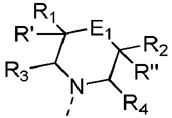 is selected from 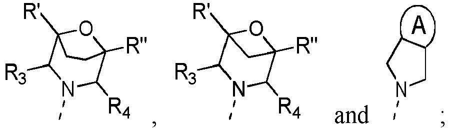
or R₃ and R₄ are connected together such that the structural moiety 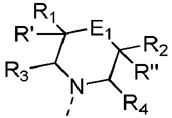 is selected from 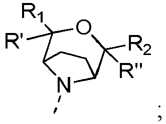
or R₁ and R₄ are connected together such that the structural moiety 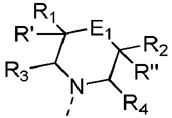 is selected from 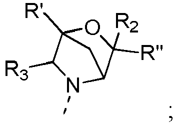
or R₂ and R" connected together with the carbon atoms to which they are attached form a C₃-₅ cycloalkyl;
R₅ is selected from F, Cl, Br, I, cyclopropyl and C₁₋₃ alkyl, and the C₁₋₃ alkyl is optionally substituted with OH or 1, 2 or 3 Rₐ;
R₆ and R₇ are each independently selected from H, F, Cl, Br, I and CN;
or R₆ and R₇ connected together with the carbon atoms to which they are attached form a cyclopropyl or a 4-membered oxetanyl;
ring A is selected from C₃-₅ cycloalkyl;
Y₁ is selected from cyclopropyl and C₁₋₃ alkyl, and the C₁₋₃ alkyl is optionally substituted with 1, 2, 3, 4 or 5 F;
Rₐ is selected from H, F, Cl, Br and I.

The present disclosure provides a compound represented by formula (III) or a pharmaceutically acceptable salt thereof, 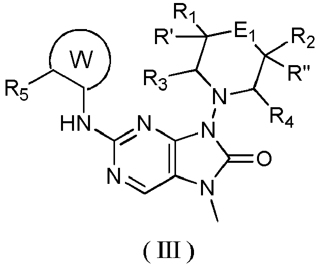 wherein,
the structural moiety 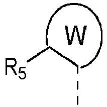 is selected from 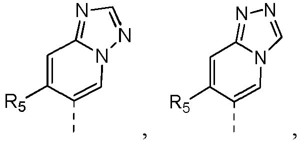 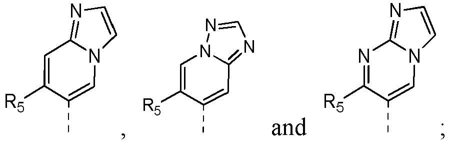
E₁ is selected from a single bond, -O- and -C(R₆R₇)-;
R₁, R₂, R₃, R₄, R' and R" are each independently selected from H, F and Cl;
or R₁ and R₂ are connected together such that the structural moiety 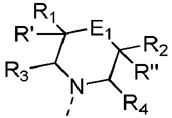 is selected from 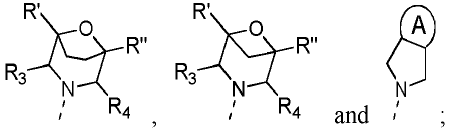
or R₃ and R₄ are connected together such that the structural moiety 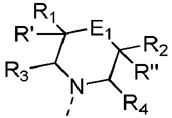 is selected from 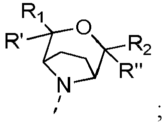
or R₁ and R₄ are connected together such that the structural moiety 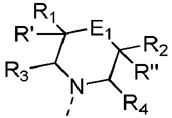 is selected from 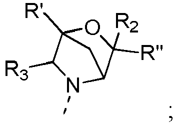
or R₂ and R" connected together with the carbon atoms to which they are attached form a C₃-₅ cycloalkyl;
R₅ is selected from F, Cl, Br, I, cyclopropyl and C₁₋₃ alkyl, and the C₁₋₃ alkyl is optionally substituted with OH or 1, 2 or 3 Rₐ;
R₆ and R₇ are each independently selected from H, F, Cl, Br, I and CN;
or R₆ and R₇ connected together with the carbon atoms to which they are attached form a cyclopropyl or a 4-membered oxetanyl;
ring A is selected from C₃-₅ cycloalkyl;
Rₐ is selected from H, F, Cl, Br and I.

The present disclosure provides a compound represented by formula (II) or a pharmaceutically acceptable salt thereof, 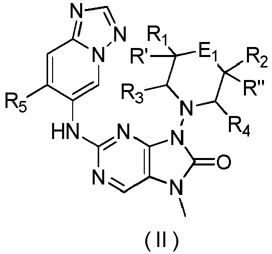 wherein,
E₁ is selected from a single bond, -O- and -C(R₆R₇)-;
R₁, R₂, R₃, R₄, R' and R" are each independently selected from H, F and Cl;
or R₁ and R₂ are connected together such that the structural moiety 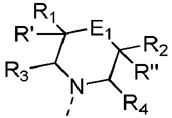 is selected from 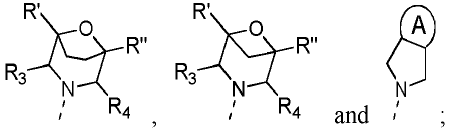
or R₃ and R₄ are connected together such that the structural moiety 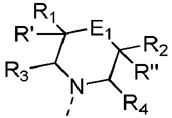 is selected from 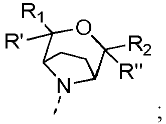
or R₁ and R₄ are connected together such that the structural moiety 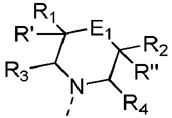 is selected from 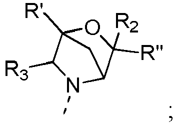
or R₂ and R" connected together with the carbon atoms to which they are attached form a C₃-₅ cycloalkyl;
R₅ is selected from F, Cl, Br, I, cyclopropyl and C₁₋₃ alkyl, and the C₁₋₃ alkyl is optionally substituted with OH or 1, 2 or 3 Rₐ;
R₆ and R₇ are each independently selected from H, F, Cl, Br, I and CN;
or R₆ and R₇ connected together with the carbon atoms to which they are attached form a cyclopropyl or a 4-membered oxetanyl;
ring A is selected from C₃-₅ cycloalkyl;
Rₐ is selected from H, F, Cl, Br, I.

The present disclosure provides a compound represented by formula (I) or a pharmaceutically acceptable salt thereof 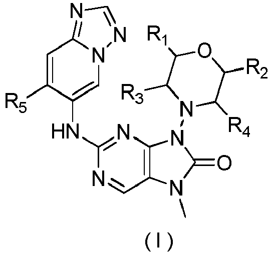 wherein,
R₁, R₂, R₃ and R₄ are each independently selected from H, F and Cl;
or R₁ and R₂ are connected together such that the structural moiety 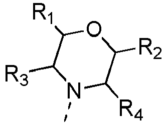 is 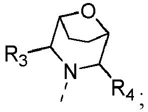
or R₃ and R₄ are connected together such that the structural moiety 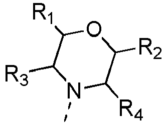 is 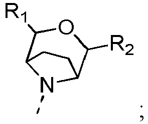
R₅ is selected from F, Cl, Br, I, cyclopropyl and C₁₋₃ alkyl, and the C₁₋₃ alkyl is optionally substituted with OH or 1, 2 or 3 Rₐ;
Rₐ is selected from H, F, Cl, Br, I.

In some embodiments of the present disclosure, the compound is selected from 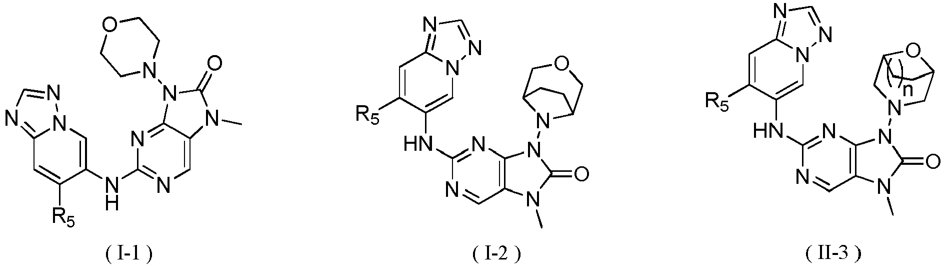 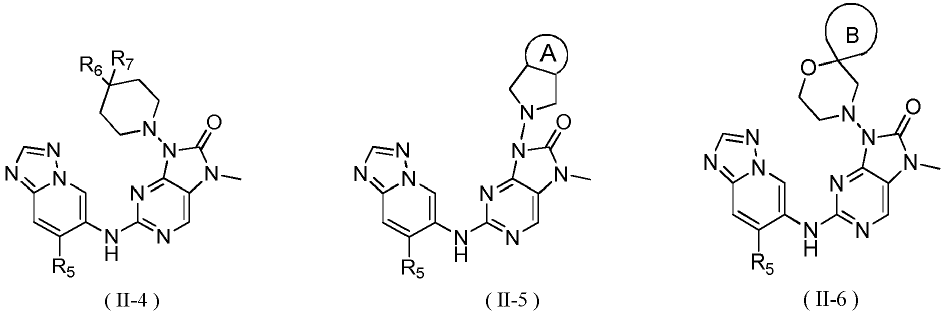 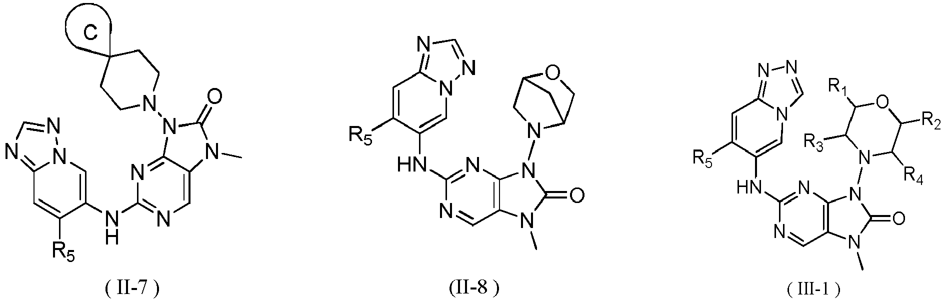 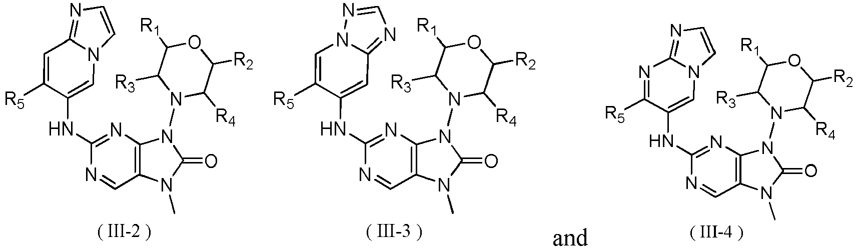 wherein, ring B is selected from C₃-₅ cycloalkyl;
ring C is cyclopropyl or 4-membered oxetanyl;
n is selected from 0 and 1;
rings A, R₁, R₂, R₃, R₄, R₅, R₆ and R₇ are as defined in the present disclosure.

In some embodiments of the present disclosure, the compound is selected from 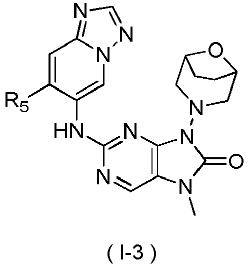 wherein, R₅ is as defined in the present disclosure.

In some embodiments of the present disclosure, the R₁ and R₂ are connected together such that the structural moiety 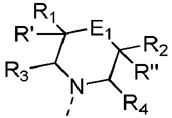 is selected from 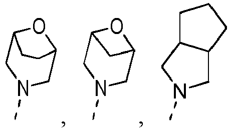 and 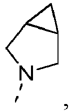 and other variables are as defined in the present disclosure.

In some embodiments of the present disclosure, the R₃ and R₄ are connected together such that the structural moiety 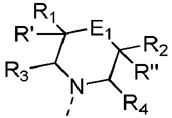 is selected from 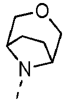 , and other variables are as defined in the present disclosure.

In some embodiments of the present disclosure, the R₁ and R₄ are connected together such that the structural moiety 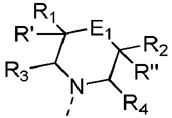 is selected from 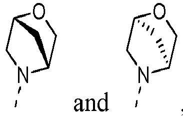 , and other variables are as defined in the present disclosure.

In some embodiments of the present disclosure, the ring A is selected from 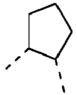 and 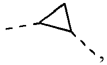 and other variables are as defined in the present disclosure.

In some embodiments of the present disclosure, the R₆ and R₇ connected together with the carbon atoms to which they are attached form 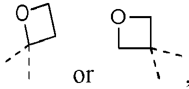 , and other variables are as defined in the present disclosure.

In some embodiments of the present disclosure, R₂ and R" connected together with the carbon atoms to which they are attached form 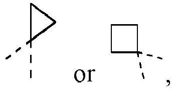 and other variables are as defined in the present disclosure.

In some embodiments of the present disclosure, the R₁ and R₂ are connected together such that the structural moiety 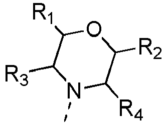 is 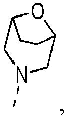 and other variables are as defined in the present disclosure.

In some embodiments of the present disclosure, the R₃ and R₄ are connected together such that the structural moiety 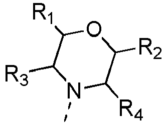 is 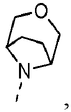 and other variables are as defined in the present disclosure.

In some embodiments of the present disclosure, the structural moiety 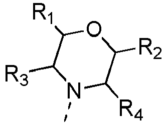 is selected from 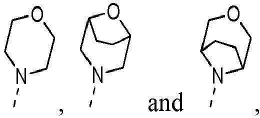 and other variables are as defined in the present disclosure.

In some embodiments of the present disclosure, the R₅ is selected from F, Cl, CH₂OH, CF₃ and CH₃, and other variables are as defined in the present disclosure.

In some embodiments of the present disclosure, the R₅ is selected from F, Cl and CH₃, and other variables are as defined in the present disclosure.

There are also some embodiments of the present disclosure that come from any combination of the above-mentioned variables.

The present disclosure also provides a compound represented by the following formula or a pharmaceutically acceptable salt thereof

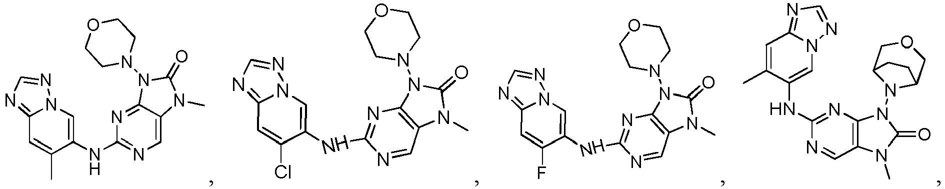

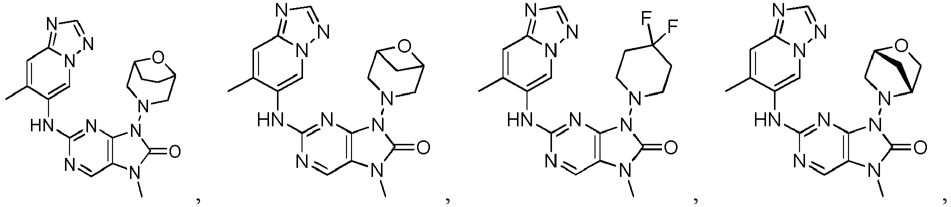

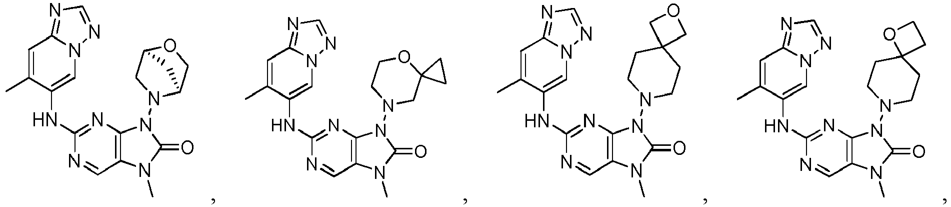

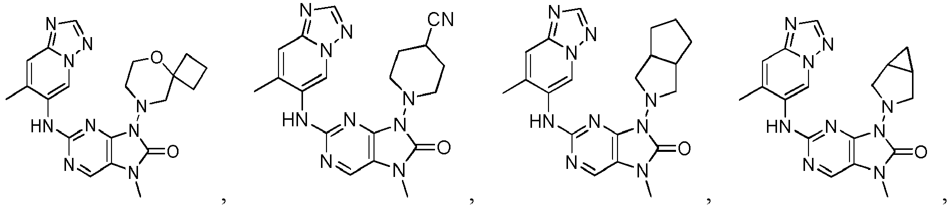

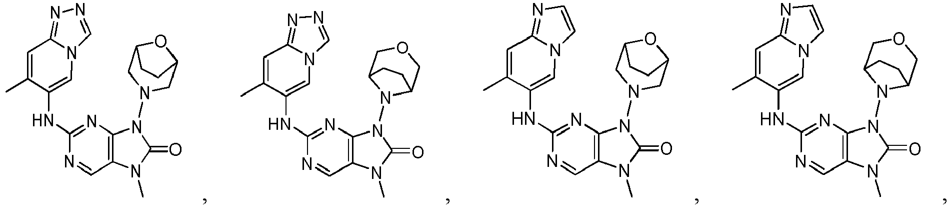

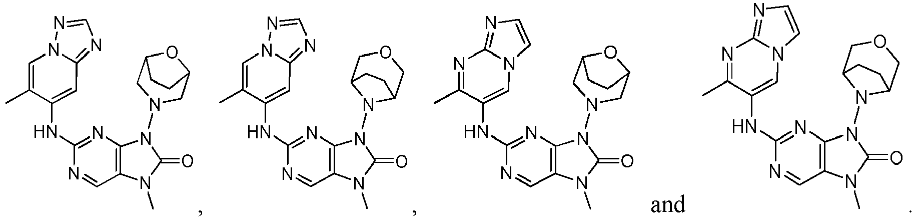

In some embodiments of the present disclosure, a use of the compound or the pharmaceutically acceptable salt thereof in the manufacture of a medicament related to DNA-PK inhibitor.

In some embodiments of the present disclosure, the medicament related to DNA-PK inhibitor plays a therapeutic effect as a single medicament in tumors with defects in other DNA repair pathways.

In some embodiments of the present disclosure, the medicament related to DNA-PK inhibitor is used in combination with a chemoradiotherapy medicament to enhance the inhibitory effect on solid tumors and hematological tumors.

### Technical effect

As a class of DNA-PK inhibitors, the compounds of the present disclosure exhibit significant DNA-PK kinase inhibitory activity. The PK results show that the compounds of the present disclosure have a longer half-life, a lower clearance rate and a higher drug exposure, and have excellent pharmacokinetic properties, which are good candidate molecules that can be developed for oral administration. The *in vivo* pharmacodynamic results show that the compounds of the present disclosure have significant antitumor effect.

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)-and (+)-enantiomers, (*R*)-and (*S*)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 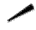 ) and a wedged dashed bond ( 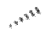 ), and the relative configuration of a stereogenic center is represented by a straight solid bond ( 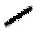 ) and a straight dashed bond ( 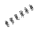 ), a wave line ( 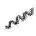 ) is used to represent a wedged solid bond ( 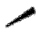 ) or a wedged dashed bond ( 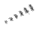 ), or the wave line ( 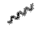 ) is used to represent a straight solid bond ( 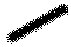 ) or a straight dashed bond ( 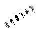 ).

Unless otherwise specified, the terms "enriched in one isomer", "enriched in isomers", "enriched in one enantiomer" or "enriched in enantiomers" refer to the content of one of the isomers or enantiomers is less than 100 %, and the content of the isomer or enantiomer is greater than or equal to 60 %, or greater than or equal to 70 %, or greater than or equal to 80 %, or greater than or equal to 90 %, or greater than or equal to 95 %, or greater than or equal to 96 %, or greater than or equal to 97 %, or greater than or equal to 98 %, or greater than or equal to 99 %, or greater than or equal to 99.5 %, or greater than or equal to 99.6 %, or greater than or equal to 99.7 %, or greater than or equal to 99.8 %, or greater than or equal to 99.9 %.

Unless otherwise specified, the term "isomer excess" or "enantiomeric excess" refers to the difference between the relative percentages of two isomers or two enantiomers. For example, if the content of one isomer or enantiomer is 90 %, and the content of the other isomer or enantiomer is 10 %, the isomer or enantiomer excess (ee value) is 80 %.

Optically active (R)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond.

When a substituent is 0, it means that the substituent does not exist, for example, -A-(R)o means that the structure is actually -A.

When a substituent is vacant, it means that the substituent does not exist, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such a substituent can be bonded to any atom on the ring, for example, a structural moiety 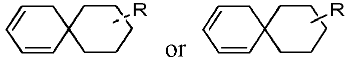 means that R can substitute on any position of cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 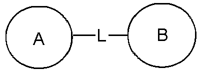 is -M-W-, then -M-W- can link ring A and ring B to form 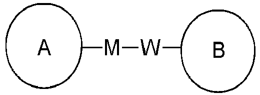 in the direction same as left-to-right reading order, and form 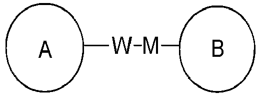 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of chemical bond linking thereto so as to meet the corresponding valence. The chemical bond between the site and other groups can be represented by a straight solid bond 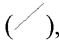 a straight dashed bond 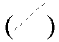 or a wavy line 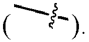 For example, the straight solid bond in -OCH₃ means that it is linked to other groups through the oxygen atom in the group; the straight dashed bonds in 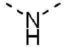 means that it is linked to other groups through the two ends of nitrogen atom in the group; the wave lines in 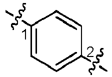 means that the phenyl group is linked to other groups through carbon atoms at position 1 and position 2; 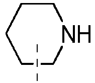 means that it can be linked to other groups through any linkable sites on the piperidinyl by one chemical bond, including at least four types of linkage, including 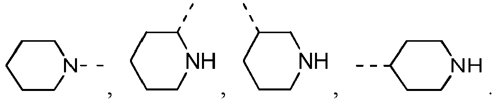 Even though the H atom is drawn on the -N-, 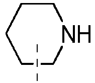 still includes the linkage of 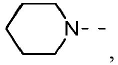 merely when one chemical bond was connected, the H of this site will be reduced by one to the corresponding monovalent piperidinyl.

Unless otherwise specified, the number of atoms in a ring is generally defined as the number of ring members, eg, "5- to 7- membered ring" refers to a "ring" of 5-7 atoms arranged around it.

Unless otherwise specified, the term "C₁₋₃ alkyl" refers to a linear or branched saturated hydrocarbon group composed of 1 to 3 carbon atoms. The C₁₋₃ alkyl group includes C₁₋₂ and C₂₋₃ alkyl groups and the like; it can be monovalent (such as methyl), divalent (such as methylene) or multivalent (such as methine). Examples of C₁₋₃ alkyl include, but are not limited to methyl (Me), ethyl (Et), propyl (including *n*-propyl and isopropyl), etc.

Unless otherwise specified, "C₃₋₅ cycloalkyl" refers to a saturated cyclic hydrocarbon group composed of 3 to 5 carbon atoms, which is a monocyclic ring system, and the C₃-₅ cycloalkyl includes C₃₋₄ and C₄₋₅ cycloalkyl, etc.; it can be monovalent, divalent or multivalent. Examples of C₃-₅ alkoxy include, but are not limited to, cyclopropyl, cyclobutyl, cyclopentyl, etc.

Unless otherwise specified, Cn-n+m or Cn-Cn+m includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The solvent used in the present disclosure is commercially available.

The present disclosure uses the following abbreviations: eq stands for equivalent; DMSO stands for dimethyl sulfoxide; ATP stands for adenosine triphosphate, EDTA stands for ethylenediaminetetraacetic acid; DNA stands for deoxyribonucleic acid; PEG stands for polyethylene glycol; Balb/c stands for mouse strain.

The compounds of the present disclosure are named according to the conventional naming principles in the art or by ChemDraw^{®} software, and the commercially available compounds use the supplier catalog names.

### Brief description of the drawings

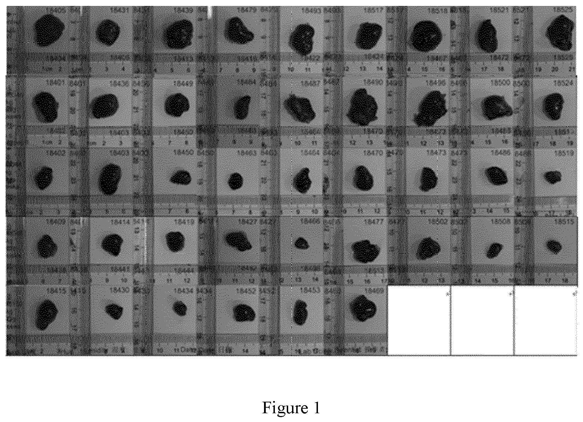

Figure 1. Tumor photographs of day 21 of an *in vivo* pharmacodynamic study of human NCI-H1703 non-small cell lung cancer.

### Detailed description of the preferred embodiment

The present disclosure will be specifically described below by way of embodiments, but the scope of the present disclosure is not limited thereto. The present disclosure has been described in detail herein, wherein specific embodiments thereof are also disclosed, for those skilled in the art, it is obvious that various changes and improvements can be made to the specific embodiments of the present disclosure without departing from the spirit and scope of the present disclosure.

### Embodiment 1

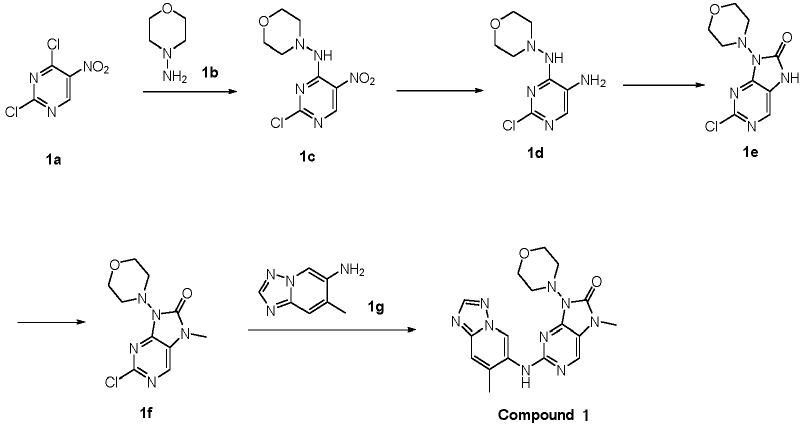

### Step 1

At 0 °C, compound **1b** (1.84 g, 18.0 mmol, 1.2 *eq)* was added to a solution of compound **1a** (2.91 g, 15.0 mmol, 1 *eq)* in 1,4-dioxane (50 mL), and after the addition was completed, the reaction was carried out at 0 °C for 8 hours. After the reaction was completed, the reaction solution was concentrated under reduced pressure and purified by column chromatography (ethyl acetate: petroleum ether=0:1-1:3) to obtain compound **1c**. MS: m/z. 259.8 [M+H]⁺.

¹H NMR (400 MHz, CDCl₃) δ ppm 9.08 (s, 1H), 9.06 (s, 1H), 3.84-4.06 (m, 4H), 2.93-3.11 (m, 4H).

### Step 2

Iron powder (1.18 g, 21.18 mmol, 5 *eq)* and ammonium chloride (1.13 g, 21.18 mmol, *5 eq)* were added sequentially to a mixed solution of compound **1c** (1.1 g, 4.24 mmol, 1 *eq)* in ethanol (10 mL) and water (10 mL), and after the addition was completed, the reaction was carried out at 80 °C for 1 hour. After the reaction was completed, the reaction mixture was filtered through celite, and the filtrate was concentrated under reduced pressure to obtain a crude product. The crude product was diluted with water (50 mL), extracted with ethyl acetate (50 mL^{∗}2), washed with saturated brine (50 mL), dried over anhydrous sodium sulfate, and filtered, and the filtrate was concentrated under reduced pressure to obtain a crude product of compound **1d.** MS: *m*/*z.* 229.9 [M+H]⁺.

### Step 3

*N,N'*-Carbonyldiimidazole (0.97 g, 6.0 mmol, 1.22 mL, 2 *eq)* was added to a solution of compound **1d** (0.69 g, 3.0 mmol, 1 *eq)* in acetonitrile (15 mL), and after the addition was completed, the reaction was carried out at 80 °C for 1 hour. After the reaction was completed, the reaction solution was concentrated under reduced pressure and purified by column chromatography (ethyl acetate: petroleum ether=0:1-4:1) to obtain compound **1e**. MS: *m*/*z* 255.9 [M+H]⁺.

¹H NMR (400 MHz, CDCl₃) δ: 8.09 (s, 1H), 3.86-3.97 (m, 4H), 3.45-3.65 (m, 4H).

### Step 4

Cesium carbonate (3.5 g, 10.76 mmol, 5 *eq)* and iodomethane (1.06 g, 7.45 mmol, 465 µL, 3 *eq)* were added sequentially to a solution of compound **1e** (1.1 g, 2.15 mmol, 1 *eq)* in *N,N-*dimethylformamide (50 mL), and after the addition was completed, the reaction was carried out at 20 °C for 1 hour. After the reaction was completed, the reaction mixture was diluted with water (50 mL), extracted with ethyl acetate (50 mL^{∗}3), washed with saturated brine (50 mL), dried over anhydrous sodium sulfate, filtered, and the filtrate was concentrated under reduced pressure and purified by column chromatography (ethyl acetate: petroleum ether = 0:1-1:1) to obtain compound **1f.** MS: m/z 269.8 [M+H]⁺.

### Step 5

A solution of compound **1f** (0.13 g, 500 µmol, 1 *eq),* compound **1g** (188.9 mg, 600.00 µmol, 1.2 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (90.6 mg, 100.00 µmol, 0.2 *eq)* and cesium carbonate (325.8 mg, 1.00 mmol, 2 *eq)* in dioxane (5 mL) and water (0.5 mL) was replaced with nitrogen three times, and the reaction was carried out at 100 °C for 16 hours under nitrogen protection. After the reaction was completed, the reaction mixture was filtered with celite, and concentrated under reduced pressure to obtain a crude product. The crude product was purified by preparative high performance liquid chromatography (Welch Xtimate C18 150^{∗}25 mm^{∗}5 µm; mobile phase: [water (0.225 % formic acid)-acetonitrile]; B (acetonitrile) %: 8 %-38 %, 8 minutes) to obtain compound 1. MS: m/z 382.2 [M+H].

¹H NMR (400 MHz, CDCl₃) δ ppm 9.81 (s, 1H), 8.27 (s, 1H), 7.94 (s, 1H), 7.58 (s, 1H), 6.80 (s, 1H), 3.96 (t, *J* =4.6 Hz, 4H), 3.47-3.55 (m, 4H), 3.42 (s, 3H), 2.53 (s, 3H).

### Embodiment 2

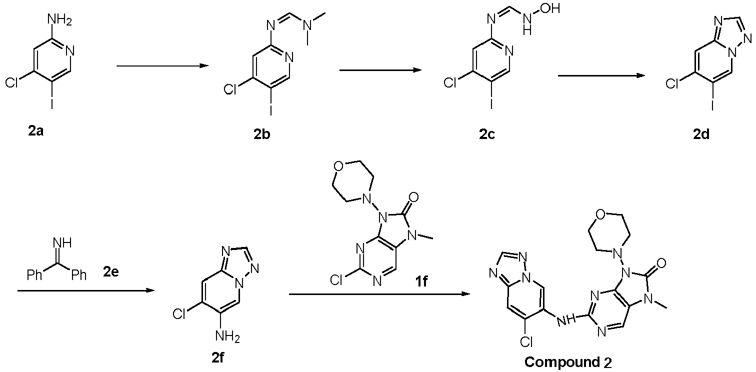

### Step 1

*N,N*-Dimethylformamide dimethyl acetal (14.30 g, 120 mmol, 15.94 mL, 3 *eq)* was added to a solution of compound 2a (10.18 g, 40 mmol, 1 *eq)* in toluene (80 mL), and after the addition was completed, the reaction solution was reacted at 110 °C for 4 hours. After the reaction was completed, the reaction solution was concentrated under reduced pressure to obtain a crude product of compound **2b.** MS: *m*/*z* 309.8 [M+H]⁺.

### Step 2

Hydroxylamine hydrochloride (5.56 g, 80 mmol, 2 *eq)* was added to a solution of compound **2b** (12.38 g, 40 mmol, 1 *eq)* in methanol (100 mL), and after the addition was completed, the reaction solution was reacted at 70 °C for 2 hours. After the reaction was completed, the reaction solution was concentrated under reduced pressure to obtain a crude product of compound **2c.** MS: *m*/*z* 297.7 [M+H]⁺.

### Step 3

At 0 °C, trifluoroacetic anhydride (12.60 g, 60 mmol, 8.35 mL, 1.5 *eq)* was added to a solution of compound **2c** (11.90 g, 40 mmol, 1 *eq)* in tetrahydrofuran (100 mL), and after the addition was completed, the reaction solution was reacted at 25 °C for 12 hours. After the reaction was completed, the reaction solution was concentrated under reduced pressure to remove the solvent. The concentrated reaction solution was diluted with water (100 mL), extracted with 300 mL of ethyl acetate (100 mL^{∗}3), washed with 30 mL of saturated brine, dried over anhydrous sodium sulfate, filtered, and the filtrate was concentrated under reduced pressure and purified by column chromatography (ethyl acetate: petroleum ether=0:1-1:1) to obtain compound **2d.** MS: *m*/*z* 279.7 [M+H]⁺.

¹H NMR (400 MHz, DMSO-*d*₆) δ ppm 9.55 (s, 1H), 8.51 (s, 1H), 8.26 (s, 1H).

### Step 4

Compound **2d** (3.91 g, 14 mmol, 1 *eq),* compound **2e** (2.79 g, 15.40 mmol, 1.1 *eq),* tris(dibenzylideneacetone)dipalladium (641 mg, 700 µmol, 0.05 *eq),* 4,5-bis(diphenylphosphino)-9,9-dimethylxanthene (810.1 mg, 1.4 mmol, 0.1 *eq)* and cesium carbonate (9.12 g, 28 mmol, 2 *eq)* were placed in a reaction flask, and the reaction flask was vacuumized and replaced with nitrogen three times, and then anhydrous *N,N-*dimethylformamide (30 mL) was added to the mixture and the reaction was carried out at 80 °C for 6 hours. After the reaction was completed, the reaction solution was filtered through celite and concentrated under reduced pressure to obtain a crude product. The crude product was dissolved in tetrahydrofuran (100 mL), and 3 N hydrochloric acid (20 mL) was added thereto, and the mixture was stirred at 20 °C for 0.5 hours. After the reaction was completed, water (100 mL) was added to the reaction solution. The reaction solution was extracted with ethyl acetate (100 mL^{∗}3), and the organic phase was discarded; ammonium hydroxide (30 mL) was added to the aqueous phase to adjust the pH to basic, and the aqueous phase was extracted with ethyl acetate (100 mL^{∗}3) again. The organic phase was washed with 50 mL of saturated brine, dried over anhydrous sodium sulfate, filtered, and the filtrate was concentrated under reduced pressure and purified by column chromatography (ethyl acetate: petroleum ether=0:1-1:0) to obtain compound **2f.** MS: *m*/*z* 168.8 [M+H]⁺.

¹H NMR (400 MHz, DMSO-*d*₆) δ ppm 8.28 (s, 1H), 8.26 (s, 1H), 7.96 (s, 1H), 5.35-5.43 (m, 2H).

### Step 5

Compound **2f** (67.4 mg, 400 µmol, 1 *eq),* compound **1f** (107.8 mg, 400 µmol, 1 *eq),* tris(dibenzylideneacetone)dipalladium (36.6 mg, 40 µmol, 0.1 *eq),* 4,5-bis(diphenylphosphino)-9,9-dimethylxanthene (46.3 mg, 80 µmol, 0.2 *eq)* and cesium carbonate (195.5 mg, 600 µmol, 1.5 *eq)* were placed in a reaction flask, and the reaction flask was vacuumized and repaced with nitrogen three times, and then anhydrous dioxane (8 mL) was added to the mixture and the reaction was carried out at 100 °C for 2 hours. After the reaction was completed, the reaction solution was filtered through celite and concentrated under reduced pressure to obtain a crude product. The crude product was purified by thin-layer preparative chromatography (dichloromethane: methanol=10:1) to obtain compound 2. MS: *m*/*z* 401.9 [M+H]⁺.

¹H NMR (400 MHz, CDCl₃) δ ppm 10.24 (s, 1H), 8.31 (s, 1H), 7.98 (s, 1H), 7.87 (s, 1H), 7.47 (s, 1H), 3.96 (t, J=4.69 Hz, 4H), 3.51-3.57 (m, 4H), 3.43 (s, 3H).

### Embodiment 3

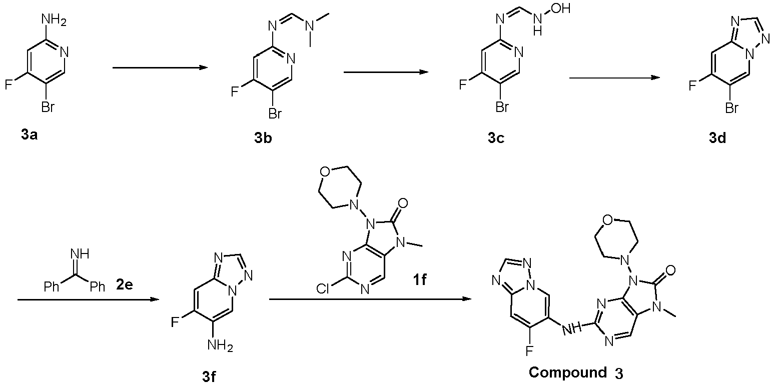

### Step 1

*N,N*-Dimethylformamide dimethyl acetal (12.54 g, 105.25 mmol, 3 *eq)* was added to a solution of compound **3a** (6.7 g, 35.08 mmol, 1 *eq)* in toluene (100 mL), and after the addition was completed, the reaction was carried out at 110 °C for 1.5 hours. After the reaction was completed, the reaction solution was concentrated under reduced pressure to obtain a crude product of compound **3b.**

### Step 2

Hydroxylamine hydrochloride (4.69 g, 67.46 mmol, 2 *eq)* was added to a solution of compound **3b** (8.3 g, 33.73 mmol, 1 *eq)* in methanol (100 mL), and the reaction was carried out at 80 °C for 1 hour. After the reaction was completed, the reaction solution was concentrated under reduced pressure to obtain a crude product of compound **3c.** MS: *m*/*z* 233.8 [M+H]⁺.

### Step 3

At 0 °C, trifluoroacetic anhydride (15.33 mL, 100.24 mmol, 2 *eq)* was added to a solution of compound **3c** (12.9 g, 55.12 mmol, 1 *eq)* in tetrahydrofuran (100 mL), and after the addition was completed, the reaction was carried out at 21 °C for 21 hours. After the reaction was completed, the crude product obtained by concentrating the reaction solution under reduced pressure was purified by column chromatography (ethyl acetate: petroleum ether=0:1-1:2) to obtain compound **3d.** MS: *m*/*z* 217.8 [M+H]⁺.

### Step 4

1,1'-Binaphthyl-2,2'-diphemyl phosphine (288.3 mg, 462.94 µmol, 0.1 *eq),* compound **2e** (922.9 mg, 5.09 mmol, 1.1 *eq),* tris(dibenzylideneacetone)dipalladium (211.9 mg, 231.47 µmol, 0.05 *eq)* and potassium *tert*-butoxide (1.04 g, 9.26 mmol, 2 *eq)* were added sequentially to a solution of compound **3d** (1 g, 4.63 mmol, 1 *eq)* in toluene (50 mL), and after the addition was completed, the reaction was carried out at 110 °C for 4 hours. After the reaction was completed, the reaction solution was concentrated under reduced pressure to remove the solvent, and the concentrated reaction solution was diluted with water (50 mL), extracted with ethyl acetate (50 mL^{∗}2), washed with saturated brine (100 mL), dried over anhydrous sodium sulfate, filtered, and the filtrate was concentrated under reduced pressure to obtain a crude product. Then, the crude product was dissolved in ethanol (20 mL), and 1 N hydrochloric acid (12 mL) was added thereto and stirred for half an hour. After the reaction was completed, the pH was adjusted to basic by adding ammonium hydroxide, and the mixture was concentrated under reduced pressure to remove the solvent and purified by column chromatography (methanol: dichloromethane= 0:1-1:6) to obtain compound **3f.**

### Step 5

Methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (21.7 mg, 20.93 µmol, 0.05 *eq)* and cesium carbonate (311.5 mg, 956.13 µmol, 2 *eq)* were added sequentially to a solution of compound **3f** (132 mg, 489.46 µmol, 1.02 *eq)* and compound **1f** (80 mg, 525.87 µmol, 1.1 *eq)* in dioxane (20 mL), and after the addition was completed, the reaction was carried out at 100 °C for 2 hours. After the reaction was completed, the crude product obtained by concentrating the reaction solution under reduced pressure was purified by column chromatography (methanol: dichloromethane=0:1-1:9) to obtain compound **3.** MS: m/z 386.0 [M+H]⁺.

¹H NMR (400 MHz, CDCl₃) δ ppm 10.15 (br d, J=7.03 Hz, 1H), 8.28 (s, 1H), 7.99 (s, 1H), 7.47 (br d, *J*=9.54 Hz, 1H), 3.96 (br s, 4H), 3.53 (br s, 4H), 3.43 (s, 3 H).

### Embodiment 4

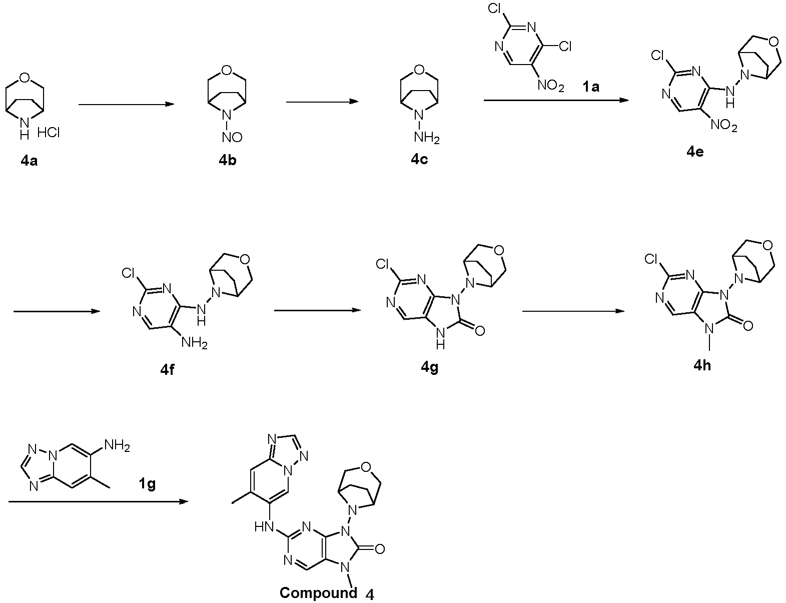

### Step 1

At 0 °C, a solution of sodium nitrite (1.52 g, 22 mmol, 1.1 *eq)* in water (3 mL) was slowly added to a mixed solution of compound **4a** (2.99 g, 20 mmol, 1 *eq,* hydrochloride) in acetic acid (30 mL) and water (9 mL), and after the addition was completed, the reaction was carried out at 20 °C for 3 hours. After the reaction was completed, the reaction solution was diluted with water (50 mL), extracted with 300 mL of ethyl acetate (100 mL^{∗}3), washed with saturated brine (50 mL), dried over anhydrous sodium sulfate, filtered, and the filtrate was concentrated under reduced pressure and purified by column chromatography (ethyl acetate: petroleum ether=0:1-1:1) to obtain compound **4b.**

¹H NMR (400 MHz, CDCl₃) δ ppm 5.08 (br d, J=7.03 Hz, 1H), 4.89 (br d, *J*=6.27 Hz, 1H), 3.77-3.93 (m, 2H), 3.54-3.68 (m, 2H), 2.05-2.22 (m, 3H), 1.78-1.94 (m, 1H).

### Step 2

At 0 °C, zinc powder (4.71 g, 72 mmol, 4 *eq)* and acetic acid (20 mL) were added sequentially to a solution of compound **4b** (2.56 g, 18 mmol, 1 *eq)* in methanol (20 mL), and after the addition was completed, the reaction solution was reacted at 20 °C for 4 hours. After the reaction was completed, the reaction solution was filtered through celite and washed with ethyl acetate (200 mL), and the filtrate was concentrated under reduced pressure to obtain a crude product of compound **4c.**

### Step 3

At 0 °C, compound **4c** (3.01 g, 16 mmol, 1 *eq,* acetate) and triethylamine (8.10 g, 80 mmol, 5 *eq,* 11.14 mL) were added sequentially to a solution of compound **1a** (6.21 g, 32 mmol, *2 eq)* in dioxane (150 mL), and after the addition was completed, the reaction solution was reacted at 20 °C for 5 hours. After the reaction was completed, the reaction solution was diluted with water (100 mL), extracted with 300 mL of ethyl acetate (100 mL^{∗}3), washed with saturated brine (50 mL), dried over anhydrous sodium sulfate, filtered, and the filtrate was concentrated under reduced pressure and purified by column chromatography (ethyl acetate: petroleum ether=0:1-1:1) to obtain compound **4e.** MS: m/z 285.9 [M+H]⁺.

¹H NMR (400MHz, CDCl₃) δ ppm 9.29 (br s, 1H), 9.04 (s, 1H), 4.02 (d, *J*=11.13 Hz, 2H), 3.64 (dd, *J*=11.32, 1.94 Hz, 2H), 3.48 (br d, *J*=2.88 Hz, 2H), 2.07-2.21 (m, 4H).

### Step 4

Iron powder (1.79 g, 32 mmol, 5 *eq)* and ammonium chloride (1.71 g, 32 mmol, 5 *eq)* were added sequentially to a mixed solution of compound **4e** (2.03 g, 6.4 mmol, 1 *eq)* in ethanol (16 mL) and water (4 mL), and after the addition was completed, the reaction solution was reacted at 75 °C for 3 hours. After the reaction was completed, the reaction solution was cooled to room temperature and diluted with ethyl acetate (300 mL), filtered through celite, and the filtrate was concentrated under reduced pressure to obtain a crude product of compound **4f.** MS: *m*/*z* 256.0 [M+H]⁺.

### Step 5

*N,N'*-Carbonyldiimidazole (1.62 g, 10 mmol, 2 *eq)* was added to a solution of compound **4f** (1.28 g, 5 mmol, 1 *eq)* in acetonitrile (20 mL), and after the addition was completed, the reaction solution was reacted at 80 °C for 2 hours. After the reaction was completed, the crude product obtained by concentrating the reaction solution under reduced pressure was purified by column chromatography (ethyl acetate: petroleum ether=0:1-1:0) and slurrying (methanol/dichloromethane: 2 mL/10 mL, 25 °C, 15 minutes) to obtain compound **4g.** MS: *m*/*z* 281.2 [M+H]⁺.

¹H NMR (400 MHz, DMSO-*d*₆) δ ppm 11.63 (br s, 1H), 8.09 (s, 1H), 3.76-3.86 (m, 4H), 3.59 (br d, J=8.63 Hz, 2H), 2.27 -2.36 (m, 2H), 1.90-1.99 (m, 2H).

### Step 6

Cesium carbonate (0.489 g, 1.5 mmol, 1.5 *eq)* and iodomethane (0.177 g, 1.25 mmol, 1.25 *eq)* were added sequentially to a solution of compound **4g** (0.282 g, 1 mmol, 1 *eq)* in *N,N-*dimethylformamide (10 mL), and after the addition was completed, the reaction solution was reacted at 21 °C for 4 hours. After the reaction was completed, the reaction solution was diluted with water (20 mL), extracted with 90 mL of ethyl acetate (30 mL^{∗}3), washed with saturated brine (20 mL), dried over anhydrous sodium sulfate, filtered, and the filtrate was concentrated under reduced pressure and purified by column chromatography (ethyl acetate: petroleum ether=0:1-4:1) to obtain compound **4h.** MS: *m*/*z* 295.9 [M+H]⁺.

¹H NMR (400 MHz, CDCl₃) δ ppm 7.99 (s, 1H), 4.10 (d, *J*=10.51 Hz, 2H), 3.83-3.91 (m, 2H), 3.68 (dd, *J*=10.63, 2.00 Hz, 2H), 3.42 (s, 3H), 2.39-2.47 (m, 2H), 2.12-2.20 (m, 2H).

### Step 7

Compound **4h** (221.8 mg, 0.75 mmol, 1 *eq),* compound **1g** (88.9 mg, 0.6 mmol, 0.8 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (136 mg, 150 µmol, 0.2 *eq)* and cesium carbonate (366.6 mg, 1.13 mmol, 1.5 *eq)* were placed in a reaction flask, and the reaction flask was vacuumized and repaced with nitrogen three times, then anhydrous dioxane (20 mL) was added to the mixture, and the reaction was carried out at 100 °C for 3 hours. After the reaction was completed, the reaction solution was filtered through celite and concentrated under reduced pressure to obtain a crude product, then the crude product was purified by column chromatography (methanol: dichloromethane=0:1-1:9) and slurrying (dichloromethane/ethyl acetate: 3 mL/3 mL, 25 °C, 15 minutes) to obtain compound **4.** MS: *m*/*z* 408.2 [M+H]⁺.

¹H NMR (400 MHz, CDCl₃) δ ppm 9.77 (s, 1H), 8.26 (s, 1H), 7.89 (s, 1H), 7.57 (s, 1H), 6.72 (s, 1H), 4.15 (d, *J*=10.54 Hz, 2H), 3.84-3.90 (m, 2H), 3.71 (br d, *J*=10.54 Hz, 2H), 3.39 (s, 3H), 2.51 (s, 3H), 2.37-2.46 (m, 2H), 2.09-2.18 (m, 2H).

### Embodiment 5

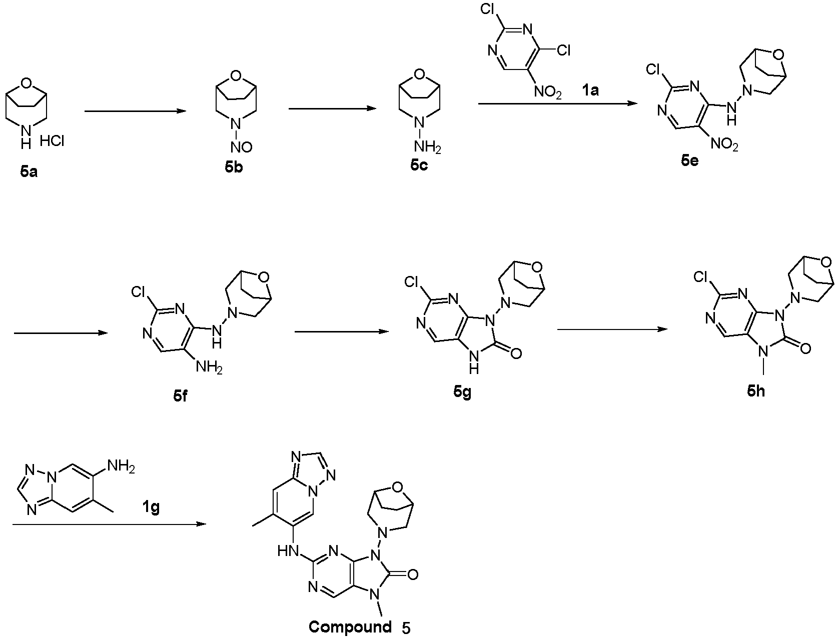

### Step 1

At 0 °C, a solution of sodium nitrite (2.28 g, 33 mmol, 1.1 *eq)* in water (4.5 mL) was slowly added to a mixed solution of compound **5a** (4.49 g, 30 mmol, 1 *eq,* hydrochloride) in acetic acid (50 mL) and water (18 mL), and after the addition was completed, the reaction solution was reacted at 20 °C for 3 hours. After the reaction was completed, the reaction solution was diluted with water (50 mL), extracted with 150 mL of ethyl acetate (50 mL^{∗}3), and the organic phase was washed with saturated brine (30 mL), dried over anhydrous sodium sulfate, filtered, and the filtrate was concentrated under reduced pressure and purified by column chromatography (ethyl acetate: petroleum ether=0:1-1:1) to obtain compound 5b. MS: *m*/*z.* 143.0 [M+H]⁺.

### Step 2

At 0 °C, zinc powder (6.80 g, 104 mmol, 4 *eq)* and acetic acid (20 mL) were added sequentially to a solution of compound **5b** (3.70 g, 26 mmol, 1 *eq)* in methanol (20 mL), and after the addition was completed, the reaction solution was reacted at 20 °C for 4 hours. After the reaction was completed, the reaction solution was filtered through celite and washed with ethyl acetate (200 mL), and the filtrate was concentrated under reduced pressure to obtain a crude product of compound **5c.**

### Step 3

At 0 °C, compound **5c** (4.89 g, 26 mmol, 1 *eq)* and triethylamine (13.15 g, 130 mmol, *5 eq,* 18.09 mL) were added sequentially to a solution of compound **1a** (10.09 g, 52 mmol, 2 *eq*) in dioxane (150 mL), and after the addition was completed, the reaction solution was reacted at 20 °C for 5 hours. After the reaction was completed, the reaction solution was diluted with water (100 mL), extracted with 300 mL of ethyl acetate (100 mL^{∗}3), and the organic phase was washed with saturated brine (50 mL), dried over anhydrous sodium sulfate, filtered, and the filtrate was concentrated under reduced pressure and purified by column chromatography (ethyl acetate: petroleum ether=0:1-1:1) to obtain compound **5e.** MS: *m*/*z* 285.9 [M+H]⁺.

¹H NMR (400 MHz, CDCl₃) δ ppm 9.05 (s, 1H), 8.97 (br s, 1H), 4.43 (br dd, J=4.44, 2.06 Hz, 2H), 2.94-3.06 (m, 4H), 2.19-2.28 (m, 2H), 1.90-2.03 (m, 2H).

### Step 4

Iron powder (2.37 g, 42.5 mmol, 5 *eq)* and ammonium chloride (2.27 g, 42.5 mmol, 5 *eq)* were added sequentially to a mixed solution of compound **5e** (2.43 g, 8.5 mmol, 1 *eq)* in ethanol (120 mL) and water (30 mL), and after the addition was completed, the reaction solution was reacted at 75 °C for 3 hours. After the reaction was completed, the reaction solution was cooled to room temperature and diluted with ethyl acetate (200 mL), filtered through celite and concentrated under reduced pressure to obtain a crude product of compound **5f.** MS: *m*/*z* 256.0 [M+H]⁺.

### Step 5

*N,N'*-Carbonyldiimidazole (2.76 g, 17 mmol, 2 *eq)* was added to a solution of compound **5f** (2.17 g, 8.5 mmol, 1 *eq)* in acetonitrile (30 mL), and after the addition was completed, the reaction solution was reacted at 80 °C for 2 hours. After the reaction was completed, the reaction solution was concentrated under reduced pressure and purified by column chromatography (ethyl acetate: petroleum ether=0:1-1:0) to obtain compound **5g.** MS: *m*/*z* 281.9 [M+H]⁺.

¹H NMR (400 MHz, DMSO-*d*₆) δ ppm 11.61 (brs, 1H), 8.12(s, 1H), 4.38 (brd, *J*=2.01 Hz, 2H), 3.73 (dd, J=9.91, 1.63 Hz, 2H), 2.81 (d, *J*=9.54 Hz, 2H), 1.99-2.09 (m, 2H), 1.78-1.87 (m, 2H).

### Step 6

Cesium carbonate (2.15 g, 6.6 mmol, 1.5 *eq)* and iodomethane (780 mg, 5.5 mmol, 1.25 *eq)* were added sequentially to a solution of compound **5g** (1.24 g, 4.4 mmol, 1 *eq)* in *N,N-*dimethylformamide (40 mL), and after the addition was completed, the reaction solution was reacted at 21 °C for 4 hours. After the reaction was completed, the reaction solution was diluted with water (50 mL), extracted with 180 mL of ethyl acetate (60 mL^{∗}3), and the organic phase was washed with saturated brine (50 mL), dried over anhydrous sodium sulfate, filtered, and the filtrate was concentrated under reduced pressure and purified by column chromatography (ethyl acetate: petroleum ether=0:1-4:1) to obtain compound 5h. MS: *m*/*z* 295.9 [M+H]⁺.

¹H NMR (400 MHz, CDCl₃) δ ppm 7.98-8.05 (m, 1H), 4.45 (br d, J=2.25 Hz, 2H), 3.99 (dd, *J*=9.69, 1.81 Hz, 2H), 3.41 (s, 3H), 2.80 (br d, *J*=9.51 Hz, 2H), 2.23-2.31 (m, 2H), 1.94-2.04 (m, 2H).

### Step 7

Compound **5h** (502.7 mg, 1.7 mmol, 1 *eq),* compound **1g** (201.5 mg, 1.36 mmol, 0.8 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (231.2 mg, 255µmol, 0.15 *eq)* and cesium carbonate (830.8 mg, 2.55 mmol, 1.5 *eq)* were placed in a reaction flask, and the reaction flask was vacuumized and repaced with nitrogen three times, then anhydrous dioxane (30 mL) was added to the mixture and the reaction was carried out at 100 °C for 3 hours. After the reaction was completed, the reaction solution was filtered through celite and concentrated under reduced pressure to obtain a crude product, then the crude product was purified by column chromatography (methanol/dichloromethane: 0-10 %) and slurrying (dichloromethane/ethyl acetate: 1.5 mL/3 mL, 25 °C, 15 min) to obtain compound 5. MS: *m*/*z* 408.2 [M+H]⁺.

¹H NMR (400 MHz, CDCl₃) δ ppm 9.87 (s, 1H), 8.26 (s, 1H), 7.91 (s, 1H), 7.57 (s, 1H), 6.76 (s, 1H), 4.48 (br d, *J*=2.25 Hz, 2H), 4.04 (dd, J=9.76, 1.88 Hz, 2H), 3.40 (s, 3H), 2.85 (d, *J*=9.51 Hz, 2H), 2.53 (s, 3H) 2.29-2.37 (m, 2H), 2.00-2.10 (m, 2H).

### Embodiment 6

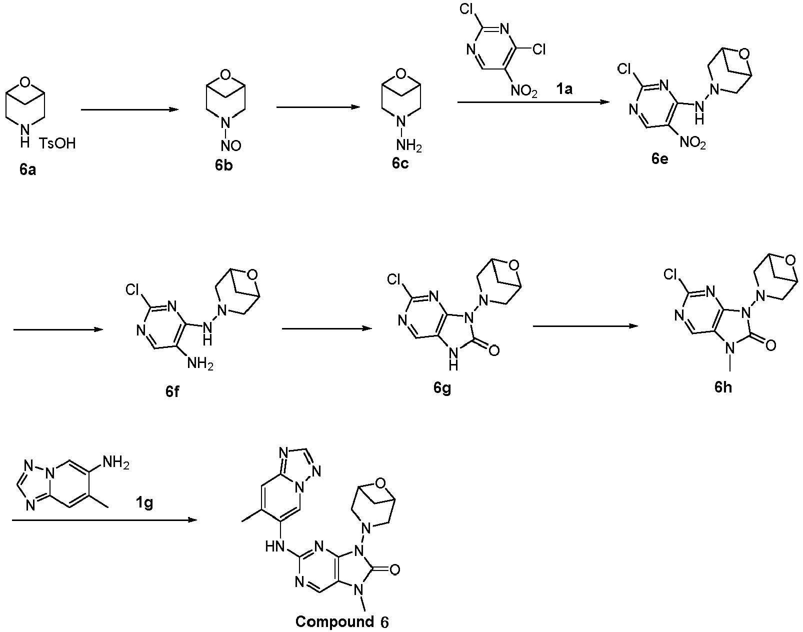

### Step 1

At 0 °C, a solution of sodium nitrite (97.90 mg, 1.42 mmol, 1.1 *eq)* in water (1 mL) was slowly added to a mixed solution of compound **6a** (350 mg, 1.29 mmol, 1 *eq, p-*toluenesulfonate) in acetic acid (5 mL) and water (1 mL), and after the addition was completed, the reaction solution was reacted at 20 °C for 3 hours. After the reaction was completed, the reaction solution was diluted with water (30 mL), extracted with 60 mL of ethyl acetate (20 mL^{∗}3), washed with saturated brine (20 mL), dried over anhydrous sodium sulfate, filtered, and the filtrate was concentrated under reduced pressure and purified by column chromatography (ethyl acetate: petroleum ether=0:1-1:1) to obtain compound **6b.**

¹H NMR (400 MHz, CDCl₃) δ ppm 4.79-4.87 (m, 1H), 4.68-4.76 (m, 2H), 4.59-4.66 (m, 1H), 4.20 (d, J=15.26 Hz, 1H), 3.61-3.73 (m, 1H), 3.29-3.40 (m, 1H), 1.79 (d, *J*=9.51 Hz, 1H).

### Step 2

At 0 °C, zinc powder (1.28 g, 19.51 mmol, 4 *eq)* and acetic acid (7 mL) were added sequentially to a solution of compound **6b** (625 mg, 4.88 mmol, 1 *eq)* in methanol (7 mL), and after the addition was completed, the reaction solution was reacted at 20 °C for 4 hours. After the reaction was completed, the reaction solution was filtered through celite and washed with ethyl acetate (100 mL), and the filtrate was concentrated under reduced pressure to obtain compound **6c.**

### Step 3

At 0 °C, compound **6c** (1.92 g, 3.56 mmol, 1 *eq,* acetate) was added to a solution of compound **1a** (1.72 g, 8.89 mmol, 2.5 *eq)* in dioxane (35 mL), and after the addition was completed, the reaction solution was reacted at 20 °C for 1 hour. After the reaction was completed, the reaction solution was diluted with water (30 mL), extracted with ethyl acetate (30 mL^{∗}3), washed with saturated brine (30 mL), dried over anhydrous sodium sulfate, filtered, and the filtrate was concentrated under reduced pressure and purified by column chromatography (ethyl acetate: petroleum ether=0:1-1:1) to obtain compound **6e.** MS: *m*/*z* 271.9 [M+H]⁺.

### Step 4

Iron powder (234.35 mg, 4.2 mmol, 5 *eq)* and ammonium chloride (224.47 mg, 4.2 mmol, 5 *eq)* were added sequentially to a solution of compound **6e** (228 mg, 839.28 µmol, 1 *eq)* in ethanol (5 mL) and water (5 mL), and after the addition was completed, the reaction solution was reacted at 75 °C for 1 hour. After the reaction was completed, the reaction solution was cooled to room temperature, filtered through celite and washed with ethanol (20 mL), and the washing solution was concentrated under reduced pressure to obtain a crude product, and the crude product was dissolved in a solution of dichloromethane/methanol (20 mL: 2 mL), stirred for 15 minutes, filtered, and the filtrate was concentrated under reduced pressure to obtain compound **6f.**

### Step 5

*N,N'*-Carbonyldiimidazole (207.99 mg, 1.28 mmol, 2 *eq)* was added to a solution of compound **6f** (155 mg, 641.35 µmol, 1 *eq)* in acetonitrile (6 mL), and after the addition was completed, the reaction solution was reacted at 80 °C for 2 hours. After the reaction was completed, the reaction solution was concentrated under reduced pressure, and purified by column chromatography (methanol: dichloromethane=0:1-1:9) to obtain compound **6g.** MS: *m*/*z* 267.9 [M+H]⁺.

¹H NMR (400 MHz, CD₃OD) δ ppm 9.00 (s, 1H), 5.38 (d, *J*=6.13 Hz, 2H), 4.76 (d, *J*=10.13 Hz, 2H), 4.04-4.13 (m, 2H), 3.80-3.91 (m, 1H), 3.21 (d, *J*=8.38 Hz, 1H).

### Step 6

Cesium carbonate (255.62 mg, 784.54 µmol, 2 *eq)* and iodomethane (0.58 g, 4.09 mmol, 1.2 *eq)* were added sequentially to a solution of compound **6g** (105 mg, 392.27 µmol, 1 *eq)* in *N,N-*dimethylformamide (6 mL), and after the addition was completed, the reaction solution was reacted at 20 °C for 1 hour. After the reaction was completed, the reaction solution was diluted with water (10 mL), extracted with ethyl acetate (10 mL^{∗}3), washed with saturated brine (20 mL), dried over anhydrous sodium sulfate, filtered, and the filtrate was concentrated under reduced pressure and purified by column chromatography (methanol: dichloromethane=0:1-1:9) to obtain compound **6h.** MS: m/z 281.8 [M+H]⁺

¹H NMR (400 MHz, DMSO-*d*₆) δ ppm 8.38 (s, 1H), 4.54 (d, J=6.13 Hz, 2H), 3.90 (d, *J*=10.13 Hz, 2H), 3.35 (s, 3 H), 3.23-3.28 (m, 2H), 2.96-3.03 (m, 1H), 2.37 (d, *J*=8.38 Hz, 1H).

### Step 7

Compound **6h** (95 mg, 337.24 µmol, 1 *eq),* compound **1g** (44.97 mg, 303.52 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (61.14 mg, 67.45 µmol, 0.2 *eq)* and cesium carbonate (219.76 mg, 674.48 µmol, 2 *eq)* were placed in a reaction flask, and the reaction flask was vacuumized and repaced with nitrogen three times, then anhydrous dioxane (7 mL) was added to the mixture and the reaction was carried out at 100 °C for 3 hours. After the reaction was completed, the reaction solution was filtered through celite and concentrated under reduced pressure to obtain a crude product, and the crude product was purified by preparative high performance liquid chromatography (Welch Xtimate C18 100^{∗}40 mm^{∗}3 µm; mobile phase: [water 0.225 % formic acid)-acetonitrile]; B (acetonitrile) %: 6 %-36 %, 8 minutes) to obtain compound **6.**

MS: *m*/*z* 394.0 [M+H]⁺.

¹H NMR (400 MHz, CDCl₃) δ ppm 9.85 (s, 1H), 8.26 (s, 1H), 7.95 (s, 1H), 7.57 (s, 1H), 6.80 (s, 1H), 4.68 (d, J=6.53 Hz, 2H), 4.23 (d, *J*=9.79 Hz, 2H), 3.45-3.48 (m, 1H), 3.44 (s, 3H), 3.42-3.44 (m, 1H), 3.19-3.31 (m, 1H), 2.71 (d, *J*=8.53 Hz, 1H), 2.52 (s, 3H).

### Embodiment 7

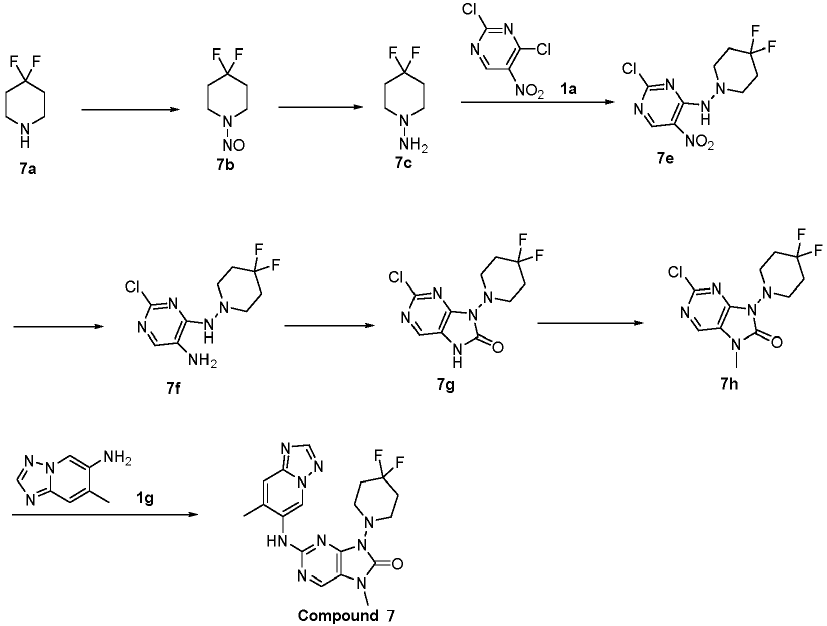

### Step 1

At 0 °C, a solution of sodium nitrite (1.31 g, 18.99 mmol, 1 *eq)* in water (2.4 mL) was slowly added to a mixed solution of compound **7a** (2.3 g, 18.99 mmol, 1 *eq)* in acetic acid (24 mL) and water (8 mL), and after the addition was completed, the reaction solution was reacted at 25 °C for 1.5 hours. After the reaction was completed, the reaction solution was diluted with water (20 mL), extracted with ethyl acetate (30 mL^{∗}3), dried over anhydrous sodium sulfate, filtered, and the filtrate was concentrated under reduced pressure and purified by column chromatography (ethyl acetate: petroleum ether= 0:1-1:3) to obtain compound **7b.**

¹H NMR (400 MHz, CDCl₃) δ ppm 4.32-4.36 (m, 2H), 3.84-3.89 (m, 2H), 2.17 (tt, J=12.89, 6.31 Hz, 2H), 1.90 (tt, J=13.05, 6.40 Hz, 2H).

### Step 2

At 0 °C, zinc powder (3.12 g, 47.69 mmol, 4 *eq)* was added to a mixed solution of compound **7b** (1.79 g, 11.92 mmol, 1 *eq)* in acetic acid (10 mL) and methanol (10 mL), and after the addition was completed, the reaction solution was reacted at 25 °C for 0.5 hours. After the reaction was completed, ethyl acetate (40 mL) was added to dilute the reaction solution, and the reaction solution was filtered through celite, and the filtrate was concentrated under reduced pressure to obtain compound **7c.**

### Step 3

At 0 °C, a solution of compound **7c** (3.8 g, 19.37 mmol, 1 *eq,* acetate) in dioxane (80 mL) and triethylamine (7.84 g, 77.47 mmol, 4 *eq,* 10.78 mL) were added sequentially to a solution of compound **1a** (7.51 g, 38.74 mmol, 2 *eq)* in dioxane (100 mL), and after the addition was completed, the reaction solution was reacted at 25 °C for 1 hour. After the reaction was completed, the reaction was diluted with water (100 mL), extracted with ethyl acetate (100 mL^{∗}3), dried over anhydrous sodium sulfate, filtered, and the filtrate was concentrated under reduced pressure and purified by column chromatography (ethyl acetate: petroleum ether=0:1-1:4) to obtain compound **7e.** MS: *m*/*z* 293.8 [M+H]⁺;

¹H NMR (400 MHz, CDCl₃) δ ppm 9.12 (s, 1H), 9.08 (s, 1H), 3.13 (t, J=5.65 Hz, 4H), 2.20-2.30 (m, 4H).

### Step 4

Iron powder (475.47 mg, 8.51 mmol, 5 *eq)* and ammonium chloride (455.38 mg, 8.51 mmol, 5 *eq)* were added sequentially to a mixed solution of compound **7e** (0.5 g, 1.70 mmol, 1 *eq)* in ethanol (20 mL) and water (5 mL), and after the addition was completed, the reaction solution was reacted at 75 °C for 1 hour. After the reaction was completed, the reaction solution was cooled to room temperature, filtered through celite and concentrated under reduced pressure to obtain a crude product. At 25 °C, the crude product was dissolved in a solution of dichloromethane/methanol (20 mL: 2 mL), stirred for 15 minutes, filtered. The filtrate was concentrated under reduced pressure to obtain compound **7f.** MS: *m*/*z* 263.8 [M+H]⁺.

### Step 5

*N,N'*-Carbonyldiimidazole (848.64 mg, 5.23 mmol, 3 *eq)* was added to a solution of compound **7f** (460 mg, 1.74 mmol, 1 *eq)* in acetonitrile (10 mL), and after the addition was completed, the reaction solution was reacted at 80 °C for 1 hour. After the reaction was completed, the reaction solution was concentrated under reduced pressure and purified by column chromatography (ethyl acetate: petroleum ether=0:1-1:1) to obtain compound **7g.** MS: *m*/*z* 289.7 [M+H]⁺;

¹H NMR (400 MHz, CDCl₃) δ ppm 8.32 (br s, 1H), 7.99 (s, 1H), 3.53 (brt, J=5.52 Hz, 4H), 2.13-2.25 (m, 4H).

### Step 6

Cesium carbonate (2.43 g, 7.44 mmol, 4 *eq)* and iodomethane (792.34 mg, 5.58 mmol, *3 eq)* were added sequentially to a solution of compound **7g** (539 mg, 1.86 mmol, 1 *eq)* in *N,N-*dimethylformamide (6 mL), and after the addition was completed, the reaction solution was reacted at 25 °C for 1 hour. After the reaction was completed, water (6 mL) was added to quench the reaction, and the reaction solution was concentrated under reduced pressure, then diluted with water (6 mL), extracted with ethyl acetate (10 mL^{∗}3), dried over anhydrous sodium sulfate, filtered, and the filtrate was concentrated under reduced pressure and purified by column chromatography (ethyl acetate: petroleum ether=0:1-1:1) to obtain compound **7h.** MS: *m*/*z* 303.8 [M+H]⁺;

¹H NMR (400 MHz, CDCl₃) δ ppm 8.06 (s, 1H), 3.59 (br t, J=5.52 Hz, 4H), 3.46 (s, 3H), 2.24-2.34 (m, 4H).

### Step 7

Compound **7h** (218 mg, 717.82 µmol, 1 *eq),* compound **1g** (95.72 mg, 646.04 µmol, 0.9 *eq),* methanesulfonato(2-dicyclohexylphosphino-3,6-dimethoxy-2',4',6'-tri-i-propyl-1, 1'-biphenyl)(2'-amino-1,1'-biphenyl-2-yl)palladium(II) (130.14 mg, 143.56 µmol, 0.2 *eq)* and cesium carbonate (350.82 mg, 1.08 mmol, 1.5 *eq)* were placed in a reaction flask, and the reaction flask was vacuumized and repaced with nitrogen three times, then anhydrous dioxane (6 mL) was added to the mixture and the reaction was carried out at 100 °C for 2 hours. After the reaction was completed, the reaction solution was concentrated under reduced pressure, and then purified by column chromatography (methanol: dichloromethane=0:1-1:9) to obtain compound 7. MS: *m*/*z* 416.1 [M+H]⁺;

¹H NMR (400 MHz, CDCl₃) δ ppm 9.75 (s, 1H), 8.28 (s, 1H), 7.94 (s, 1H), 7.60 (s, 1H), 6.79 (s, 1H), 3.56-3.66 (m, 4H), 3.43 (s, 3H), 2.54 (s, 3H), 2.30 (s, 4H).

### Embodiments 8 and 9

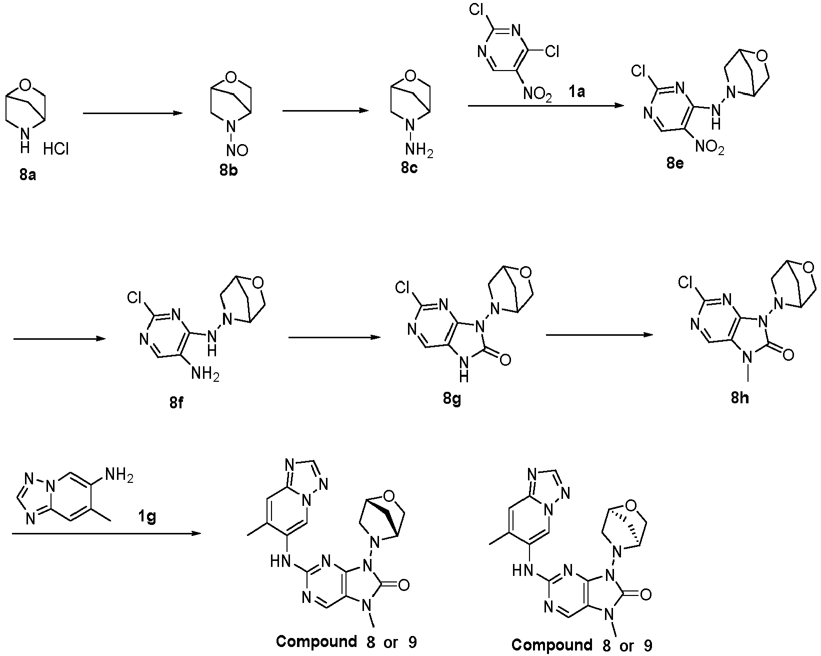

### Step 1

At 0 °C, a solution of sodium nitrite (2.54 g, 36.88 mmol, 1 *eq)* in water (5 mL) was slowly added to a mixed solution of compound 8a (5 g, 36.88 mmol, 1 *eq,* hydrochloride) in acetic acid (50 mL) and water (17 mL), and after the addition was completed, the reaction solution was reacted at 25 °C for 18 hours. After the reaction was completed, the reaction solution was diluted with water (40 mL), extracted with ethyl acetate (50 mL^{∗}8), dried over anhydrous sodium sulfate, filtered, and the filtrate was concentrated under reduced pressure and purified by column chromatography (ethyl acetate: petroleum ether= 0:1-2:1) to obtain compound **8b.**

¹H NMR (400 MHz, CDCl₃) δ ppm 5.47 (s, 1H), 4.79 (s, 1H), 4.02 (s, 2H), 3.46-3.62 (m, 2H), 2.12 (d, J=10.29 Hz, 1H), 1.96 (dd, *J*=10.29, 2.26 Hz, 1H).

### Step 2

At 0 °C, zinc powder (3.94 g, 60.25 mmol, 4 *eq)* was added to a mixed solution of compound **8b** (1.93 g, 15.06 mmol, 1 *eq)* in acetic acid (10 mL) and methanol (10 mL), and after the addition was completed, the reaction solution was reacted at 25 °C for 1 hour. After the reaction was completed, the reaction solution was filtered through celite, and the filtrate was concentrated under reduced pressure to obtain compound **8c.**

### Step 3

At 0 °C, a solution of compound **8c** (4.4 g, 25.26 mmol, 1 *eq,* acetate) in dioxane (80 mL) and triethylamine (12.78 g, 126.29 mmol, 5 *eq,* 17.58 mL) were added sequentially to a solution of compound **1a** (9.80 g, 50.52 mmol, 2 *eq)* in dioxane (140 mL), and after the addition was completed, the reaction solution was reacted at 25 °C for 1 hour. After the reaction was completed, the reaction was diluted with water (100 mL), extracted with ethyl acetate (100 mL^{∗}3), dried over anhydrous sodium sulfate, filtered, and the filtrate was concentrated under reduced pressure and purified by column chromatography (ethyl acetate: petroleum ether=0:1-1:2) to obtain compound **8e.** MS: *m*/*z* 272.0 [M+H]⁺.

¹H NMR (400 MHz, CDCl₃) δ ppm 9.37 (br s, 1H), 8.99 (s, 1H), 4.48 (s, 1H), 4.11 (d, J=9.29 Hz, 1H), 3.95 (s, 1H), 3.72 (dd, J=9.16, 1.63 Hz, 1H), 3.39-3.48 (m, 1H), 2.90-2.97 (m, 1H), 2.11 (d, *J*=10.29 Hz, 1H), 1.81-2.02 (m, 1H).

### Step 4

Iron powder (513.97 mg, 9.20 mmol, 5 *eq)* and ammonium chloride (492.25 mg, 9.20 mmol, 5 *eq)* were added sequentially to a mixed solution of compound **8e** (0.5 g, 1.84 mmol, 1 *eq)* in ethanol (5 mL) and water (5 mL), and after the addition was completed, the reaction solution was reacted at 75 °C for 0.5 hours. After the reaction was completed, the reaction solution was cooled to room temperature, filtered through celite and concentrated under reduced pressure to obtain a crude product. At 25 °C, the crude product was dissolved in a solution of dichloromethane/methanol (10 mL: 1 mL), stirred for 15 minutes, filtered, and the filtrate was concentrated under reduced pressure to obtain compound **8f.** MS: *m*/*z* 242.0 [M+H]⁺.

### Step 5

*N,N'*-Carbonyldiimidazole (509.91 mg, 3.14 mmol, 2 *eq)* was added to a solution of compound **8f** (380 mg, 1.57 mmol, 1 *eq)* in acetonitrile (8 mL), and after the addition was completed, the reaction solution was reacted at 80 °C for 1.5 hours. After the reaction was completed, the reaction solution was concentrated under reduced pressure and purified by column chromatography (ethyl acetate: petroleum ether=0:1-1:0) to obtain compound **8g.** MS: *m*/*z* 267.8 [M+H]⁺;

¹H NMR (400 MHz, CDCl₃) δ ppm 8.00 (s, 1H), 4.63 (s, 1H), 4.21 (d, J=7.53 Hz, 1H), 3.76-3.83 (m, 2H), 3.71 (dd, J=7.78, 1.76 Hz, 1H), 3.60 (d, J=10.04 Hz, 1H), 2.73 (d, J=10.04 Hz, 1H), 1.87 (d, J=10.79 Hz, 1H).

### Step 6

Cesium carbonate (2.53 g, 7.77 mmol, 4 *eq)* and methyl iodide (827.22 mg, 5.83 mmol, *3 eq)* were added sequentially to a solution of compound **8g** (520 mg, 1.94 mmol, 1 *eq)* in *N,N-*dimethylformamide (6 mL), and after the addition was completed, the reaction solution was reacted at 25 °C for 1 hour. After the reaction was completed, water (5 mL) was added to quench the reaction, and the reaction solution was concentrated under reduced pressure, then diluted with water (5 mL), extracted with ethyl acetate (10 mL^{∗}6), dried over anhydrous sodium sulfate, filtered, and the filtrate was concentrated under reduced pressure and purified by column chromatography (ethyl acetate: petroleum ether=0:1-4:1) to obtain compound **8h.** MS: *m*/*z* 281.8 [M+H]⁺;

¹H NMR (400 MHz, CDCl₃) δ ppm 8.05 (s, 1H), 4.71 (s, 1H), 4.28 (d, J=7.78 Hz, 1H), 3.82-3.88 (m, 2H), 3.79 (dd, J=7.78, 1.76 Hz, 1H), 3.66 (d, *J*=9.29 Hz, 1H), 3.47 (s, 3H), 2.81 (d, *J*=8.53 Hz, 1H), 1.95 (d, J=11.04 Hz, 1H).

### Step 7

Compound **8h** (160 mg, 567.98 µmol, 1 *eq),* compound **1g** (75.74 mg, 511.18 µmol, 0.9 *eq),* methanesulfonato(2-dicyclohexylphosphino-3,6-dimethoxy-2',4',6'-tri-i-propyl-1, 1'-biphenyl)(2'-amino-1,1'-biphenyl-2-yl)palladium(II) (102.98 mg, 113.60 µmol, 0.2 *eq)* and cesium carbonate (277.59 mg, 851.97 µmol, 1.5 *eq)* were placed in a reaction flask, and the reaction flask was vacuumized and repaced with nitrogen three times, then anhydrous dioxane (5 mL) was added to the mixture and the reaction was carried out at 100 °C for 2 hours. After the reaction was completed, the reaction solution was concentrated under reduced pressure and purified by column chromatography (methanol: dichloromethane=0:1-1:9) to obtain a racemate, then the racemate was purified by supercritical fluid chromatography (column: Phenomenex-Cellulose-2 (250 mm^{∗}3\0 mm,10 µm); Mobile Phase: [0.1 % ammonium hydroxideisopropanol];-B (0.1 % ammonium hydroxide/isopropanol)%: 55 %-55 %)to obtain compound **8** and compound **9.**

Compound **8:** (retention time 9.25 min) MS: *m*/*z* 394.1 [M+H]⁺;

¹H NMR (400 MHz, CDCl₃) δ ppm 9.86 (s, 1H), 8.29 (s, 1H), 7.95 (s, 1H), 7.59 (s, 1H), 6.85 (s, 1H), 4.77 (s, 1H), 4.32 (d, *J*=8.03 Hz, 1H), 3.99 (d, J=9.79 Hz, 1H), 3.91 (s, 1H), 3.83 (dd, *J*=7.78, 1.51 Hz, 1H), 3.70 (d , J=8.78 Hz, 1H), 3.44 (s, 3H), 2.75 (d, J=10.29 Hz, 1H), 2.54 (s, 3H), 1.97 (d, *J*=10.04 Hz, 1H).

Compound **9:** (retention time 11.75 min) MS: *m*/*z* 394.1 [M+H]⁺;

¹H NMR (400 MHz, CDCl₃) δ ppm 9.86 (s, 1H), 8.29 (s, 1H), 7.95 (s, 1H), 7.59 (s, 1H), 6.84 (s, 1H), 4.77 (s, 1H), 4.32 (d, J=7.78 Hz, 1H), 3.99 (d, J=9.79 Hz, 1H), 3.91 (s, 1H), 3.83 (dd, J=7.78, 1.76 Hz, 1H), 3.70 (d , J=9.79 Hz, 1H) 3.45 (s, 3H), 2.75 (d, *J*=8.53 Hz, 1H), 2.54 (s, 3 H) 1.97 (d, J=10.04 Hz, 1H).

### Embodiment 10

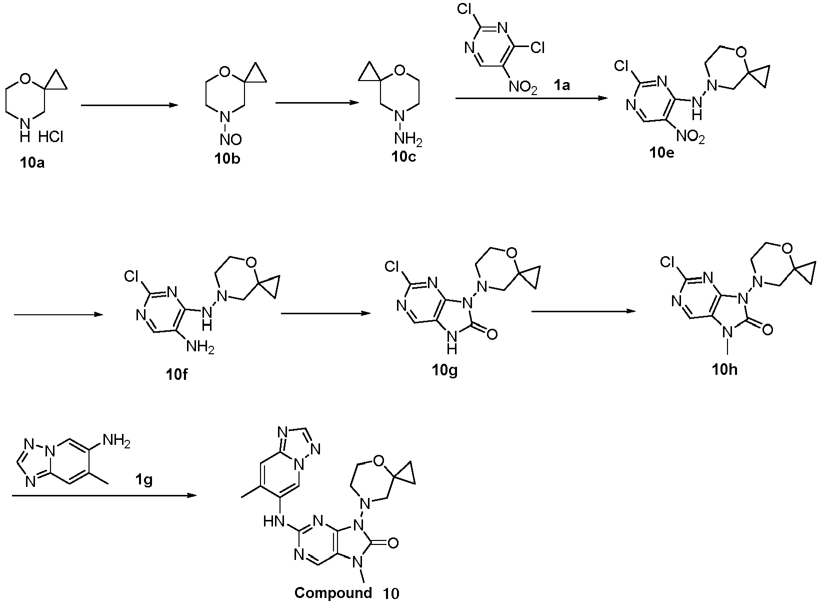

### Step 1

At 0 °C, a solution of sodium nitrite (507.3 mg, 7.35 mmol, 1.1 *eq)* in water (1 mL) was slowly added to a mixed solution of compound **10a** (1 g, 1.29 mmol, 1 *eq,* hydrochloride) in acetic acid (12 mL) and water (4 mL), and after the addition was completed, the reaction solution was reacted at 20 °C for 4 hours. After the reaction was completed, the reaction solution was diluted with water (30 mL), extracted with ethyl acetate (30 mL^{∗}3), washed with saturated brine (20 mL), dried over anhydrous sodium sulfate, filtered, and the filtrate was concentrated under reduced pressure and purified by column chromatography (ethyl acetate: petroleum ether=0:1-1:1) to obtain compound **10b.**

### Step 2

At 0 °C, zinc powder (1.58 g, 24.2 mmol, 4 *eq)* and acetic acid (5 mL) were added sequentially to a solution of compound **10b** (860 mg, 6.05 mmol, 1 *eq)* in methanol (5 mL), and after the addition was completed, the reaction solution was reacted at 20 °C for 4 hours. After the reaction was completed, the reaction solution was diluted with ethyl acetate (100 mL), filtered through celite, and the filtrate was concentrated under reduced pressure to obtain compound **10c.**

### Step 3

At 0 °C, compound **10c** (1.41 g, 4.5 mmol, 1 *eq,* acetate) and triethylamine (910.7 mg, 9 mmol, 2 *eq)* were added to a solution of compound **1a** (1.75 g, 9 mmol, 2 *eq)* in dioxane (60 mL), and after the addition was completed, the reaction solution was reacted at 20 °C for 5 hours. After the reaction was completed, the reaction solution was diluted with water (100 mL), extracted with ethyl acetate (100 mL^{∗}3), washed with saturated brine (50 mL), dried over anhydrous sodium sulfate, filtered, and the filtrate was concentrated under reduced pressure and purified by column chromatography (ethyl acetate: petroleum ether=0:1-1:1) to obtain compound **10e.** MS: *m*/*z* 285.9 [M+H]⁺;

¹H NMR (400 MHz, CDCl₃) δ ppm 9.32 (br s, 1H), 9.07 (s, 1H), 3.90-3.99 (m, 2H), 3.12-3.22 (m, 2H), 3.03 (s, 2H), 0.89-0.97 (m, 2H), 0.64-0.75 (m, 2H).

### Step 4

Iron powder (156.38 mg, 2.8 mmol, 5 *eq)* and ammonium chloride (149.79 mg, 2.8 mmol, 5 *eq)* were added sequentially to a mixed solution of compound **10e** (160 mg, 560.05 µmol, 1 *eq)* in ethanol (8 mL) and water (2 mL), and after the addition was completed, the reaction solution was reacted at 75 °C for 3 hours. After the reaction was completed, the reaction solution was cooled to room temperature, filtered through celite and washed with ethanol (100 mL), and the washing solution was concentrated under reduced pressure to obtain a crude product **10f**.

### Step 5

*N,N'*-Carbonyldiimidazole (190.24 mg, 1.17 mmol, 2 *eq)* was added to a solution of compound **10f** (150 mg, 586.62 µmol, 1 *eq)* in acetonitrile (4 mL), and after the addition was completed, the reaction solution was reacted at 80 °C for 2 hours. After the reaction was completed, the reaction solution was concentrated under reduced pressure, and the obtained crude product was purified by column chromatography (ethyl acetate: petroleum ether=0:1-1:0) to obtain compound **10g**. MS: *m*/*z* 281.8 [M+H]⁺.

### Step 6

Cesium carbonate (242.89 mg, 745.48 µmol, 1.5 *eq)* and iodomethane (88.18 mg, 624.23 µmol, 1.25 *eq)* were added sequentially to a solution of compound **10g** (140 mg, 496.99 µmol, 1 *eq)* in *N,N-*dimethylformamide (6 mL), and after the addition was completed, the reaction solution was reacted at 21 °C for 4 hours. After the reaction was completed, the reaction solution was diluted with water (10 mL), extracted with ethyl acetate (10 mL^{∗}3), washed with saturated brine (10 mL), dried over anhydrous sodium sulfate, filtered, and the filtrate was concentrated under reduced pressure and purified by column chromatography (ethyl acetate: petroleum ether=0:1-1:0) to obtain compound **10h**. MS: m/z 296.0 [M+H]⁺;

¹H NMR (400 MHz, CDCl₃) δ ppm 8.02 (s, 1H), 3.92-4.00 (m, 2H), 3.52-3.64 (m, 2H), 3.39-3.46 (m, 5H), 0.84-0.97 (m, 2H), 0.64-0.71 (m, 2H).

### Step 7

Compound **10h** (59.14 mg, 200 µmol, 1 *eq),* compound **1g** (26.67 mg, 180 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (36.26 mg, 40 µmol, 0.2 *eq)* and cesium carbonate (97.75 mg, 300 µmol, 1.5 *eq)* were placed in a reaction flask, and the reaction flask was vacuumized and repaced with nitrogen three times, then anhydrous dioxane (5 mL) was added to the mixture and the reaction was carried out at 100 °C for 3 hours. After the reaction was completed, the reaction solution was filtered through celite and concentrated under reduced pressure to obtain a crude product, and the crude product was first purified by column chromatography (methanol: dichloromethane=0:1-1:9), and then purified by supercritical fluid chromatography (DAICEL CHIRALPAK AD-H (250 mm^{∗}30 mm, 5 µm); mobile phase: [0.1 % ammonium hydroxide/ethanol];-B (0.1 % ammonium hydroxide/ethanol)%: 30 %-30 %) to obtain compound **10** (retention time 1.59 minutes). MS: m/z 430.0 [M+Na]⁺;

¹H NMR (400 MHz, CDCl₃) δ ppm 9.83 (s, 1H), 8.26 (s, 1H), 7.92 (s, 1H), 7.57 (s, 1H), 6.77 (s, 1H), 3.96-4.04 (m, 2H), 3.60-3.69 (m, 2H), 3.44-3.50 (m, 2H), 3.41 (s, 3H), 2.53 (s, 3H), 0.91-1.03 (m, 2H), 0.62-0.76 (m, 2H).

### Embodiment 11

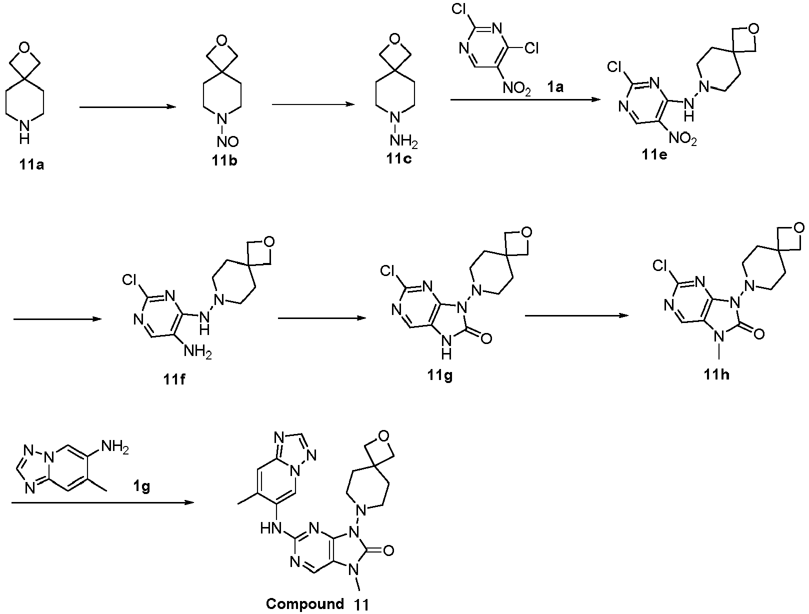

### Step 1

At 0 °C, a solution of sodium nitrite (501.83 mg, 7.27 mmol, 1 *eq)* in water (1.5 mL) was slowly added to a mixed solution of compound **11a** (1.25 g, 7.27 mmol, 1 *eq,* hemioxalate) in acetic acid (15 mL) and water (5 mL), and after the addition was completed, the reaction solution was reacted at 25 °C for 2 hours. After the reaction was completed, the reaction solution was diluted with water (20 mL), extracted with ethyl acetate (30 mL^{∗}6), dried over anhydrous sodium sulfate, filtered, and the filtrate was concentrated under reduced pressure and purified by column chromatography (ethyl acetate: petroleum ether=0: 1-2:1) to obtain compound **11b**.

¹H NMR (400 MHz, CDCl₃) δ ppm 4.53 (q, *J*=6.00 Hz, 4H), 4.17-4.22 (m, 2H), 3.74-3.79 (m, 2H), 2.09-2.16 (m, 2H), 1.83 -1.90 (m, 2H).

### Step 2

At 0 °C, zinc powder (574.43 mg, 8.78 mmol, 4 *eq)* was added to a mixed solution of compound **11b** (343 mg, 2.20 mmol, 1 *eq)* in acetic acid (2 mL) and methanol (2 mL), and after the addition was completed, the reaction solution was reacted at 25 °C for 6 hours. After the reaction was completed, ethyl acetate (30 mL) was added to dilute the reaction solution, and the reaction solution was filtered through celite, and the filtrate was concentrated under reduced pressure to obtain compound **11c.**

### Step 3

At 0 °C, a solution of compound **11c** (755 mg, 1.87 mmol, 1 *eq,* acetate) in dioxane (5 mL) and triethylamine (755.48 mg, 7.47 mmol, 5 *eq,* 1.04 mL) were added sequentially to a solution of compound **1a** (724.11 mg, 3.73 mmol, 2 *eq)* in dioxane (30 mL), and after the addition was completed, the reaction solution was reacted at 25 °C for 1 hour. After the reaction was completed, the reaction solution was diluted with water (50 mL), extracted with ethyl acetate (50 mL^{∗}3), dried over anhydrous sodium sulfate, filtered, and the filtrate was concentrated under reduced pressure and purified by column chromatography (ethyl acetate: petroleum ether=0: 1-1:1) to obtain compound **11e**. MS: *m*/*z* 299.8 [M+H]⁺;

¹H NMR (400 MHz, CDCl₃) δ ppm 9.06 (s, 1H), 9.01 (br s, 1H), 4.48 (s, 4H), 2.89 (t, *J*=5.14 Hz, 4H), 2.11 (t, *J=* 5.52 Hz, 4H).

### Step 4

Iron powder (93.17 mg, 1.67 mmol, 5 *eq)* and ammonium chloride (89.24 mg, 1.67 mmol, 5 *eq)* were added sequentially to a mixed solution of compound **11e** (0.1 g, 333.65 µmol, 1 *eq)* in ethanol (2 mL) and water (2 mL), and after the addition was completed, the reaction solution was reacted at 75 °C for 1 hour. After the reaction was completed, the reaction solution was cooled to room temperature, filtered through celite and concentrated under reduced pressure to obtain a crude product. At 25 °C, the crude product was dissolved in a solution of dichloromethane/methanol (10 mL: 1 mL), stirred for 15 minutes, filtered, and the filtrate was concentrated under reduced pressure to obtain compound **11f.**

### Step 5

*N,N'*-Carbonyldiimidazole (120.83 mg, 745.19 µmol, 3 *eq)* was added to a solution of compound **11f** (67 mg, 248.40 µmol, 1 *eq)* in acetonitrile (2 mL), and after the addition was completed, the reaction solution was reacted at 80 °C for 1 hour. After the reaction was completed, the reaction solution was concentrated under reduced pressure and purified by thin-layer preparative chromatography (ethyl acetate: petroleum ether=1:0) to obtain compound **11g**. MS: *m*/*z* 295.8 [M+H]⁺.

### Step 6

Cesium carbonate (282.05 mg, 865.67 µmol, 4 *eq)* and iodomethane (92.16 mg, 649.25 µmol, 3 *eq)* were added sequentially to a solution of compound **11g** (64 mg, 216.42 µmol, 1 *eq)* in *N,N-*dimethylformamide (2 mL), and after the addition was completed, the reaction solution was reacted at 25 °C for 1 hour. After the reaction was completed, water (3 mL) was added to quench the reaction, and the reaction solution was concentrated under reduced pressure, then diluted with water (3 mL), extracted with ethyl acetate (10 mL^{∗}6), dried over anhydrous sodium sulfate, filtered, and the filtrate was concentrated under reduced pressure to obtain compound **11h**.

### Step 7

Compound **11h** (21 mg, 67.80 µmol, 1 *eq),* compound **1g** (9.04 mg, 61.02 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (12.29 mg, 13.56 µmol, 0.2 *eq)* and cesium carbonate (33.13 mg, 101.69 µmol, 1.5 *eq)* were placed in a reaction flask, and the reaction flask was vacuumized and repaced with nitrogen three times, then anhydrous dioxane (3 mL) was added to the mixture and the reaction was carried out at 100 °C for 2 hours. After the reaction was completed, the reaction solution was concentrated under reduced pressure, and then purified by thin-layer preparative chromatography (methanol: dichloromethane=1:20) to obtain compound **11.** MS: *m*/*z* 422.4 [M+H]⁺;

¹H NMR (400 MHz, CDCl₃) δ ppm 9.69 (s, 1H), 8.26 (s, 1H), 7.91 (s, 1H), 7.57 (s, 1H), 6.75 (s, 1H), 4.52 (s, 4H), 3.39 (s, 7H) 2.50 (s, 3H), 2.13 (t, *J*=4.64 Hz, 4H).

### Embodiment 12

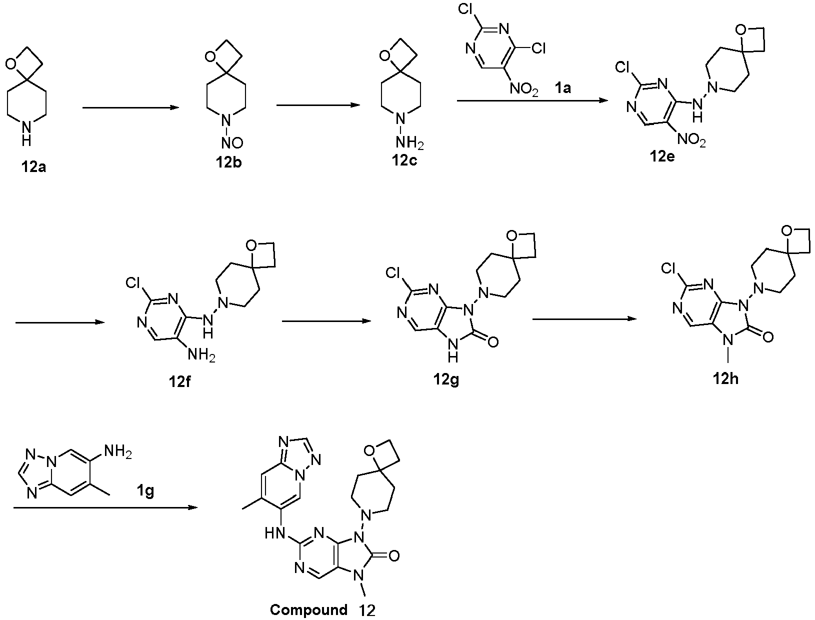

### Step 1

At 0 °C, a solution of sodium nitrite (834.47 mg, 12.09 mmol, 1 *eq)* in water (3.5 mL) was slowly added to a mixed solution of compound **12a** (3.55 g, 12.09 mmol, 1 *eq,* hemioxalate) in acetic acid (35 mL) and water (12 mL), and after the addition was completed, the reaction solution was reacted at 25 °C for 16 hours. After the reaction was completed, water (10 mL) was added to quench the reaction, and the reaction solution was concentrated under reduced pressure, then diluted with water (15 mL), extracted with ethyl acetate (50 mL^{∗}6), dried over anhydrous sodium sulfate, filtered, and the filtrate was concentrated under reduced pressure and purified by column chromatography (ethyl acetate: petroleum ether=0:1-1:1) to obtain compound **12b.**

¹H NMR (400 MHz, CDCl₃) δ ppm 4.52-4.69 (m, 2H), 4.40-4.50 (m, 2H), 4.09-4.17 (m, 1H), 3.25-3.34 (m, 1H), 2.43-2.54 (m, 2H), 2.24-2.33 (m, 1H), 2.02-2.11 (m, 1H), 1.88-1.99 (m, 1H), 1.61-1.70 (m, 1H).

### Step 2

At 0 °C, zinc powder (1.59 g, 24.33 mmol, 4 *eq)* was added to a mixed solution of compound **12b** (0.95 g, 6.08 mmol, 1 *eq)* in acetic acid (5 mL) and methanol (5 mL), and after the addition was completed, the reaction solution was reacted at 25 °C for 2 hours. After the reaction was completed, ethyl acetate (50 mL) was added to dilute the reaction solution, and the reaction solution was filtered through celite, and the filtrate was concentrated under reduced pressure to obtain compound **12c.**

### Step 3

At 0 °C, a solution of compound **12c** (2.7 g, 6.67 mmol, 1 *eq,* acetate) in dioxane (35 mL) and triethylamine (2.70 g, 26.70 mmol, 4 *eq,* 3.72 mL) were added sequentially to a solution of compound 1a (2.59 g, 13.35 mmol, 2 *eq)* in dioxane (100 mL), and after the addition was completed, the reaction solution was reacted at 25 °C for 1 hour. After the reaction was completed, water (50 mL) was added to quench the reaction, and the reaction solution was diluted with water (25 mL), extracted with ethyl acetate (50 mL^{∗}3), dried over anhydrous sodium sulfate, filtered, and the filtrate was concentrated under reduced pressure and purified by column chromatography (ethyl acetate: petroleum ether=0:1-1:1) to obtain compound **12e.** MS: *m*/*z* 299.8 [M+H]⁺;

¹H NMR (400 MHz, CDCl₃) δ ppm 9.05 (s, 2H), 4.55 (t, J=7.78 Hz, 2H), 2.90-3.07 (m, 4H), 2.46 (t, *J*=7.78 Hz, 2H), 2.13-2.22 (m, 2H), 2.04-2.11 (m, 2H).

### Step 4

Iron powder (234.79 mg, 4.20 mmol, 5 *eq)* and ammonium chloride (224.87 mg, 4.20 mmol, 5 *eq)* were added sequentially to a mixed solution of compound **12e** (252 mg, 840.80 µmol, 1 *eq)* in ethanol (12 mL) and water (4 mL), and after the addition was completed, the reaction solution was reacted at 75 °C for 1 hour. After the reaction was completed, the reaction solution was cooled to room temperature, filtered through celite and concentrated under reduced pressure to obtain a crude product. At 25 °C, the crude product was dissolved in a solution of dichloromethane/methanol (10 mL: 1 mL), stirred for 15 minutes, filtered, and the filtrate was concentrated under reduced pressure to obtain compound **12f.**

### Step 5

*N,N'*-Carbonyldiimidazole (180.35 mg, 1.11 mmol, 3 *eq)* was added to a solution of compound **12f** (100 mg, 370.74 µmol, 1 *eq)* in acetonitrile (3 mL), and after the addition was completed, the reaction solution was reacted at 80 °C for 1 hour. After the reaction was completed, the reaction solution was concentrated under reduced pressure and purified by thin-layer preparative chromatography (ethyl acetate: petroleum ether=1:0) to obtain compound 12g. MS: *m*/*z* 296.0 [M+H]⁺.

### Step 6

Cesium carbonate (581.73 mg, 1.79 mmol, 4 *eq)* and iodomethane (190.07 mg, 1.34 mmol, 3 *eq)* were added sequentially to a solution of compound **12g** (132 mg, 446.36 µmol, 1 *eq)* in *N,N-*dimethylformamide (4 mL), and after the addition was completed, the reaction solution was reacted at 20 °C for 1 hour. After the reaction was completed, water (5 mL) was added to quench the reaction, and the reaction solution was concentrated under reduced pressure, then diluted with water (5 mL), extracted with ethyl acetate (10 mL^{∗}6), dried over anhydrous sodium sulfate, filtered, and the filtrate was concentrated under reduced pressure to obtain a crude product of compound **12h.**

### Step 7

Compound **12h** (55 mg, 177.56 µmol, 1 *eq),* compound **1g** (23.68 mg, 159.81 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (32.19 mg, 35.51 µmol, 0.2 *eq)* and cesium carbonate (86.78 mg, 266.34 µmol, 1.5 *eq)* were placed in a reaction flask, and the reaction flask was vacuumized and repaced with nitrogen three times, then anhydrous dioxane (3 mL) was added to the mixture and the reaction was carried out at 100 °C for 2 hours. After the reaction was completed, the reaction solution was concentrated under reduced pressure, and then purified by thin-layer preparative chromatography (methanol: dichloromethane=1:20) to obtain compound **12.** MS: *m*/*z* 422.2 [M+H]⁺;

¹H NMR (400 MHz, CDCl₃) δ ppm 9.70 (s, 1H), 8.25 (s, 1H), 7.89 (s, 1H), 7.57 (s, 1H), 6.85 (s, 1H), 4.57 (t, *J* =7.69 Hz, 2H), 3.58 (br s, 2H), 3.39 (s, 3H), 3.36 (br s, 2H), 2.50 (s, 3H), 2.45-2.49 (m, 2H), 2.07-2.23 (m, 4H).

### Embodiment 13

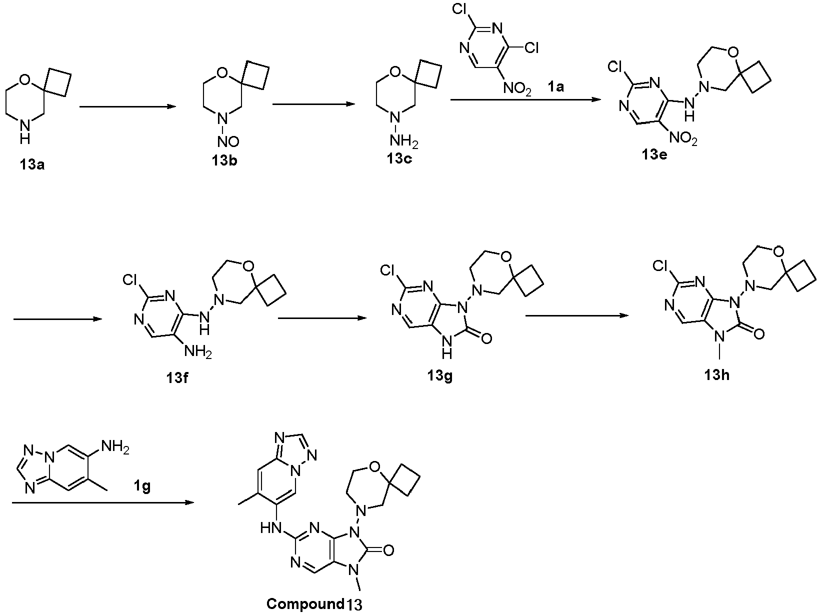

### Step 1

At 0 °C, sodium nitrite (477.39 mg, 6.92 mmol, 1.1 *eq)* was slowly added to a mixed solution of compound **13a** (800 mg, 6.29 mmol, 1 *eq)* in acetic acid (8 mL) and water (3.2 mL), and after the addition was completed, the reaction solution was reacted at 20 °C for 3 hours. After the reaction was completed, the reaction solution was concentrated under reduced pressure and purified by column chromatography (ethyl acetate: petroleum ether=0:1-1:1) to obtain compound **13b.**

¹H NMR (400 MHz, CDCl₃) δ ppm 4.18-4.23 (m, 2H), 3.86 (s, 1H), 3.78-3.83 (m, 2H), 3.53-3.59 (m, 1H), 2.0-2.12 (m, 3H), 1.63-1.95 (m, 3H)

### Step 2

At 0 °C, zinc powder (1.5 g, 22.92 mmol, 4 *eq)* was added to a mixed solution of compound **13b** (895 mg, 5.73 mmol, 1 *eq)* in acetic acid (5 mL) and methanol (5 mL), and after the addition was completed, the reaction solution was reacted at 20 °C for 1 hour. After the reaction was completed, ethyl acetate (100 mL) was added to dilute the reaction solution, and the reaction solution was filtered through celite, and the filtrate was concentrated under reduced pressure to obtain compound **13c.**

### Step 3

At 0 °C, compound **13c** (813.37 mg, 5.72 mmol, 1 *eq,* acetate) was added sequentially to a solution of compound **1a** (2.22 g, 11.44 mmol, 2 *eq)* in dioxane (50 mL), and after the addition was completed, the reaction solution was reacted at 20 °C for 1 hour. After the reaction was completed, the reaction solution was concentrated under reduced pressure and purified by column chromatography (ethyl acetate: petroleum ether=0:1-1:1) to obtain compound **13e.** MS: *m*/*z* 299.9 [M+H]⁺

¹H NMR (400 MHz, CDCl₃) δ ppm 9.07 (s, 1H), 9.03 (s, 1H), 3.80-3.83 (m, 2H), 2.93-2.98 (m, 4H), 2.08-2.22 (m, 4H), 1.83-1.94 (m, 1H), 1.61-1.74 (m, 1H).

### Step 4

Iron powder (130.43 mg, 2.34 mmol, 5 *eq)* and ammonium chloride (124.93 mg, 2.34 mmol, 5 *eq)* were added sequentially to a mixed solution of compound **13e** (140 mg, 467.11 µmol, 1 *eq)* in ethanol (3 mL) and water (3 mL), and after the addition was completed, the reaction solution was reacted at 75 °C for 1 hour. After the reaction was completed, the reaction solution was cooled to room temperature, filtered through celite and washed with ethanol (20 mL), and the washing solution was concentrated under reduced pressure to obtain a crude product. At 25 °C, the crude product was dissolved in a solution of dichloromethane/methanol (10 mL: 1 mL), stirred for 15 minutes, filtered, and the filtrate was concentrated under reduced pressure to obtain compound **13f.**

### Step 5

*N,N'*-Carbonyldiimidazole (174.33 mg, 1.08 mmol, 2 *eq)* was added to a solution of compound **13f** (145 mg, 537.57 µmol, 1 *eq)* in acetonitrile (4 mL), and after the addition was completed, the reaction solution was reacted at 80 °C for 2 hours. After the reaction was completed, the reaction solution was concentrated under reduced pressure and purified by column chromatography (methanol: dichloromethane=0:1-1:10) to obtain compound **13g.** MS: *m*/*z* 295.8 [M+H]⁺.

### Step 6

Cesium carbonate (275.44 mg, 845.38 µmol, 2 *eq)* and iodomethane (71.9 mg, 507.22 µmol, 1.2 *eq)* were added sequentially to a solution of compound **13g** (125 mg, 422.69 µmol, 1 *eq)* in *N,N-*dimethylformamide (2 mL), and after the addition was completed, the reaction solution was reacted at 20 °C for 1 hour. After the reaction was completed, water (10 mL) was added to quench the reaction, and the reaction solution was extracted with ethyl acetate (10 mL^{∗}3), washed with saturated brine (20 mL), dried over anhydrous sodium sulfate, filtered, and the filtrate was concentrated under reduced pressure and purified by column chromatography (methanol: dichloromethane=0:1-1:10) to obtain compound 13h. MS: *m*/*z* 309.9 [M+H]⁺;

¹H NMR (400 MHz, CDCl₃) δ ppm 8.02 (s, 1H), 3.83-3.84 (m, 2H), 3.40-3.44 (m, 7H), 2.10-2.24 (m, 4H), 1.81-1.90 (m, 1H), 1.56-1.63 (m, 1H).

### Step 7

Compound **13h** (45 mg, 145.28 µmol, 1 *eq),* compound **1g** (19.37 mg, 130.75 µmol, 0.9 *eq),* methanesulfonato(2-dicyclohexylphosphino-3,6-dimethoxy-2',4',6'-tri-i-propyl-1, 1'-biphenyl)(2'-amino-1,1'-biphenyl-2-yl)palladium(II) (26.34 mg, 29.06 µmol, 0.2 *eq)* and cesium carbonate (94.67 mg, 290.56 µmol, 2 *eq)* were placed in a reaction flask, and the reaction flask was vacuumized and repaced with nitrogen three times, then anhydrous dioxane (2 mL) was added to the mixture and the reaction was carried out at 100 °C for 3 hours. After the reaction was completed, the reaction solution was concentrated under reduced pressure and purified by column chromatography (methanol: dichloromethane=0:1-1:10) to obtain compound **13.** MS: *m*/*z* 422.0 [M+H]⁺;

¹H NMR (400 MHz, CDCl₃) δ ppm 9.84 (s, 1H), 8.26 (s, 1H), 7.92 (s, 1H), 7.57 (s, 1H), 6.74 (s, 1H), 3.83-3.88 (m, 2H), 3.43-3.51 (m, 4H), 3.41 (s, 3H), 2.53 (s, 3H), 2.18-2.29 (m, 3H), 2.10-2.16 (m, 1H), 1.79-1.94 (m, 1H), 1.59-1.69 (m, 1H).

### Embodiment 14

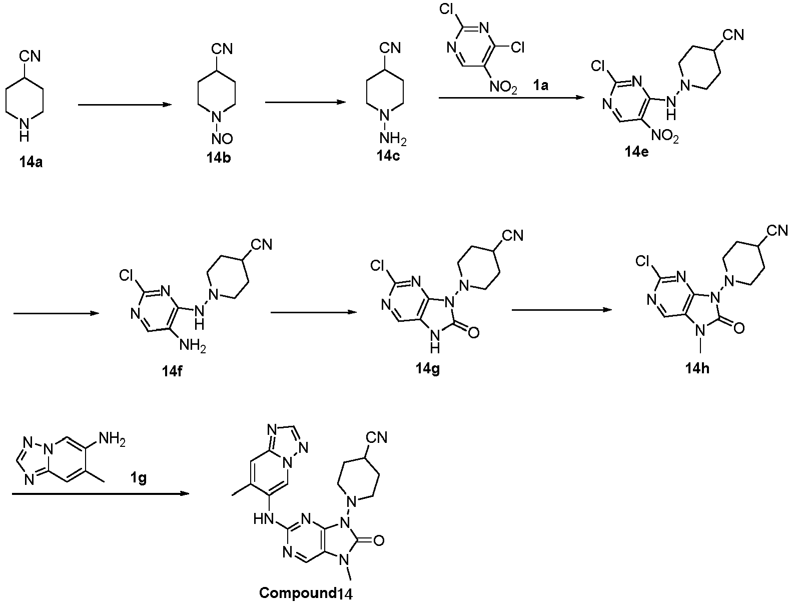

### Step 1

At 0 °C, sodium nitrite (688.97 mg, 9.99 mmol, 1.1 *eq)* was added to a mixed solution of compound **14a** (1 g, 9.08 mmol, 1 *eq)* in acetic acid (10 mL) and water (4 mL), and after the addition was completed, the reaction solution was reacted at 20 °C for 3 hours. After the reaction was completed, the reaction solution was concentrated under reduced pressure and purified by column chromatography (ethyl acetate: petroleum ether=0:1-1:1) to obtain compound **14b.**

¹H NMR (400 MHz, CDCl₃) δ ppm 4.31-4.46 (m, 2H), 3.97-4.10 (m, 1H), 3.73-3.85 (m, 1H), 2.99-3.11 (m, 1H), 2.05-2.18 (m, 2H), 1.77-1.91 (m, 2H).

### Step 2

At 0 °C, zinc powder (1.88 g, 28.69 mmol, 4 *eq)* was added to a mixed solution of compound **14b** (998 mg, 7.17 mmol, 1 *eq)* in acetic acid (8 mL) and methanol (8 mL), and after the addition was completed, the reaction solution was reacted at 20 °C for 1 hour. After the reaction was completed, ethyl acetate (20 mL) was added to dilute the reaction solution, and the reaction solution was filtered through celite, and the filtrate was concentrated under reduced pressure to obtain compound **14c.**

### Step 3

At 0 °C, compound **14c** (897.48 mg, 7.17 mmol, 1 *eq,* acetate) was added sequentially to a solution of compound **1a** (2.78 g, 14.34 mmol, 2 *eq)* in dioxane (26 mL), and after the addition was completed, the reaction solution was reacted at 20 °C for 1 hour. After the reaction was completed, the reaction solution was concentrated under reduced pressure and purified by column chromatography (ethyl acetate: petroleum ether=0:1-1:1) to obtain compound **14e.** MS: *m*/*z* 282.9 [M+H]⁺;

¹H NMR (400 MHz, CDCl₃) δ ppm 9.11 (s, 1H), 9.08 (s, 1H), 3.10-3.19 (m, 2H), 3.01-3.09 (m, 2H), 2.77-2.86 (m, 1H), 2.09-2.20 (m, 4H).

### Step 4

Iron powder (96.80 mg, 1.73 mmol, 5 *eq)* and ammonium chloride (92.72 mg, 1.73 mmol, 5 *eq)* were added sequentially to a mixed solution of compound **14e** (98 mg, 346.67 µmol, 1 *eq)* in ethanol (2 mL) and water (2 mL), and after the addition was completed, the reaction solution was reacted at 75 °C for 1 hour. After the reaction was completed, the reaction solution was cooled to room temperature, filtered through celite and washed with ethanol (20 mL), and the washing solution was concentrated under reduced pressure to obtain a crude product. At 25 °C, the crude product was dissolved in a solution of dichloromethane/methanol (10 mL: 1 mL), stirred for 15 minutes, filtered, and the filtrate was concentrated under reduced pressure to obtain compound **14f.**

### Step 5

*N,N'*-Carbonyldiimidazole (93.68 mg, 577.75 µmol, 2 *eq)* was added to a solution of compound **14f** (73 mg, 288.88 µmol, 1 *eq)* in acetonitrile (2 mL), and after the addition was completed, the reaction solution was reacted at 80 °C for 1 hour. After the reaction was completed, the reaction solution was concentrated under reduced pressure and purified by column chromatography (methanol: dichloromethane=0:1-1:10) to obtain compound **14g.** MS: *m*/*z* 278.9 [M+H]⁺.

### Step 6

Cesium carbonate (135.61 mg, 416.22 µmol, 2 *eq)* and iodomethane (35.45 mg, 249.73 µmol, 1.2 *eq)* were added sequentially to a solution of compound **14g** (58 mg, 208.11 µmol, 1 *eq)* in *N,N*-dimethylformamide (2 mL), and after the addition was completed, the reaction solution was reacted at 20 °C for 1 hour. After the reaction was completed, water (10 mL) was added to quench the reaction, and the reaction solution was extracted with 30 mL of ethyl acetate (10 mL^{∗}3), washed with saturated brine (20 mL), dried over anhydrous sodium sulfate, filtered, and the filtrate was concentrated under reduced pressure and purified by column chromatography (methanol: dichloromethane=0:1-1:10) to obtain compound **14h.** MS: *m*/*z* 292.9 [M+H]⁺;

¹H NMR (400 MHz, CDCl₃) δ ppm 8.03 (s, 1H), 3.51-3.59 (m, 2H), 3.44-3.50 (m, 2H), 3.43 (s, 3H), 2.61-2.93 (m, 1H), 2.13-2.24 (m, 4H).

### Step 7

Compound **14h** (38 mg, 129.82 µmol, 1 *eq),* compound **1g** (17.31 mg, 116.83 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (23.54 mg, 25.96 µmol, 0.2 *eq)* and cesium carbonate (84.59 mg, 259.63 µmol, 2 *eq)* were placed in a reaction flask, and the reaction flask was vacuumized and repaced with nitrogen three times, then anhydrous dioxane (2 mL) was added to the mixture and the reaction was carried out at 100 °C for 3 hours. After the reaction was completed, the reaction solution was concentrated under reduced pressure and purified by thin-layer preparative chromatography (methanol: dichloromethane=1:10) to obtain compound **14.** MS: *m*/*z* 405.0 [M+H]⁺;

¹H NMR (400 MHz, CDCl₃) δ ppm 9.75 (s, 1H), 8.27 (s, 1H), 7.93 (s, 1H), 7.58 (s, 1H), 6.81 (s, 1H), 3.52-3.62 (m, 4H), 3.41 (s, 3H), 2.75-2.87 (m, 1H), 2.51 (s, 3H), 2.16-2.24 (m, 4H).

### Embodiment 15

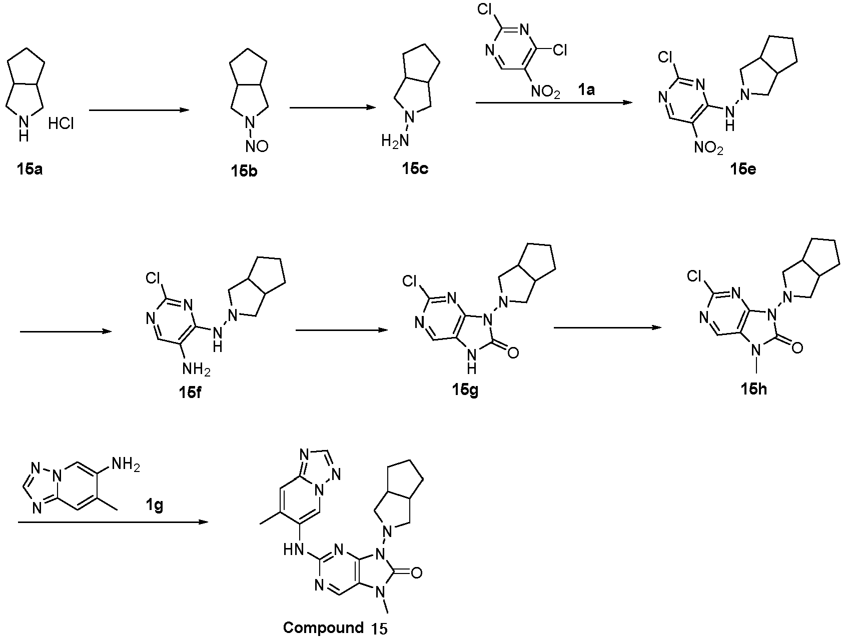

### Step 1

At 0 °C, a solution of sodium nitrite (1.40 g, 20.32 mmol, 1 *eq)* in water (3 mL) was slowly added to a mixed solution of compound 15a (3 g, 20.32 mmol, 1 *eq,* hydrochloride) in acetic acid (30 mL) and water (10 mL), and after the addition was completed, the reaction solution was reacted at 25 °C for 16 hours. After the reaction was completed, water (10 mL) was added to quench the reaction, and the reaction solution was concentrated under reduced pressure, diluted with water (30 mL), extracted with ethyl acetate (50 mL^{∗}3), dried over anhydrous sodium sulfate, filtered, and the filtrate was concentrated under reduced pressure and purified by column chromatography (ethyl acetate: petroleum ether=0:1-1:2) to obtain compound **15b.**

¹H NMR (400 MHz, CDCl₃) δ ppm 4.43 (dd, *J*=12.69, 7.82 Hz, 1H), 4.10 (dd, *J*=12.63, 4.50 Hz, 1H), 3.79 (dd, *J*=15.20, 8.69 Hz, 1H), 3.42 (dd, *J*=15.32, 4.69 Hz, 1H), 2.69-2.89 (m, 2H), 1.85-2.00 (m, 2H), 1.61-1.82 (m, 2H), 1.41-1.56 (m, 2H).

### Step 2

At 0 °C, zinc powder (1.51 g, 23.11 mmol, 4 *eq)* was added to a mixed solution of compound **15b** (0.81 g, 5.78 mmol, 1 *eq)* in acetic acid (5 mL) and methanol (5 mL), and after the addition was completed, the reaction solution was reacted at 20 °C for 2 hours. After the reaction was completed, ethyl acetate (100 mL) was added to dilute the reaction solution, and the reaction solution was filtered through celite, and the filtrate was concentrated under reduced pressure to obtain compound **15c.**

### Step 3

At 0 °C, a solution of compound **15c** (2.6 g, 6.98 mmol, 1 *eq,* acetate) in dioxane (3 mL) and triethylamine (2.83 g, 27.92 mmol, 4 *eq,* 3.89 mL) were added sequentially to a solution of compound 1a (2.71 g, 13.96 mmol, 2 *eq)* in dioxane (100 mL), and after the addition was completed, the reaction solution was reacted at 20 °C for 1 hour. After the reaction was completed, water (30 mL) was added to quench the reaction, and the reaction solution was concentrated under reduced pressure, diluted with water (50 mL), extracted with ethyl acetate (60 mL^{∗}4), dried over anhydrous sodium sulfate, filtered, and the filtrate was concentrated under reduced pressure and purified by column chromatography (ethyl acetate: petroleum ether=0:1-1:6) to obtain compound 15e. MS: *m*/*z* 284.0 [M+H]⁺.

¹H NMR (400 MHz, CDCl₃) δ ppm 9.04 (s, 1H), 8.99 (br s, 1H), 3.37 (br t, *J*=7.94 Hz, 2H), 2.76 (br s, 2H), 2.50-2.59 (m, 2H), 1.65-1.77 (m, 4H), 1.25 (br s, 2H).

### Step 4

Iron powder (113.19 mg, 2.03 mmol, 5 *eq)* and ammonium chloride (108.41 mg, 2.03 mmol, 5 *eq)* were added sequentially to a mixed solution of compound **15e** (115 mg, 405.34 µmol, 1 *eq)* in ethanol (2 mL) and water (2 mL), and after the addition was completed, the reaction solution was reacted at 75 °C for 1 hour. After the reaction was completed, the reaction solution was cooled to room temperature, filtered through celite and concentrated under reduced pressure to obtain acrude product. At 25 °C, the crude product was dissolved in a solution of dichloromethane/methanol (10 mL: 1 mL), stirred for 15 minutes, filtered, and the filtrate was concentrated under reduced pressure to obtain compound **15f.**

### Step 5

*N,N*'-Carbonyldiimidazole (230.06 mg, 1.42 mmol, 3 *eq)* was added to a solution of compound **15f** (120 mg, 472.94 µmol, 1 *eq)* in acetonitrile (4 mL), and after the addition was completed, the reaction solution was reacted at 80 °C for 1 hour. After the reaction was completed, the reaction solution was concentrated under reduced pressure and purified by thin-layer preparative chromatography (ethyl acetate: petroleum ether=1:0) to obtain compound **15g.** MS: *m*/*z* 280.0 [M+H]⁺.

### Step 6

Cesium carbonate (172.39 mg, 529.09 µmol, 4 *eq)* and iodomethane (56.32 mg, 396.82 µmol, 3 *eq)* were added sequentially to a solution of compound **15g** (37 mg, 132.27 µmol, 1 *eq)* in *N,N-*dimethylformamide (1.5 mL), and after the addition was completed, the reaction solution was reacted at 20 °C for 1 hour. After the reaction was completed, water (3 mL) was added to quench the reaction, and the reaction solution was concentrated under reduced pressure, then diluted with water (3 mL), extracted with ethyl acetate (10 mL^{∗}2), dried over anhydrous sodium sulfate, filtered, and the filtrate was concentrated under reduced pressure to obtain a crude product of compound **15h.**

### Step 7

Compound **15h** (26.5 mg, 90.21 µmol, 1 *eq),* compound **1g** (12.03 mg, 81.19 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (16.36 mg, 18.04 µmol, 0.2 *eq)* and cesium carbonate (44.09 mg, 135.32 µmol, 1.5 *eq)* were placed in a reaction flask, and the reaction flask was vacuumized and repaced with nitrogen three times, then anhydrous dioxane (2 mL) was added to the mixture and the reaction was carried out at 100 °C for 2 hours. After the reaction was completed, the reaction solution was concentrated under reduced pressure, and then purified by thin-layer preparative chromatography (methanol: dichloromethane=1:20) to obtain compound **15.** MS: *m*/*z* 406.2 [M+H]⁺;

¹H NMR (400 MHz, CDCl₃) δ ppm 9.70 (s, 1H), 8.24 (s, 1H), 7.89 (s, 1H), 7.55 (s, 1H), 6.80 (s, 1H), 3.58 (s, 2H), 3.39 (s, 3H), 3.29 (s, 2H), 2.79 (s, 2H), 2.48 (s, 3H), 1.62-1.89 (m, 6H).

### Embodiment 16

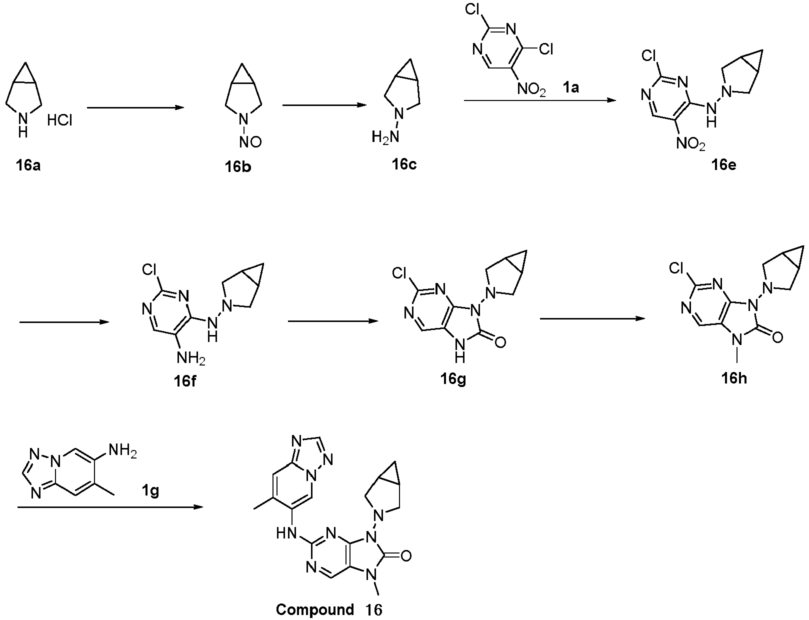

### Step 1

At 0 °C, a solution of sodium nitrite (634.66 mg, 9.20 mmol, 1.1 *eq)* in water (1 mL) was slowly added to a mixed solution of compound **16a** (1 g, 8.36 mmol, 1 *eq,* hydrochloride) in acetic acid (10 mL) and water (3.5 mL), and after the addition was completed, the reaction solution was reacted at 20 °C for 3 hours. After the reaction was completed, saturated aqueous sodium bicarbonate solution (200 mL) was added to quench the reaction, and the reaction solution was extracted with ethyl acetate (100 mL^{∗}2), dried over anhydrous sodium sulfate, filtered, and the filtrate was concentrated under reduced pressure and purified by column chromatography (ethyl acetate: petroleum ether = 0:1-1:4) to obtain compound **16b.**

¹H NMR (400 MHz, CDCl₃) δ ppm 4.51 (d, *J*=12.05 Hz, 1H), 4.31 (dd, *J*=11.92, 4.14 Hz, 1H), 4.08 (d, *J*=14.4 Hz, 1H), 3.38 (ddd, *J*=14.31, 4.64, 1.13 Hz, 1H), 1.57-1.73 (m, 2H), 0.83-0.95 (m, 1H), 0.08-0.20 (m, 1H).

### Step 2

At 0 °C, zinc powder (1.70 g, 26.04 mmol, 4 *eq)* was added to a mixed solution of compound **16b** (0.73 g, 6.51 mmol, 1 *eq)* in acetic acid (3 mL) and methanol (3 mL), and after the addition was completed, the reaction solution was reacted at 25 °C for 1 hour. After the reaction was completed, ethyl acetate (50 mL) was added to dilute the reaction solution, and the reaction solution was filtered through celite, and the filtrate was concentrated under reduced pressure to obtain compound **16c.**

### Step 3

At 0 °C, compound **16c** (2.2 g, 22.42 mmol, 1 *eq)* and diisopropylethylamine (14.49 g, 112.08 mmol, 5 *eq,* 19.52 mL) were added sequentially to a solution of compound **1a** (4.35 g, 22.42 mmol, 1 *eq)* in ethanol (30 mL), and after the addition was completed, the reaction solution was reacted at 0 °C for 1 hour. After the reaction was completed, water (100 mL) was added to quench the reaction, and the reaction solution was extracted with dichloromethane (100 mL^{∗}2), dried over anhydrous sodium sulfate, filtered, and the filtrate was concentrated under reduced pressure and purified by column chromatography (ethyl acetate: petroleum ether=0:1-1:9) to obtain compound **16e.** MS: *m*/*z* 255.7 [M+H]⁺;

¹H NMR (400 MHz, CDCl₃) δ ppm 9.03 (s, 1H), 8.99 (br s, 1H), 3.37 (d, *J*=8.25 Hz, 2H), 3.09 (br d, *J*=7.88 Hz, 2H), 1.55-1.58 (m, 2H), 0.87 (q, *J*=4.17 Hz, 1H), 0.58-0.68 (m, 1H).

### Step 4

Iron powder (87.37 mg, 1.56 mmol, 4 *eq)* and ammonium chloride (83.69 mg, 1.56 mmol, 4 *eq)* were added sequentially to a solution of compound **16e** (100 mg, 391.14 µmol, 1 *eq)* in ethanol (2 mL) and water (2 mL), and after the addition was completed, the reaction solution was reacted at 80 °C for 2 hours. After the reaction was completed, the reaction solution was cooled to room temperature, filtered through celite and washed with ethanol (50 mL), and the filtrate was concentrated under reduced pressure to obtain a crude product. At 25 °C, the crude product was dissolved in a solution of dichloromethane/methanol (50 mL: 10 mL), stirred for 15 minutes, filtered, and the filtrate was concentrated under reduced pressure to obtain compound **16f.**

### Step 5

*N,N*'-Carbonyldiimidazole (71.85 mg, 443.11 µmol, 1 *eq)* was added to a solution of compound **16f** (100 mg, 443.11 µmol, 1 *eq)* in acetonitrile (2 mL), and after the addition was completed, the reaction solution was reacted at 80 °C for 1 hour. After the reaction was completed, the reaction solution was extracted with ethyl acetate (20 mL^{∗}2), washed with water (20 mL^{∗}2), and purified by thin-layer preparative chromatography (ethyl acetate: petroleum ether=1:2) to obtain compound **16g.** MS: *m*/*z* 252.0 [M+H]⁺;

¹H NMR (400 MHz, DMSO-*d*₆) δ ppm 8.12 (s, 1H), 3.73 (d, *J*=7.03 Hz, 2H), 3.11 (d, J=7.78 Hz, 2H), 1.55-1.61 (m, 2H), 0.73-0.77 (m, 1H), 0.57-0.65 (m, 1H).

### Step 6

Cesium carbonate (51.78 mg, 158.94 µmol, 2 *eq)* and iodomethane (11.28 mg, 79.47 µmol, 1 *eq)* were added sequentially to a solution of compound **16g** (20 mg, 79.47 µmol, 1 *eq)* in *N,N-*dimethylformamide (3 mL), and after the addition was completed, the reaction solution was reacted at 25 °C for 3 hours. After the reaction was completed, the reaction solution was diluted with water (50 mL), extracted with ethyl acetate (20 mL^{∗}2), washed with saturated brine (100 mL), dried over anhydrous sodium sulfate, filtered, and the filtrate was concentrated under reduced pressure to obtain a crude product of compound **16h.**

### Step 7

Compound **16h** (28 mg, 105.38 µmol, 1 *eq),* compound **1g** (14.05 mg, 94.84 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (19.11 mg, 21.08 µmol, 0.2 *eq)* and cesium carbonate (51.50 mg, 158.07 µmol, 1.5 *eq)* were placed in a reaction flask, and the reaction flask was vacuumized and repaced with nitrogen three times, then anhydrous dioxane (3 mL) was added to the mixture and the reaction was carried out at 100 °C for 3 hours. After the reaction was completed, the reaction solution was filtered and washed with ethyl acetate (50 mL), and the filtrate was concentrated under reduced pressure, then purified by thin-layer preparative chromatography (methanol: dichloromethane= 1:20) to obtain compound **16.** MS: *m*/*z* 378.3 [M+H]⁺;

¹H NMR (400 MHz, DMSO-*d*₆) δ ppm 9.16 (s, 1H), 8.72 (s, 1H), 8.36 (s, 1H), 8.08 (s, 1H), 7.69 (s, 1H), 3.76 (br d, *J*=7.03 Hz, 2H), 3.27 (s, 3H), 3.11 (br d, *J*=7.78 Hz, 2H), 2.39 (s, 3H), 1.52-1.61 (m, 2H), 0.70-0.76 (m, 1H), 0.53-0.62 (m, 1H).

### Embodiment 17

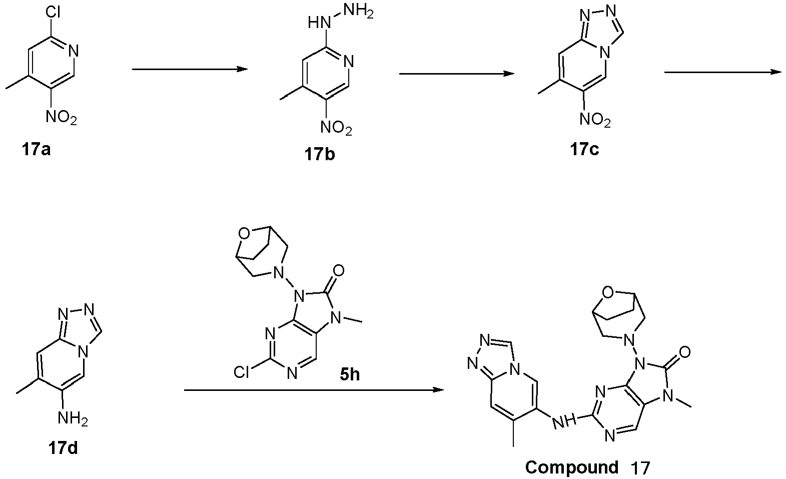

### Step 1

At 15 °C, hydrazine monohydrate (4.61 g, 120.0 mmol, 4 *eq)* was added to a solution of compound **17a** (5.18 g, 30.0 mmol, 1 *eq)* in 1,4-dioxane (30 mL), and after the addition was completed, the reaction was carried out at 15 °C for 8 hours. After the reaction was completed, the reaction solution was filtered, and the filter cake was washed with water (50 mL) and dried under reduced pressure to obtain compound **17b.** MS: *m*/*z.* 168.9 [M+H]⁺.

### Step 2

At 25 °C, trimethyl orthoformate (12.73 g, 120 mmol, 4 *eq)* and trifluoroacetic acid (0.684 g, 6 mmol, 0.2 *eq)* were added sequentially to a solution of compound **17b** (5.04 g, 30 mmol, 1 *eq)* in dichloromethane (60 mL), and after the addition was completed, the reaction was carried out at 25 °C for 2 hours. After the reaction was completed, the reaction solution was concentrated under reduced pressure and purified by column chromatography (methanol: dichloromethane=0:1-1:9) to obtain compound **17c.** MS: *m*/*z.* 178.9 [M+H]⁺.

¹H NMR (400 MHz, DMSO-*d*₆) δ ppm 9.70 (s, 1H), 9.35 (s, 1H), 7.84 (s, 1H), 2.59 (s, 3H).

### Step 3

Iron powder (6.14 g, 110 mmol, 5 *eq)* and ammonium chloride (5.88 g, 110 mmol, 5 *eq)* were added sequentially to a mixed solution of compound **17c** (3.92 g, 22 mmol, 1 *eq)* in ethanol (80 mL) and water (20 mL), and after the addition was completed, the reaction was carried out at 70 °C for 3 hours. After the reaction was completed, the reaction solution was filtered and washed with ethanol (20 mL), and the filtrate was concentrated under reduced pressure and purified by column chromatography (methanol: dichloromethane=0:1-1:9) to obtain compound **17d.** MS: *m*/*z* 148.8 [M+H]⁺.

¹H NMR (400 MHz, DMSO-*d*₆) δ ppm 8.11 (s, 1H), 8.07 (s, 1H), 7.46 (s, 1H), 5.01 (s, 2H), 2.25 (d, *J*=0.88 Hz, 3H).

### Step 4

A solution of compound **17d** (29.63 mg, 200 µmol, 1 *eq),* compound **5h** (59.14 mg, 200.00 µmol, 1 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (27.19 mg, 30.00 µmol, 0.15 *eq)* and cesium carbonate (97.75 mg, 300 µmol, 1.5 *eq)* in dioxane (2.5 mL) was repaced with nitrogen three times, and the reaction was carried out at 100 °C for 2 hours under nitrogen protection. After the reaction was completed, the filtrate was filtered through celite, and the filtrate was concentrated under reduced pressure to obtain a crude product. The crude product was purified by thin-layer preparative chromatography (methanol: dichloromethane=1:12) to obtain compound **17.** MS: m/z 408.2 [M+H] ⁺.

¹H NMR (400 MHz, CDCl₃) δ ppm 9.88 (s, 1H), 8.28 (s, 1H), 7.91 (s, 1H), 7.60 (s, 1H), 6.93 (s, 1H), 4.46-4.54 (m, 2H), 4.05 (dd, J=9.88, 2.13 Hz, 2H), 3.41 (s, 3H), 2.86 (d, J=9.63 Hz, 2H), 2.55 (s, 3H), 2.29-2.36 (m, 2H), 2.01-2.09 (m, 2H).

### Embodiment 18

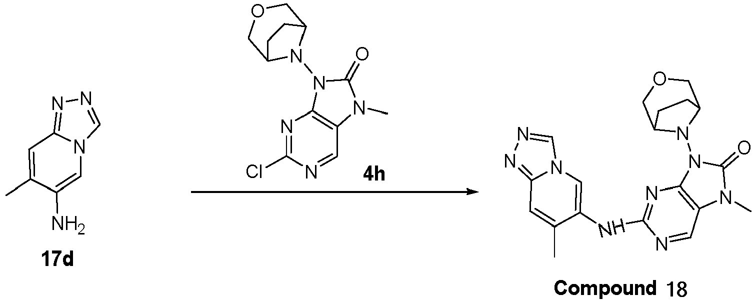

A solution of compound **17d** (29.63 mg, 200 µmol, 1 *eq),* compound **4h** (59.14 mg, 200.00 µmol, 1 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (27.19 mg, 30.00 µmol, 0.15 *eq)* and cesium carbonate (97.75 mg, 300 µmol, 1.5 *eq)* in dioxane (2.5 mL) was replaced with nitrogen three times, and the reaction was carried out at 100 °C for 2 hours under nitrogen protection. After the reaction was completed, the reaction solution was filtered through celite, concentrated under reduced pressure to obtain a crude product, and the crude product was purified by thin-layer preparative chromatography (methanol: dichloromethane=1:12) to obtain compound **18.** MS: *m*/*z* 408.1 [M+H] ⁺.

¹H NMR (400 MHz, CDCl₃) δ ppm 9.77 (s, 1H), 8.27 (s, 1H), 7.90 (s, 1H), 7.59 (s, 1H), 6.77 (s, 1H), 4.13-4.22 (m, 2H), 3.85-3.94 (m, 2H), 3.69-3.75 (m, 2H), 3.40 (s, 3H), 2.52 (s, 3H), 2.37-2.46 (m, 2H), 2.09-2.18 (m, 2H).

### Embodiment 19

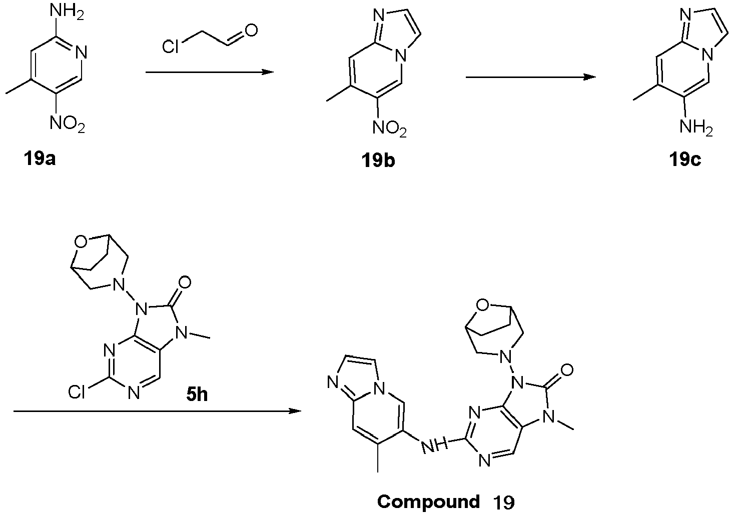

### Step 1

Chloroacetaldehyde (1.92 g, 9.80 mmol, 1.58 mL, 40 % purity, 1.5 *eq)* was added to a solution of compound **19a** (1 g, 6.53 mmol, 1 *eq)* in ethanol (10 mL), and after the addition was completed, the reaction solution was reacted at 70 °C for 2 hours. After the reaction was completed, the reaction solution was concentrated under reduced pressure and purified by column chromatography (methanol: dichloromethane=0:1-1:9) to obtain compound **19b.**

¹H NMR (400 MHz, DMSO-*d*₆) δ ppm 9.95 (s, 1H), 8.34 (d, *J*=1.50 Hz, 1H), 8.15 (d, *J*=1.88 Hz, 1H), 7.93 (s, 1H), 2.71 (s, 3H).

### Step 2

Iron powder (1.51 g, 27.09 mmol, 4 *eq)* and ammonium chloride (1.45 g, 27.09 mmol, *4 eq)* were added sequentially to a mixed solution of compound **19b** (1.2 g, 6.77 mmol, 1 *eq)* in ethanol (6 mL) and water (6 mL), and after the addition was completed, the reaction solution was reacted at 80 °C for 1 hour. After the reaction was completed, the reaction solution was cooled to room temperature, filtered through celite, and the filtrate was concentrated under reduced pressure to obtain a crude product. At 25 °C, the crude product was treated with sonication in dichloromethane: methanol=30 mL: 3 mL for 5 minutes, filtered, and the filtrate was concentrated to obtain compound **19c.** MS: *m*/*z* 147.9 [M+H]⁺.

### Step 3

Compound **5h** (50 mg, 169.08 µmol, 1 *eq),* compound **19c** (22.40 mg, 152.17 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (30.65 mg, 33.82 µmol, 0.2 *eq)* and cesium carbonate (82.63 mg, 253.61 µmol, 1.5 *eq)* were placed in a reaction flask, and the reaction flask was vacuumized and repaced with nitrogen three times, then anhydrous dioxane (2 mL) was added to the mixture, and after the addition was completed, the reaction solution was reacted at 100 °C for 2 hours. After the reaction was completed, the reaction solution was concentrated under reduced pressure, and the residue was first purified by column chromatography (methanol: dichloromethane=0:1-1:9) to obtain a crude product, and then purified by preparative high performance liquid chromatography (neutral conditions: chromatographic column: Phenomenex Gemini-NX 80^{∗}30 mm^{∗}3 µm; mobile phase: [Water (10 mM ammonium bicarbonate)-acetonitrile]; % acetonitrile: 16 %-46 %, 9 minutes) to obtain compound **19.** MS: *m*/*z* 407.3 [M+H]⁺.

¹H NMR (400 MHz, CDCl₃) δ ppm 9.46 (s, 1H), 7.91 (s, 1H), 7.62 (s, 1H), 7.56 (s, 1H), 7.48 (s, 1H), 6.77 (s, 1H), 4.47-4.56 (m, 2H), 4.07-4.15 (m, 2H), 3.39 (s, 3H), 2.82-2.93 (m, 2H), 2.46 (s, 3H), 2.26-2.35 (m, 2H), 2.01-2.11 (m, 2H).

### Embodiment 20

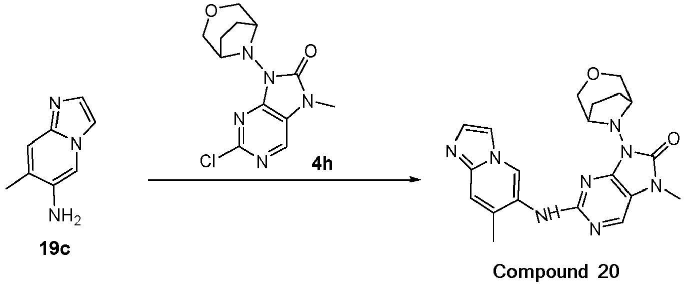

### Step 1

Compound **4h** (50 mg, 169.08 µmol, 1 *eq),* compound **19c** (22.40 mg, 152.17 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (30.65 mg, 33.82 µmol, 0.2 *eq)* and cesium carbonate (82.63 mg, 253.61 µmol, 1.5 *eq)* were placed in a reaction flask, and the reaction flask was vacuumized and repaced with nitrogen three times, then anhydrous dioxane (2 mL) was added to the mixture, and after the addition was completed, the reaction solution was reacted at 100 °C for 2 hours. After the reaction was completed, the reaction solution was concentrated under reduced pressure, and first purified by column chromatography (methanol: dichloromethane=0:1-1:9) to obtain a crude product, then purified by preparative high performance liquid chromatography (neutral condition: chromatographic column: Phenomenex Gemini-NX 80^{∗}30 mm^{∗}3 µm; mobile phase: [Water (10 mM ammonium bicarbonate)-acetonitrile]; % acetonitrile: 13 %-43 %, 9 minutes) to obtain compound **20.** MS: *m*/*z* 407.3 [M+H]⁺.

¹H NMR (400 MHz, CDCl₃) δ ppm 9.20 (s, 1H), 7.87 (s, 1H), 7.57 (s, 1H), 7.51 (s, 1H), 7.47 (s, 1H), 6.65 (s, 1H), 4.07-4.16 (m, 2H), 3.79-3.88 (m, 2H), 3.66-3.75 (m, 2H), 3.38 (s, 3H), 2.35-2.47 (m, 5H), 2.05-2.15 (m, 2H).

### Embodiment 21

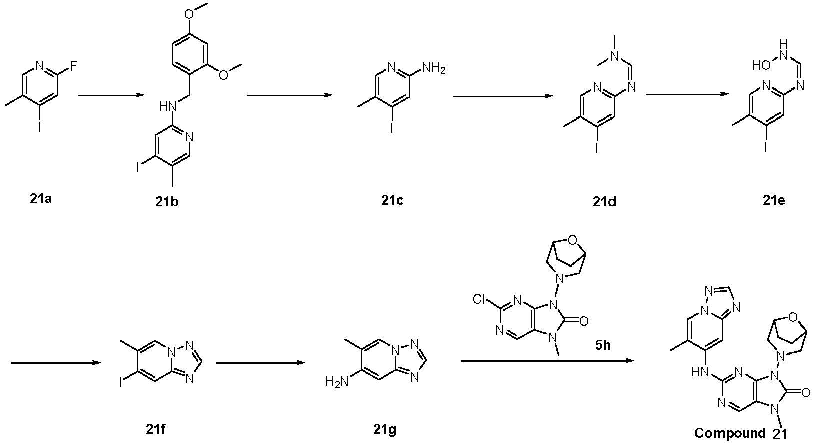

### Step 1

2,2-Dimethoxybenzylamine (6.35 g, 37.97 mmol, 5.72 mL, 3 *eq)* and *N,N-*diisopropylethylamine (4.91 g, 37.97 mmol, 6.61 mL, 3 *eq)* were added to a solution of compound **21a** (3 g, 12.66 mmol, 1 *eq)* in dioxane (20 mL), and after the addition was completed, the reaction solution was reacted at 110 °C for 16 hours. After the reaction was completed, the reaction solution was cooled to room temperature, concentrated under reduced pressure, and purified by column chromatography (ethyl acetate: petroleum ether=0:1-1:3) to obtain compound **21b.** MS: *m*/*z* 385.0 [M+H]⁺.

¹H NMR (400 MHz, CDCl₃) δ ppm 7.84 (s, 1H), 7.16-7.20 (m, 1H), 6.95 (s, 1H), 6.40-6.48 (m, 2H), 4.78 (s, 1H), 4.31-4.37 (m, 2H), 3.83 (s, 3H), 3.79 (s, 3H), 2.22 (s, 3H).

### Step 2

Trifluoroacetic acid (6.93 g, 60.78 mmol, 4.5 mL, 27 *eq)* was added to a solution of compound **21b** (865 g, 2.25 mmol, 1 *eq)* in dichloromethane (9 mL), and after the addition was completed, the reaction solution was reacted at 20 °C for 12 hours. After the reaction was completed, the reaction solution was concentrated under reduced pressure, and saturated sodium bicarbonate (20 mL) was added to adjust the pH to 8-9, then the reaction solution was extracted with ethyl acetate (10 mL^{∗}3), washed with saturated brine (20 mL), dried over anhydrous sodium sulfate, filtered, and the filtrate was concentrated under reduced pressure and purified by column chromatography (ethyl acetate: petroleum ether=0:1-1:1) to obtain compound **21c.** MS: *m*/*z* 234.9 [M+H] ⁺.

### Step 3

*N,N*-Dimethylformamide dimethyl acetal (572.80 mg, 4.81 mmol, 638.58 µL, 3 *eq)* was added to a solution of compound **21c** (375 mg, 1.6 mmol, 1 *eq)* in toluene (4 mL), and after the addition was completed, the reaction solution was reacted at 110 °C for 2 hours. After the reaction was completed, the reaction solution was concentrated under reduced pressure to obtain a crude product of compound **21d.**

### Step 4

Hydroxylamine hydrochloride (298.04 mg, 4.29 mmol, 2 *eq)* was added to a solution of compound **21d** (620 mg, 2.14 mmol, 1 *eq)* in methanol (6 mL), and after the addition was completed, the reaction solution was reacted at 70 °C for 1 hour. After the reaction was completed, the reaction solution was concentrated under reduced pressure to obtain compound **21e.**

### Step 5

At 0 °C, trifluoroacetic anhydride (1.30 g, 6.17 mmol, 858.47 µL, 1.5 *eq)* was added to a solution of compound **21e** (1.14 g, 4.11 mmol, 1 *eq)* in tetrahydrofuran (12 mL), and after the addition was completed, the reaction solution was reacted at 20 °C for 18 hours. After the reaction was completed, the reaction solution was concentrated under reduced pressure and purified by column chromatography (ethyl acetate: petroleum ether=0:1-1:1) to obtain compound **21f.** MS: *m*/*z* 259.9 [M+H] ⁺.

### Step 6

Compound 21f (175 mg, 675.55 µmol, 1 *eq),* benzophenoneimine (134.68 mg, 743.11 µmol, 124.70 µL, 1.1 *eq),* tris(dibenzylideneacetone)dipalladium (12.37 mg, 13.51 µmol), 0.02 *eq),* 1,1'-binaphthyl-2,2'-bisdiphenylphosphine (11.74 mg, 20.29 µmol, 0.03 *eq)* and sodium *tert*-butoxide (97.38 mg, 1.01 mmol, 1.5 *eq)* were placed in a reaction flask, and the reaction flask was vacuumized and repaced with nitrogen three times, then anhydrous toluene (3 mL) was added to the mixture, and after the addition was completed, the reaction solution was reacted at 90 ° C for 3 hours. After the reaction was completed, the reaction solution was concentrated under reduced pressure, then tetrahydrofuran (10 mL) and 3 mol/L hydrochloric acid solution (3 mL) were added, and the mixture was stirred at 20 °C for 0.5 hours, and then diluted with water (20 mL), extracted with ethyl acetate (15 mL^{∗}2), and the organic phase was discarded. The pH of aqueous phase was adjusted to 9-11 by adding an appropriate amount of aqueous ammonium hydroxide solution, then the aqueous phase was extracted with ethyl acetate (10 mL^{∗}3), washed with saturated brine (20 mL), dried over anhydrous sodium sulfate, filtered, and the filtrate was concentrated under reduced pressure and purified by column chromatography (methanol: dichloromethane=0:1-1:9) to obtain compound **21g.** MS: *m*/*z* 148.9 [M+H] ⁺.

¹H NMR (400 MHz, CDCl₃) δ ppm 8.20 (s, 1H), 8.08 (s, 1H), 6.78 (s, 1H), 4.17 (s, 2H), 2.20-2.24 (m, 3H).

### Step 7

Compound **21g** (22.55 mg, 152.17 µmol, 0.9 *eq),* compound **5h** (50 mg, 169.08 µmol, 1 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (30.65 mg, 33.82 µmol, 0.2 *eq)* and cesium carbonate (110.18 mg, 338.15 µmol, 2 *eq)* were placed in a reaction flask, and the reaction flask was vacuumized and repaced with nitrogen three times, then anhydrous dioxane (2 mL) was added to the mixture and the reaction was carried out at 100 °C for 2 hours. After the reaction was completed, the reaction solution was concentrated under reduced pressure and purified by thin-layer preparative chromatography (methanol: dichloromethane=1:10) to obtain compound **21.** MS: *m*/*z* 408.1 [M+H] ⁺.

¹H NMR (400 MHz, CDCl₃) δ ppm 9.03 (s, 1H), 8.35 (s, 1H), 8.21 (s, 1H), 7.97 (s, 1H), 7.11 (s, 1H), 4.46-4.54 (m, 2H), 4.01-4.11 (m, 2H), 3.42 (s, 3H), 2.80-2.91 (m, 2H), 2.45 (s, 3H), 2.30-2.39 (m, 2H), 2.01-2.08 (m, 2H).

### Embodiment 22

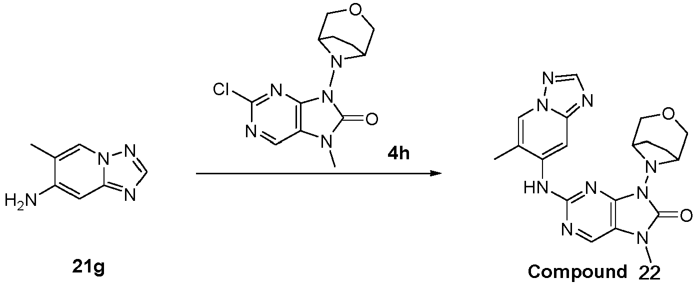

### Step 1

Compound **4h** (50 mg, 169.08 µmol, 1 *eq),* compound **21g** (22.55 mg, 152.17 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (30.65 mg, 33.82 µmol, 0.2 *eq)* and cesium carbonate (110.18 mg, 338.15 µmol, 2 *eq)* were placed in a reaction flask, and the reaction flask was vacuumized and repaced with nitrogen three times, then anhydrous dioxane (3 mL) was added to the mixture and the reaction was carried out at 100 °C for 2 hours. After the reaction was completed, the reaction solution was concentrated under reduced pressure and purified by thin-layer preparative chromatography (methanol: dichloromethane=1:10) to obtain compound **22.** MS: *m*/*z* 430.1 [M+Na]⁺.

¹H NMR (400 MHz, CDCl₃) δ ppm 8.97 (s, 1H), 8.34 (s, 1H), 8.21 (s, 1H), 7.96 (s, 1H), 7.10 (s, 1H), 4.13-4.19 (m, 2H), 3.87-3.93 (m, 2H), 3.70-3.76 (m, 2H), 3.41 (s, 3H), 2.41-2.49 (m, 5H), 2.14-2.20 (m, 2H).

### Embodiment 23

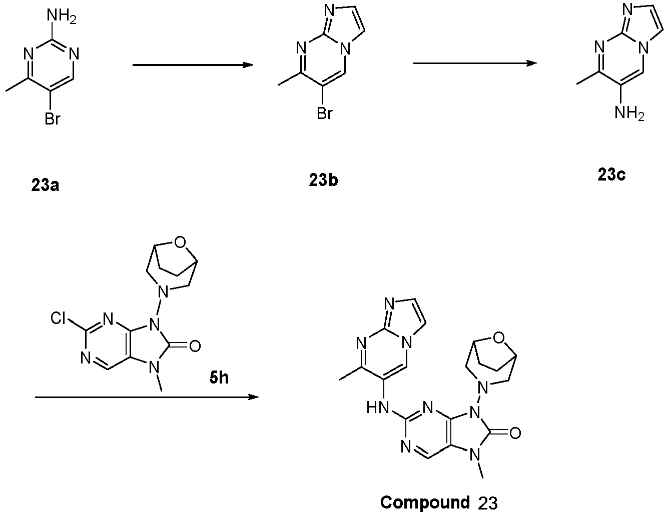

### Step 1

Chloroacetaldehyde (5.26 g, 26.80 mmol, 670.12 µL, 40 % purity, 1.2 *eq)* was added to a mixed solution of compound **23a** (4.2 g, 22.34 mmol, 1 *eq)* in ethanol (42 mL) and water (17.5 mL), and after the addition was completed, the reaction solution was reacted at 100 °C for 16 hours. After the reaction was completed, the reaction solution was diluted with saturated sodium bicarbonate solution (60 mL), extracted with ethyl acetate (50 mL^{∗}3), washed with saturated brine (30 mL), dried over anhydrous sodium sulfate, filtered, and the filtrate was concentrated under reduced pressure and purified by column chromatography (ethyl acetate: petroleum ether=0:1-1:1) to obtain compound **23b.** MS: m/z 213.8 [M+H+2]⁺.

¹H NMR (400 MHz, CDCl₃) δ ppm 8.57 (s, 1H), 7.84-7.86 (m, 1H), 7.48-7.50 (m, 1H), 2.79 (s, 3H).

### Step 2

Compound **23b** (1 g, 4.72 mmol, 1 *eq),* benzophenoneimine (940.15 mg, 5.19 mmol, 870.51 µL, 1.1 *eq),* tris(dibenzylideneacetone)dipalladium (431.85 mg, 471.59 µmol, 0.1 *eq),* 4,5-bis(diphenylphosphino)-9,9-dimethylxanthene (545.75 mg, 943.19 µmol, 0.2 *eq)* and cesium carbonate (3.07 g, 9.43 mmol, 2 *eq)* were placed in a reaction flask, and the reaction flask was vacuumized and repaced with nitrogen three times, then anhydrous dioxane (30 mL) was added to the mixture and the reaction was carried out at 120 °C for 3 hours. After the reaction was completed, the reaction solution was cooled to room temperature, concentrated under reduced pressure, then tetrahydrofuran (10 mL) and 3 mol/L hydrochloric acid solution (12 mL) were added, and the reaction solution was stirred at 20 °C for half an hour, then diluted with water (20 mL), extracted with ethyl acetate (20 mL^{∗}3), and the organic phase was discarded. An appropriate amount of ammonium hydroxide solution was added to the aqueous phase to adjust the pH to 9-11, then the aqueous phase was directly concentrated under reduced pressure and purified by column chromatography (methanol: dichloromethane=0:1-1:9) to obtain compound **23c.** MS: *m*/*z* 148.8 [M+H] ⁺.

### Step 3

Compound **23c** (40.08 mg, 270.52 µmol, 2 *eq),* compound **5h** (40 mg, 135.26 µmol, 1 *eq),* tris(dibenzylideneacetone)dipalladium (12.39 mg, 13.53 µmol, 0.1 *eq),* 4,5-bis(diphenylphosphino)-9,9-dimethylxanthene (15.65 mg, 27.05 µmol, 0.2 *eq)* and cesium carbonate (88.14 mg, 270.52 µmol, 2 *eq)* were placed in a reaction flask, and the reaction flask was vacuumized and repaced with nitrogen three times, then anhydrous dioxane (2 mL) was added to the mixture and the reaction was carried out at 100 °C for 2 hours. After the reaction was completed, the reaction solution was concentrated under reduced pressure and purified by thin-layer preparative chromatography (methanol: dichloromethane=1:10) to obtain compound **23.** MS: *m*/*z* 408.2 [M+H] ⁺.

¹H NMR (400 MHz, CDCl₃) δ ppm 9.74 (s, 1H), 7.93 (s, 1H), 7.73 (s, 1H), 7.53 (s, 1H), 6.83 (s, 1H), 4.48-4.54 (m, 2H), 4.03-4.10 (m, 2H), 3.41 (s, 3H), 2.84-2.90 (m, 2H), 2.74 (s, 3H), 2.25-2.32 (m, 2H), 2.03-2.11 (m, 2H).

### Embodiment 24

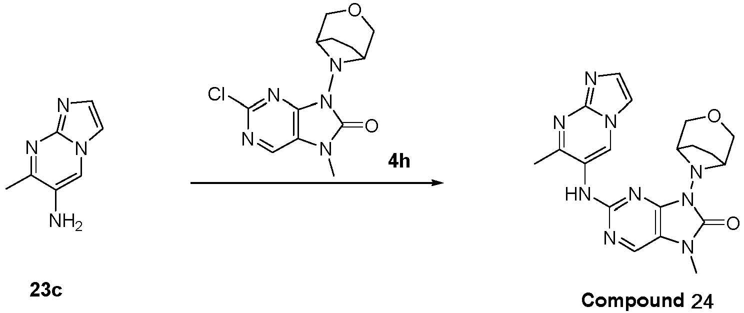

### Step 1

Compound **4h** (40 mg, 135.26 µmol, 1 *eq),* compound **23c** (40.08 mg, 270.52 µmol, *2 eq),* tris(dibenzylideneacetone)dipalladium (12.39 mg, 13.53 µmol, 0.1 *eq),* 4,5-bis(diphenylphosphino)-9,9-dimethylxanthene (15.65 mg, 27.05 µmol, 0.2 *eq)* and cesium carbonate (88.14 mg, 270.52 µmol, 2 *eq)* were placed in a reaction flask, and the reaction flask was vacuumized and repaced with nitrogen three times, and then anhydrous dioxane (2 mL) was added to the mixture and the reaction was carried out at 100 °C for 2 hours. After the reaction was completed, the reaction solution was concentrated under reduced pressure and purified by thin-layer preparative chromatography (methanol: dichloromethane=1:10) to obtain compound **24.** MS: *m*/*z* 408.1 [M+H]⁺.

¹H NMR (400 MHz, CDCl₃) δ ppm 9.60 (s, 1H), 7.89 (s, 1H), 7.74 (s, 1H), 7.43-7.50 (m, 1H), 6.76 (s, 1H), 4.06-4.14 (m, 2H), 3.79-3.86 (m, 2H), 3.67-3.75 (m, 2H), 3.39 (s, 3H), 2.72 (s, 3H), 2.39-2.47 (m, 2H), 2.09-2.18 (m, 2H).

### Biological test data:

### Experimental embodiment 1: DNA-dependent protein kinase (DNA-PK) inhibitory activity screening experiment

This experiment was tested in the Eurofins

Experimental materials and methods:
Human DNA-PK; Mg/ATP; GST-cMyc-p53; EDTA; Ser15 antibody; ATP: 10 µM.

Experimental method (Eurofins Pharma Discovery Service):
DNA-PK (h) was incubated in assay buffer containing 50 nM GST-cMyc-p53 and Mg/ATP (according to the required concentration). The reaction was initiated by adding Mg/ATP mixture. After 30 minutes of incubation at room temperature, the reaction was stopped by adding a stop solution containing EDTA. Finally, detection buffer (containing labeled anti-GST monoclonal antibody and europium-labeled anti-Ser15 antibody against phosphorylated p53) was added. The plate was then read in time-resolved fluorescence mode, and the homogeneous time-resolved fluorescence (HTRF) signal was determined according to the formula HTRF = 10000 × (Em665 nm/Em620 nm).

### Test results:

**Table 1 DNA-PK kinase activity test results**

| **Testing sample** | **DNA-PK kinase inhibition IC₅₀ (nM)** |
|---|---|
| Compound 1 | 2 |
| Compound 4 | 1 |
| Compound 5 | 1 |
| Compound 6 | 1 |
| Compound 7 | 2 |
| Compound 8 | 7 |
| Compound 9 | 3 |
| Compound 10 | 2.5 |
| Compound 11 | 5 |
| Compound 12 | 4 |
| Compound 13 | 2 |
| Compound 14 | 5 |
| Compound 15 | 1 |
| Compound 16 | 0.5 |
| Compound 17 | 0.5 |
| Compound 18 | 0.9 |
| Compound 19 | 0.6 |
| Compound 20 | 0.9 |
| Compound 21 | 0.5 |
| Compound 22 | 0.4 |
| Compound 23 | 10 |
| Compound 24 | 68 |

| | |
|---|---|
| Conclusion: The compounds of the present disclosure have significant DNA-PK kinase inhibitory activity. | |

### Experimental embodiment 2: Pharmacokinetic evaluation (1)

### 1. Experimental method

Test compounds were mixed with 10 % dimethyl sulfoxide/50 % polyethylene glycol 200/40 % water (compounds 1, 4, 10, 17) or 20 % *N,N*-dimethylacetamide/80 % water (compound 5), vortexed and sonicated to prepare a nearly clear solution of 0.08 mg/mL (compounds 1, 4, 10, 17) or 0.50 mg/mL (compound 5), then filtered through a microporous membrane for next step. Balb/c male mice of 18 to 20 grams were selected and administered intravenously with the candidate compound solution at a dose of 0.4 (compounds 1, 4, 10, 17) or 1 mg/kg (compound 5). Test compounds were mixed with 10 % dimethyl sulfoxide/50 % polyethylene glycol 200/40 % water (compounds 1, 4, 10, 17) or 20 %-hydroxypropyl-β-cyclodextrin (compound 5), vortexed and sonicated to prepare a nearly clear solution of 0.2 mg/mL (compounds 1, 4, 10, 17) or 1 mg/mL (compound 5), then filtered through a microporous membrane for next step. Balb/c male mice of 18 to 20 grams were selected and orally administered with the candidate compound solution at a dose of 2 (compounds 1, 4, 10, 17) or 5 mg/kg (compound 5). Whole blood was collected for a certain period of time, and plasma was prepared, then drug concentration was detected by LC-MS/MS method, and pharmacokinetic parameters were calculated by Phoenix WinNonlin software (Pharsight, USA).

### Definition of each parameter:

IV: intravenous injection; PO: oral; Co: instantaneous required concentration after intravenous injection; Cₘₐₓ: highest plasma concentration after administration; Tₘₐₓ: time required to reach peak of drug concentration after administration; T_{1/2}: time required for the plasma drug concentration to decrease by half; V_{dss}: apparent volume of distribution, refers to the proportional constant of the drug dose *in vivo* and the blood drug concentration when the drug reaches a dynamic equilibrium *in vivo.* Cl: clearance rate, refers to the apparent volume of distribution of the drug cleared from the body per unit time; Tₗₐₛₜ: time at the last detection point; AUC₀₋ₗₐₛₜ: area under the drug-time curve, refers to the area covered by the plasma concentration curve and the time axis; F: a measure of the speed and degree of drug absorption into the blood circulation, which is an important indicator for evaluating the degree of drug absorption.

### Test results:

The experimental results are shown in Table 2.

**Table 2 Cassette PK test results of compounds in plasma**

| Parameter | | Co (nM) | Cmax (nM) | Tmax (h) | T_{1/2} (h) | V_{dss} (L/kg) | Cl (mL/min/k g) | Tₗₐₛₜ (h) | AUC₀₋ₗₐₛₜ (nM.h) | F (%) |
|---|---|---|---|---|---|---|---|---|---|---|
| Compound 1 | IV(0.4 mg/kg) | 959 | - | - | 0.598 | 1.51 | 37.7 | 4 | 426 | - |
| | PO(2 mg/kg) | - | 1425 | 0.5 | 0.841 | - | - | 8 | 2497 | 96.4 |
| Compound 4 | IV(0.4 mg/kg) | 919 | - | - | 0.24 | 1.08 | 57 | 2 | 286 | - |
| | PO(2 mg/kg) | - | 1595 | 0.25 | 0.609 | - | - | 4 | 1736 | 123 |
| Compound 5 | IV(1 mg/kg) | 3604 | - | - | 1.28 | 1.49 | 24.7 | ND | 1657 | - |
| | PO(5 | - | 2170 | 0.25 | 3.85 | - | - | 10 | 3489 | 44.5 |
| | mg/kg) | | | | | | | | | |
| Compound 10 | IV(0.4 mg/kg) | 1121 | - | - | 0.591 | 0.883 | 25.4 | 4 | 640 | - |
| | PO(2 mg/kg) | - | 2065 | 0.5 | 1.22 | - | - | 8 | 3832 | 120 |
| Compound 17 | IV(0.4 mg/kg) | 748 | - | - | 0.559 | 1.39 | 34.7 | 4 | 470 | - |
| | PO(2 mg/kg) | - | 1545 | 1.0 | 0.81 | - | - | 8 | 3397 | 144 |

| | | | | | | | | | | |
|---|---|---|---|---|---|---|---|---|---|---|
| ND: Not detected "--" means that not tested or no data was obtained. | | | | | | | | | | |

Conclusion: The compounds of the present disclosure exhibit longer half-life, lower clearance rate and higher drug exposure, and have good pharmacokinetic properties *in vivo.*

### Experimental embodiment 3: Pharmacokinetic evaluation in mice (2)

### Experimental method:

Test compounds were mixed with 10 % dimethyl sulfoxide/50 % polyethylene glycol 200/40 % water, vortexed and sonicated to prepare a nearly clear solution of 0.4 mg/mL (compound 5), then filtered through a microporous membrane for next step. Balb/c male mice of 18 to 20 grams were selected and administered intravenously with the candidate compound solution at a dose of 2 mg/kg. Test compounds were mixed with 10 % dimethyl sulfoxide/50 % polyethylene glycol 200/40 % water, vortexed and sonicated to prepare a nearly clear solution of 1 mg/mL, then filtered with a microporous membrane for next step. Balb/c male mice of 18 to 20 grams were selected and orally administered with the candidate compound solution at a dose of 10 mg/kg. Whole blood was collected for a certain period of time, and plasma was prepared, then drug concentration was detected by LC-MS/MS method, and pharmacokinetic parameters were calculated by Phoenix WinNonlin software (Pharsight, USA).

### Test results:

The experimental results are shown in Table 3.

**Table 3 PK test results of compound in plasma**

| Parameter | | Co (nM) | Cmax (nM) | Tmax (h) | T_{1/2} (h) | V_{dss} (L/kg) | Cl (mL/min/kg ) | Tₗₐₛₜ (h) | AUC₀₋ₗₐₛₜ (nM.h) | F (%) |
|---|---|---|---|---|---|---|---|---|---|---|
| Compound 5 | IV (2 mpk) | 4742 | - | - | 0.733 | 1.07 | 23.7 | 8 | 3454 | - |
| | PO (10 | - | 11433 | 0.50 | 0.850 | - | - | 10 | 23184 | 134 |
| | mpk) | | | | | | | | | |

| | | | | | | | | | | |
|---|---|---|---|---|---|---|---|---|---|---|
| "--" means that not tested or no data was obtained. | | | | | | | | | | |

Conclusion: The compounds of the present disclosure exhibit longer half-life, lower clearance rate and higher drug exposure, and have good pharmacokinetic properties *in vivo.*

### Experimental embodiment 4: Pharmacokinetic and brain exposure evaluation in rats

### Experimental method:

Test compounds were mixed with 10 % dimethyl sulfoxide/50 % polyethylene glycol 200/40 % water, vortexed and sonicated to prepare a nearly clear solution of 0.5 mg/mL (compound 5) or 1 mg/mL (compound 17), then filtered through a microporous membrane for next step. SD male rats were selected and administered intravenously with the candidate compound solution at a dose of 1 mg/kg (compound 5) or 2 mg/kg (compound 17). Test compounds were mixed with 10 % dimethyl sulfoxide/50 % polyethylene glycol 200/40 % water, vortexed and sonicated to prepare a homogeneous suspension of 4 mg/mL (compound 5) or a nearly clear solution of 1 mg/mL (compound 17). SD male rats were selected and orally administered with the candidate compound solution at a dose of 40 mg/kg (compound 5) or 10 mg/kg (compound 17). Whole blood, cerebrospinal fluid, brain tissue were collected for a certain period of time and plasma was prepared, and the drug concentration was detected by LC-MS/MS method, and the pharmacokinetic parameters were calculated by Phoenix WinNonlin software (Pharsight, USA).

### Test results:

The experimental results are shown in Table 4.

**Table 4 PK test results of compounds in plasma, cerebrospinal fluid, and brain tissue**

| Parameter | | | Co (nM) | Cmax (nM) | Tmax (h) | T_{1/2} (h) | V_{dss} (L/kg) | Cl (mL/min/kg) | Tₗₐₛₜ (h) | AUC₀₋ₗₐₛₜ (nM.h) |
|---|---|---|---|---|---|---|---|---|---|---|
| Compound 5 | IV (1 mpk) | | 3043 | - | - | 1.45 | 1.41 | 17.0 | 8 | 2382 |
| | PO (40 mp k) | Plasma | - | 10223 | 2 | ND | - | - | 8 | 59896 |
| | | Cerebr ospinal fluid | - | 1260 | 2 | ND | - | - | 8 | 7740 |
| | | Brain tissue | - | 1019^{a} | 4 | ND | - | - | 8 | 6369^{b} |
| Compound | IV (2 mpk) | | 5303 | - | - | 1.16 | 1.40 | 16.9 | 8 | 4861 |
| | PO (10 mpk) | | - | 6245 | 2 | 2.29 | - | - | 8 | 32535 |
| 17 | | | | | | | | | | |

| | | | | | | | | | | |
|---|---|---|---|---|---|---|---|---|---|---|
| ND: Not detected "--" means that not tested or no data was obtained. a: unit nmol/kg; b: unit h^{∗}nmol/kg. | | | | | | | | | | |

Conclusion: The compounds of the present disclosure exhibit longer half-life, lower clearance rate and higher drug exposure, and have good pharmacokinetic properties *in vivo.* At the same time, the compound has a good brain exposure.

### Experimental embodiment 5: Pharmacokinetic evaluation in Beagle dogs

### Experimental method:

Test compounds were mixed with 10 % dimethyl sulfoxide/50 % polyethylene glycol 200/40 % water, vortexed and sonicated to prepare a nearly clear solution of 1 mg/mL, then filtered through a microporous membrane for next step. Male Beagle dogs were selected and administered intravenously with the candidate compound solution at a dose of 1 mg/kg. Test compounds were mixed with 10 % dimethyl sulfoxide/50 % polyethylene glycol 200/40 % water, vortexed and sonicated to prepare a nearly clear solution of 1 mg/mL, then filtered with a microporous membrane for next step. Male Beagle dogs were selected and orally administered with the candidate compound solution at a dose of 5 mg/kg. Whole blood was collected for a certain period of time, and plasma was prepared, then drug concentration was detected by LC-MS/MS method, and pharmacokinetic parameters were calculated by Phoenix WinNonlin software (Pharsight, USA).

### Test results:

The experimental results are shown in Table 5.

**Table 5 PK test results of compounds in plasma**

| Parameter | | Co (nM) | Cmax (nM) | Tmax (h) | T_{1/2} (h) | V_{dss} (L/kg) | Cl (mL/min/kg) | Tₗₐₛₜ (h) | AUC₀₋ₗₐₛₜ (nM.h) | F (%) |
|---|---|---|---|---|---|---|---|---|---|---|
| Compound 17 | IV (1 mpk) | 1450 | - | - | 5.04 | 1.58 | 5.89 | 24 | 6817 | - |
| | PO (5 mpk) | - | 4935 | 1.50 | 4.27 | - | - | 24 | 31535 | 92.3 |
| Compound 5 | IV (1 mpk) | 1820 | - | - | 4.04 | 1.87 | 8.26 | 24 | 4957 | - |
| | PO (5 mpk) | - | 9270 | 1.67 | 4.27 | - | - | 24 | 68263 | 279 |

| | | | | | | | | | | |
|---|---|---|---|---|---|---|---|---|---|---|
| "--" means that not tested or no data was obtained. | | | | | | | | | | |

Conclusion: The compounds of the present disclosure exhibit longer half-life, lower clearance rate and higher drug exposure, and have good pharmacokinetic properties *in vivo.*

### Experimental embodiment 6: Pharmacokinetic evaluation in cynomolgus monkeys

### Experimental method:

Test compound was mixed with 10 % dimethyl sulfoxide/50 % polyethylene glycol 200/40 % water, vortexed and sonicated to prepare a nearly clear solution of 1 mg/mL, then filtered through a microporous membrane for next step. Male cynomolgus monkeys were selected and administered intravenously with the candidate compound solution at a dose of 1 mg/kg. Test compound was mixed with 10 % dimethyl sulfoxide/50 % polyethylene glycol 200/40 % water, vortexed and sonicated to prepare a nearly clear solution of 1 mg/mL, then filtered with a microporous membrane for next step. Male cynomolgus monkeys were selected and orally administered with the candidate compound solution at a dose of 5 mg/kg. Whole blood was collected for a certain period of time, and plasma was prepared, then drug concentration was detected by LC-MS/MS method, and pharmacokinetic parameters were calculated by Phoenix WinNonlin software (Pharsight, USA).

### Test results:

The experimental results are shown in Table 6.

**Table 6 PK test results of compound in plasma**

| Parameter | | Co (nM) | Cmax (nM) | Tmax (h) | T_{1/2} (h) | V_{dss} (L/kg) | Cl (mL/min/kg) | Tₗₐₛₜ (h) | AUC₀₋ₗₐₛₜ (nM.h) | F (%) |
|---|---|---|---|---|---|---|---|---|---|---|
| Compound 5 | IV (1 mpk) | 2291 | - | - | 3.60 | 1.52 | 16.8 | ND | 2440 | - |
| | PO (5 mpk) | - | 4503 | 1.17 | 1.35 | - | - | 12 | 14024 | 115 |

| | | | | | | | | | | |
|---|---|---|---|---|---|---|---|---|---|---|
| ND: Not detected "--" means that not tested or no data was obtained. | | | | | | | | | | |

Conclusion: The compounds of the present disclosure exhibit longer half-life, lower clearance rate and higher drug exposure, and have good pharmacokinetic properties *in vivo.*

### Experimental embodiment 7: In vivo pharmacodynamics study of BALB/c nude mice model with human non-small cell lung cancer NCI-H1703 cell subcutaneous xenograft tumor

Experimental purpose: To study the *in vivo* pharmacodynamics of the test compounds in BALB/c nude mice model with human non-small cell lung cancer NCI-H1703 cell subcutaneous xenograft tumor

Experimental animals: Female BALB/c nude mice, 6-8 weeks old, weighing 18-22 grams; supplier: Shanghai Sippe-Bk Lab Animal Co., Ltd.

### Experimental methods and steps:

### 7.1 Cell culture

Human non-small cell lung cancer NCI-H1703 cells were cultured *in vitro* in RPMI1640 medium with 10 % fetal bovine serum, 100 U/mL penicillin and 100 µg/mL streptomycin at 37 °C in a 5% CO₂ incubator. Conventional digestion with trypsin-EDTA was performed twice a week for passage. When the cell saturation was 80 %-90 % and the number reached the requirement, the cells were collected, counted, and seeded.

### 7.2 Tumor cell seeding (tumor seeding)

0.2 mL (5×10⁶ cells) NCI-H1703 cells (added with matrigel, volume ratio of 1:1) were subcutaneously inoculated into the right back of each mouse, and the group administration started when the average tumor volume reached about 130 mm³.

### 7.3 Preparation of test sample:

Compound 5 was prepared into 3 mg/mL, 6 mg/mL, 9 mg/mL suspension solutions, and compound 17 was prepared into 9 mg/mL suspensions, and the solvent was 0.5 % HPMC+1 % Tween 80.

### 7.4 Tumor measurements and experimental indicators

Tumor diameters were measured with vernier calipers twice a week. The calculation formula of tumor volume is: V = 0.5*a* × *b*², wherein *a* and *b* represent the long and short diameters of the tumor, respectively.

The antitumor efficacy of the compounds was evaluated by TGI (%) or relative tumor proliferation rate T/C (%). Relative tumor proliferation rate T/C (%) = T_{RTV}/C_{RTV} × 100% (T_{RTV}: means RTV in the treatment group; C_{RTV}: means RTV in the negative control group). The relative tumor volume (RTV) was calculated according to the results of tumor measurement. The calculation formula is RTV = Vₜ/V₀, wherein V₀ is the tumor volume measured at the time of group administration (i.e., day 0), and Vt is the tumor volume at one measurement. Data of the same day was taken for T_{RTV} and C_{RTV}.

TGI (%), reflecting tumor growth inhibition rate. TGI(%)=[1-(average tumor volume at the end of administration of a certain treatment group-average tumor volume at the beginning of administration of this treatment group)/(average tumor volume at the end of treatment in the solvent control group-average volume at the beginning of treatment in the solvent control group tumor volume)] × 100%. After the experiment, the tumor weight was detected, and the T/weight percentage was calculated. T weight and C weight represent the tumor weight of the administration group and the solvent control group, respectively.

### 7.5 Statistical analysis

Statistical analysis was performed using SPSS software based on RTV data at the end of the experiment. The treatment group showed the best treatment effect on the 21st day after dosing at the end of the experiment, therefore statistical analysis was performed based on this data to evaluate the difference between groups. T-test was used to analyze the comparison between two groups, and one-way ANOVA was used to analyze the comparison between three or more groups. If the variance was homogeneous (F value had no significant difference), Tukey ' s method was used for analysis. If the variance was not homogeneous (F value had significant difference), the Games-Howell method was used to test. p < 0.05 was considered a significant difference.

### 7.6 Experimental conclusions and discussion

In this experiment, compound 5 at doses of 60 mg/kg and 90 mg/kg (dosed twice a day), and compound 17 at a dose of 90 mg/kg (dosed twice a day), compared with the blank control have a significant inhibitory effect. Compound 5 has a certain tumor inhibitory effect at a dose of 30 mg/kg (administered twice a day). The therapeutic effects of compound 5 are all dose-dependent, and the experimental results are shown in Table 7. Results of tumor weights and tumor photographs on day 21 are shown in Table 8 and Figure 1.

**Table 7 Tumor inhibitory effect of compounds on human lung cancer NCI-H1703 xenograft tumor model**

| **Group** | **Tumor volume (mm³)^{a} (Day 0)** | **Tumor volume (mm³)^{a} (Day 21)** | **RTV (Day 21)** | **TGI (%)^{b} (Day 21)** | **T/C (%)^{b} (Day 21)** | ***p*^{c}** |
|---|---|---|---|---|---|---|
| Blank control group | 131 ± 11 | 1200 ± 54 | 9.55 ± 0.69 | - | - | - |
| Compound 5 (30 mg/kg) | 131 ± 10 | 979 ± 86 | 7.57 ± 0.53 | 20.6 | 79.3 | 0.202 |
| Compound 5 (60 mg/kg) | 131 ± 10 | 427 ± 64 | 3.31 ± 0.51 | 72.3 | 34.7 | <0.001 |
| Compound 5 (90 mg/kg) | 130 ± 10 | 351 ± 64 | 2.64 ± 0.45 | 79.4 | 27.7 | <0.001 |
| Compound 17 (90 mg/kg) | 130 ± 13 | 435 ± 94 | 3.19 ± 0.46 | 71.6 | 33.4 | <0.001 |

| | | | | | | |
|---|---|---|---|---|---|---|
| Notes: a. Mean ± SEM, n=9 (compound 5) or n=6 (compound 17). b. Tumor growth inhibition was calculated from T/C and TGI (TGI (%) = [1-(T₂₁-T₀)/(V₂₁-V₀)] × 100). c. *p*-value was obtained by analyzing relative tumor volume (RTV) using one-way ANOVA. | | | | | | |

**Table 8 Tumor weights and photographs in each experimental group**

| **Group** | **Tumor weight (g)^{a} (PG-D21)** | **T/C_{weight}^{b} (%)** | ***p*-value^{c}** | **Corresponding photographs** |
|---|---|---|---|---|
| Blank control group | 1.296 ± 0.060 | - | - | First row |
| Compound 5 (30 mg/kg) | 1.031 ± 0.095 | 79.6 | 0.301 | Second row |
| Compound 5 (60 mg/kg) | 0.467 ± 0.078 | 36.0 | <0.001 | Third row |
| Compound 5 (90 mg/kg) | 0.379 ± 0.069 | 29.2 | <0.001 | Fourth row |
| Compound 17 (90 mg/kg) | 0.466 ± 0.103 | 35.9 | <0.001 | Fifth row |

| | | | | |
|---|---|---|---|---|
| Note: a. Mean ± SEM, n=9 (compound 5) or n=6 (compound 17). b. Tumor growth inhibition was calculated from T/C_{weight} = TWₜᵣₑₐₜₘₑₙₜ/TW solvent. c. The *p* value was analyzed by one-way ANOVA and solvent treatment group, if there was a significant difference in the F value, Games-Howell method should be used to analyze. | | | | |

Conclusion: In this experiment, compound 5 at the dose of 60 mg/kg and 90 mg/kg, and compound 17 at the dose of 90 mg/kg, have a significant tumor inhibitory effect compared with the control group, and the curative effect of compound 5 is dose-dependent. In this experiment, the tumor-bearing mice show good tolerance to the compounds, and there is no significant weight loss in all treatment groups.

## Claims

1. The present disclosure provides a compound represented by formula (IV) or a pharmaceutically acceptable salt thereof, 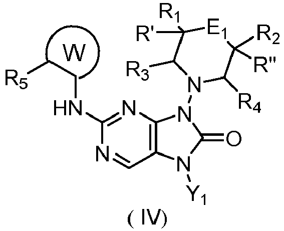 wherein,
the structural moiety 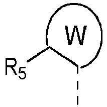 is selected from 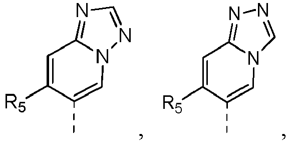 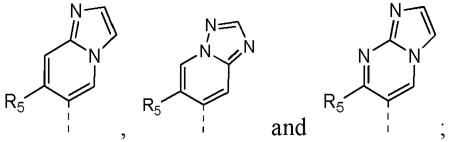
E₁ is selected from a single bond, -O- and -C(R₆R₇)-;
R₁, R₂, R₃, R₄, R' and R" are each independently selected from H, F and Cl;
or R₁ and R₂ are connected together such that the structural moiety 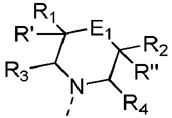 is selected from 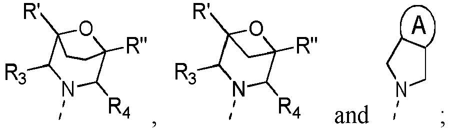
or R₃ and R₄ are connected together such that the structural moiety 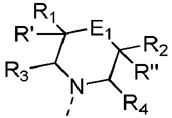 is selected from 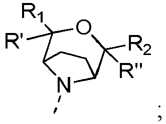
or R₁ and R₄ are connected together such that the structural moiety 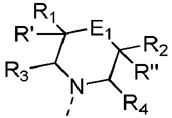 is selected from 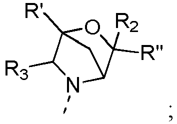
or R₂ and R" connected together with the carbon atoms to which they are attached form a C₃₋₅ cycloalkyl;
R₅ is selected from F, Cl, Br, I, cyclopropyl and C₁₋₃ alkyl, and the C₁₋₃ alkyl is optionally substituted with OH or 1, 2 or 3 Rₐ;
R₆ and R₇ are each independently selected from H, F, Cl, Br, I and CN;
or R₆ and R₇ connected together with the carbon atoms to which they are attached form a cyclopropyl or a 4-membered oxetanyl;
ring A is selected from C₃₋₅ cycloalkyl;
Y₁ is selected from cyclopropyl and C₁₋₃ alkyl, and the C₁₋₃ alkyl is optionally substituted with 1, 2, 3, 4 or 5 F;
Rₐ is selected from H, F, Cl, Br and I.

2. The compound as claimed in claim 1 or the pharmaceutically acceptable salt thereof, wherein the compound represented by formula (IV) or the pharmaceutically acceptable salt thereof is selected from a compound represented by formula (III) or a pharmaceutically acceptable salt thereof, 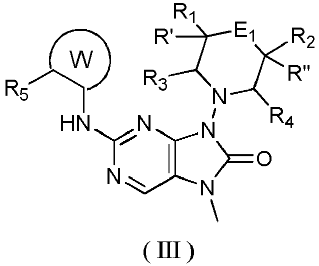 wherein, W, E₁, R₁, R₂, R₃, R₄, R₅, R' and R" are as defined in claim 1.

3. The compound as claimed in claim 1 or 2 or the pharmaceutically acceptable salt thereof, wherein the compound is selected from 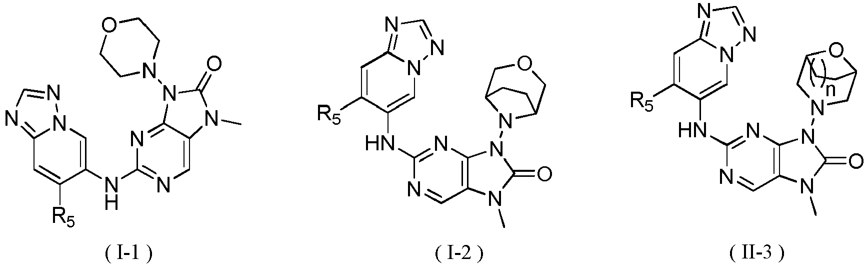 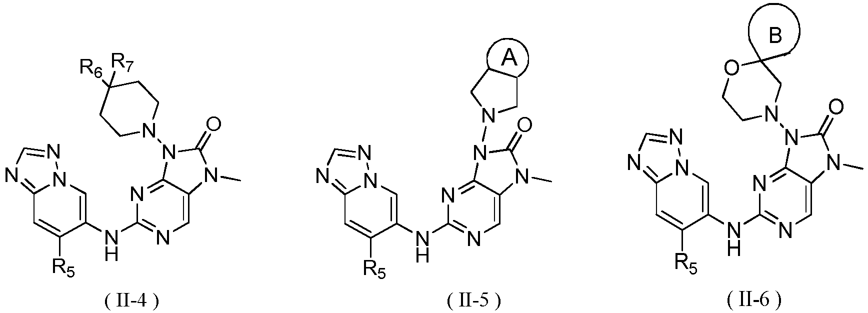 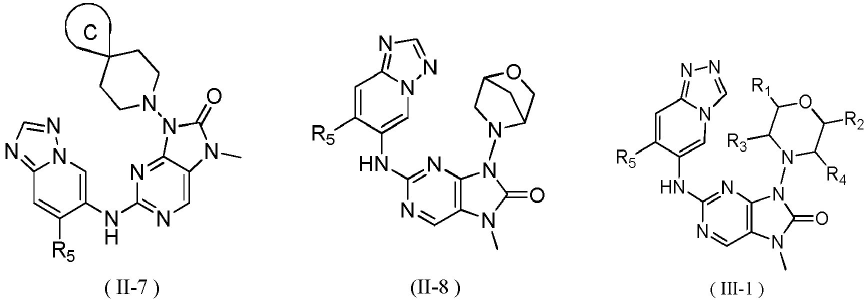 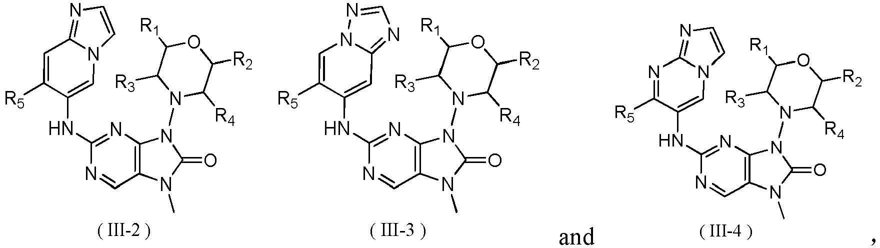 wherein, ring B is selected from C₃₋₅ cycloalkyl;
ring C is cyclopropyl or 4-membered oxetanyl;
n is selected from 0 and 1;
ring A, R₁, R₂, R₃, R₄, R₅, R₆ and R₇ are as defined in claim 1.

4. The compound as claimed in any one of claims 1-3 or the pharmaceutically acceptable salt thereof, wherein R₁ and R₂ are connected together such that the structural moiety 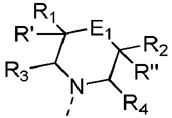 is selected from 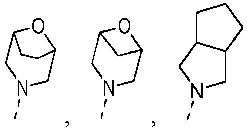 and

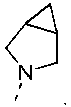

5. The compound as claimed in any one of claims 1-3 or the pharmaceutically acceptable salt thereof, wherein R₃ and R₄ are connected together such that the structural moiety 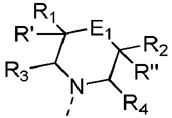 is selected from

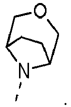

6. The compound as claimed in any one of claims 1-3 or the pharmaceutically acceptable salt thereof, wherein R₁ and R₄ are connected together such that the structural moiety 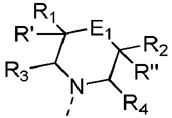 is selected from

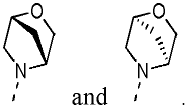

7. The compound as claimed in any one of claims 1-3 or the pharmaceutically acceptable salt thereof, wherein ring A is selected from

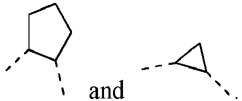

8. The compound as claimed in any one of claims 1-3 or the pharmaceutically acceptable salt thereof, wherein R₆ and R₇ connected together with the carbon atoms to which they are attached form

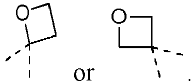

9. The compound as claimed in any one of claims 1-3 or the pharmaceutically acceptable salt thereof, wherein R₂ and R" connected together with the carbon atoms to which they are attached form

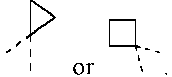

10. The compound as claimed in any one of claims 1-9 or the pharmaceutically acceptable salt thereof, wherein R₅ is selected from F, Cl, CH₂OH, CF₃ and CH₃.

11. A compound represented by following formula or a pharmaceutically acceptable salt thereof 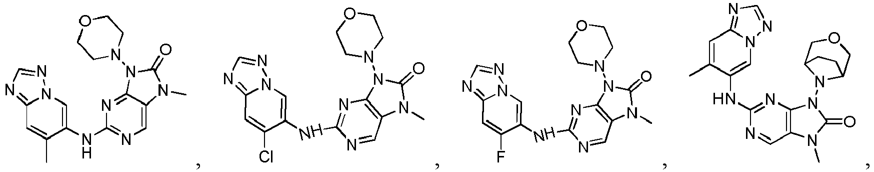 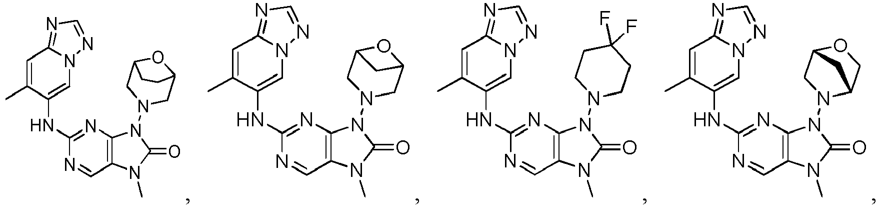 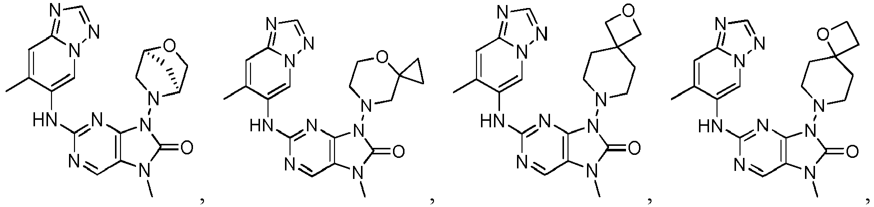 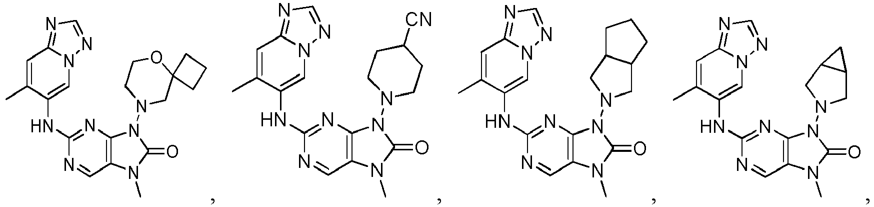 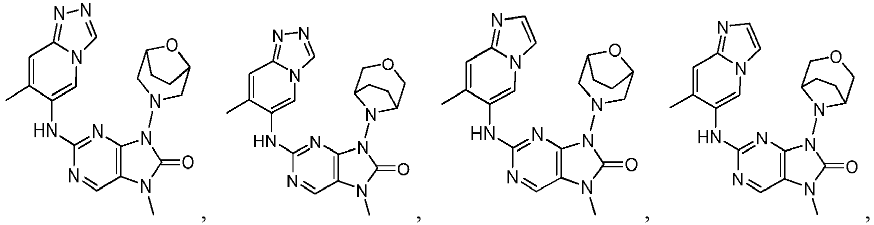 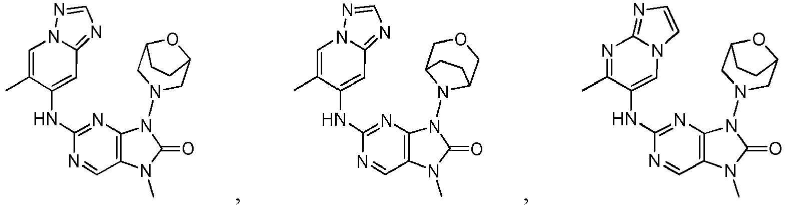 and

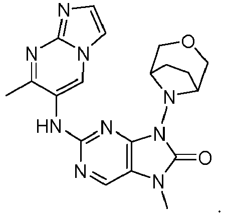

12. A use of the compound as defined in any one of claims 1-11 or the pharmaceutically acceptable salt thereof in the manufacture of a medicament related to DNA-PK inhibitor.

13. The use as claimed in claim 12, wherein the medicament related to DNA-PK inhibitor plays a therapeutic effect as a single medicament in tumors with defects in other DNA repair pathways.

14. The use as claimed in claim 12, wherein the medicament related to DNA-PK inhibitor is used in combination with a chemoradiotherapy medicament to enhance the inhibitory effect on solid tumors and hematological tumors.
